# EUROPEAN PATENT SPECIFICATION

(11) **EP 2 925 350 B1**
(45) Date of publication and mention of the grant of the patent: **30.01.2019**
(21) Application number: 13811698.3
(22) Date of filing: 03.12.2013
(51) Int. Cl.: A61K 39/00, A61P 35/00, C07K 16/28

(54) **ENHANCING ANTI-CANCER ACTIVITY OF IMMUNOMODULATORY FC FUSION PROTEINS**
VERBESSERUNG DER ANTIKREBSWIRKUNG VON IMMUNMODULATORISCHEN FC-FUSIONSPROTEINEN
AMÉLIORATION DE L'ACTIVITÉ ANTICANCÉREUSE DE PROTÉINES DE FUSION DE FC IMMUNO-MODULATRICES

(30) Priority: 03.12.2012 US 201261732760 P; 15.03.2013 US 201361801187 P
(43) Date of publication of application: 07.10.2015
(60) Divisional application: 18209431.8
(73) Proprietor: Bristol-Myers Squibb Company, Princeton, NJ 08543 (US)
(72) Inventor: ENGELHARDT, John, J., Redwood City, CA 94063 (US); KORMAN, Alan, J., Redwood City, CA 94063 (US); QUIGLEY, Michael, Redwood City, CA 94063 (US); SELBY, Mark, J., Redwood City, CA 94063 (US); WANG, Changyu, Union City, CA 94587 (US)
(74) Representative: Reitstötter Kinzebach
(86) International application number: PCT/US2013/072918
(87) International publication number: WO 2014/089113

(56) References cited:
- WO-A1-2007/133822
- WO-A2-2007/048077
- WO-A2-2008/137915
- D. O'MAHONY ET AL: "A Pilot Study of CTLA-4 Blockade after Cancer Vaccine Failure in Patients with Advanced Malignancy", CLINICAL CANCER RESEARCH, vol. 13, no. 3, 1 February 2007 (2007-02-01), pages 958-964, XP055100758, ISSN: 1078-0432, DOI: 10.1158/1078-0432.CCR-06-1974
- W. LIN ET AL: "Fc-dependent expression of CD137 on human NK cells: insights into "agonistic" effects of anti-CD137 monoclonal antibodies", BLOOD, vol. 112, no. 3, 1 August 2008 (2008-08-01), pages 699-707, XP055037053, ISSN: 0006-4971, DOI: 10.1182/blood-2007-11-122465
- C. USTUN ET AL: "Regulatory T cells in acute myelogenous leukemia: is it time for immunomodulation?", BLOOD, vol. 118, no. 19, 10 November 2011 (2011-11-10), pages 5084-5095, XP055101216, ISSN: 0006-4971, DOI: 10.1182/blood-2011-07-365817
- M. J. SELBY ET AL: "Anti-CTLA-4 Antibodies of IgG2a Isotype Enhance Antitumor Activity through Reduction of Intratumoral Regulatory T Cells", CANCER IMMUNOLOGY RESEARCH, vol. 1, no. 1, 7 April 2013 (2013-04-07), pages 32-42, XP055100395, ISSN: 2326-6066, DOI: 10.1158/2326-6066.CIR-13-0013
- SIMPSON TYLER R ET AL: "Fc-dependent depletion of tumor-infiltrating regulatory T cells co-defines the efficacy of anti-CTLA-4 therapy against melanoma", THE JOURNAL OF EXPERIMENTAL MEDICINE, ROCKEFELLER UNIVERSITY PRESS, US, vol. 210, no. 9, 26 August 2013 (2013-08-26), pages 1695-1710, XP009176006, ISSN: 0022-1007 [retrieved on 2013-07-29]
- STEFANIA LAURENT ET AL: "The engagement of CTLA-4 on primary melanoma cell lines induces antibody-dependent cellular cytotoxicity and TNF-[alpha] production", JOURNAL OF TRANSLATIONAL MEDICINE, vol. 11, no. 1, 1 January 2013 (2013-01-01), page 108, XP055100523, ISSN: 1479-5876, DOI: 10.1007/s00262-005-0668-3
- BULLIARD YANNICK ET AL: "Activating Fc gamma receptors contribute to the antitumor activities of immunoregulatory receptor-targeting antibodies", THE JOURNAL OF EXPERIMENTAL MEDICINE, ROCKEFELLER UNIVERSITY PRESS, US, vol. 210, no. 9, 26 August 2013 (2013-08-26), pages 1685-1693, XP009176020, ISSN: 0022-1007

## Description

Throughout this application, various publications are referenced in parentheses by author name and date, or by Patent No. or Patent Publication No. Full citations for these publications may be found at the end of the specification immediately preceding the claims.

### FIELD OF THE INVENTION

The present disclosure relates to afucosylated ipilimumab, a human anti-human CTLA-4 monoclonal antibody, for use in treating cancer.

### BACKGROUND OF THE INVENTION

The immune system is capable of controlling tumor development and mediating tumor regression. This requires the generation and activation of tumor antigen-specific T cells. Multiple T-cell costimulatory receptors and T-cell negative regulators, or coinhibitory receptors, act in concert to control T-cell activation, proliferation, and gain or loss of effector function. Among the earliest and best characterized T-cell costimulatory and coinhibitory molecules are CD28 and CTLA-4 (Rudd *et al.,* 2009). CD28 provides costimulatory signals to T-cell receptor engagement by binding to B7-1 and B7-2 ligands on antigen-presenting cells, while CTLA-4 provides a negative signal down-regulating T-cell proliferation and function. CTLA-4, which also binds the B7-1 (CD80) and B7-2 (CD86) ligands but with higher affinity than CD28, acts as a negative regulator of T-cell function through both cell autonomous (or intrinsic) and cell nonautonomous (or extrinsic) pathways. Intrinsic control of CD8 and CD4 T effector (T_{eff}) function is mediated by the inducible surface expression of CTLA-4 as a result of T-cell activation, and inhibition of T-cell proliferation and cytokine proliferation by multivalent engagement of B7 ligands on opposing cells (Peggs *et al.,* 2008).

Several other costimulatory and inhibitory receptors and ligands that regulate T cell responses have been identified. Examples of stimulatory receptors include Inducible T cell Co-Stimulator (ICOS), CD137 (4-1BB), CD134 (OX40), CD27, Glucocorticoid-Induced TNFR-Related protein (GITR), and HerpesVirus Entry Mediator (HVEM), whereas examples of inhibitory receptors include Programmed Death-1 (PD-1), B and T Lymphocyte Attenuator (BTLA), T cell Immunoglobulin and Mucin domain-3 (TIM-3), Lymphocyte Activation Gene-3 (LAG-3), adenosine A2a receptor (A2aR), Killer cell Lectin-like Receptor G1 (KLRG-1), Natural Killer Cell Receptor 2B4 (CD244), CD160, T cell Immunoreceptor with Ig and ITIM domains (TIGIT), and the receptor for V-domain Ig Suppressor of T cell Activation (VISTA), (Mellman *et al.,* 2011; Pardoll, 2012b; Baitsch *et al.,* 2012). These receptors and their ligands provide targets for therapeutics designed to stimulate, or prevent the suppression, of an immune response so as to thereby attack tumor cells (Weber, 2010; Flies *et al.,* 2011; Mellman *et al.,* 2011; Pardoll, 2012b). Stimulatory receptors or receptor ligands are targeted by agonist agents, whereas inhibitory receptors or receptor ligands are targeted by blocking agents. Among the most promising approaches to enhancing immunotherapeutic anti-tumor activity is the blockade of so-called "immune checkpoints," which refer to the plethora of inhibitory signaling pathways that regulate the immune system and are crucial for maintaining self-tolerance and modulating the duration and amplitude of physiological immune responses in peripheral tissues in order to minimize collateral tissue damage (*see, e.g.,* Weber, 2010; Pardoll 2012b). Because many of the immune checkpoints are initiated by ligand-receptor interactions, they can be readily blocked by antibodies or modulated by recombinant forms of ligands or receptors.

Anti-CTLA-4 antibodies, when cross-linked, suppress T cell function *in vitro* (Krummel and Allison, 1995; Walunas *et al.,* 1994). Regulatory T cells (T_{regs}), which express CTLA-4 constitutively, control effector T cell (T_{eff}) function in a non-cell autonomous fashion. T_{regs} that are deficient for CTLA-4 have impaired suppressive ability (Wing et al., 2008) and antibodies that block CTLA-4 interaction with B7 can inhibit T_{reg} function (Read *et al.*; 2000; Quezada *et al.,* 2006). More recently, T_{effs} have also been shown to control T cell function through extrinsic pathways (Corse *et al.*; 2012; Wang *et al.,* 2012). Extrinsic control of T cell function by T_{regs} and T_{effs} occurs through the ability of CTLA-4-positive cells to remove B7 ligands on antigen-presenting cells, thereby limiting their costimulatory potential (Qureshi *et al.*; 2011; Onishi *et al.,* 2008). Antibody blockade of CTLA-4/B7 interactions is thought to promote T_{eff} activation by interfering with negative signals transmitted by CTLA-4 engagement; this intrinsic control of T-cell activation and proliferation can promote both T_{eff} and T_{reg} proliferation (Krummel and Allison, 1995; Quezada *et al.,* 2006). In early studies with animal models, antibody blockade of CTLA-4 was shown to exacerbate autoimmunity (Perrin *et al.,* 1996; Hurwitz *et al.,* 1997). By extension to tumor immunity, the ability of anti-CTLA-4 to cause regression of established tumors provided a dramatic example of the therapeutic potential of CTLA-4 blockade (Leach *et al.,* 1996).

Human antibodies to human CTLA-4, ipilimumab and tremelimumab, were selected to inhibit CTLA-4-B7 interactions (Keler *et al.,* 2003; Ribas *et al.,* 2007) and have been tested in a variety of clinical trials for multiple malignancies (Hoos *et al.,* 2003; Acierto *et al.,* 2011). Tumor regressions and disease stabilization were frequently observed, and treatment with these antibodies has been accompanied by adverse events with inflammatory infiltrates capable of affecting a variety of organ systems. In 2011, ipilimumab, which has an IgG1 constant region, was approved in the US and EU for the treatment of unresectable or metastatic melanoma based on an improvement in overall survival in a phase III trial of previously treated patients with advanced melanoma (Hodi *et al.,* 2010).

O'Mahony et al., Clin Cancer Res 2007;13(3), 958-964 report a pilot study of CTLA-4 blockade after cancer vaccine failure in patients with advanced malignancy. It was observed that Tregs as detected by expression of CD4+CD25+CD62L+ declined at early time points but rebounded to levels at or above baseline values at the time of the next infusion of ipilimumab.

Several different antibodies have been used to demonstrate activity of anti-CTLA-4 blockade in mouse models, including hamster anti-CTLA-4 antibodies, 9H10 (Syrian hamster IgG2b [Krummel and Allison, 1995]) and 4F10 (Armenian hamster IgG1 [Walunas *et al.,* 1994]), and mouse anti-mouse CTLA-4 antibody (9D9-murine IgG2b) generated in a human CTLA-4 transgenic mouse (Quezada *et al.,* 2006; Peggs *et al.,* 2009). Anti-CTLA-4 9D9-IgG2b has been tested in a variety of mouse subcutaneous tumor models, such as Sa1N fibrosarcoma, MC38 and CT26 colon adenocarcinomas, and B16 melanoma. Except for Sa1N, anti-CTLA-4 monotherapy has shown modest antitumor activity (Quezada *et al.,* 2006; Mitsui *et al.,* 2010). Murine IgG2b can bind to immunoglobulin Fcγ receptors (FcγR), including FcγRIIB, FcγRIII, and FcγRIV receptors) (Nimmerjahn and Ravetch, 2005). Consequently, it is possible that multivalent engagement of CTLA-4 by 9D9-IgG2b bound to T cells and FcyR-positive cells could result in an agonistic negative signal and render this antibody less effective in CTLA-4 blockade than a blocking antibody with no FcyR binding properties.

To determine the relative potency of mouse anti-CTLA-4 antibodies in antitumor activity, a series of 9D9 isotype variants were generated that differ in their affinity for FcyRs. Data presented herein illuminate the mechanisms, involving depletion of regulatory T cells (T_{regs}) by which CTLA-4 blockade mediates antitumor effects. These mechanisms are shown to apply to certain other targets on T cells, *e.g.,* GITR, OX40 and ICOS, but not to others, e.g., PD-1. A developing understanding of the biology opens up new avenues for enhancing the anti-tumor activity of Fc fusion proteins that bind to, and alter the activity of, immunomodulatory targets on T cells, and enables predictions regarding which immunomodulatory receptors can be successfully targeted by the T_{reg} depletion mechanism.

### SUMMARY OF THE INVENTION

The present invention relates to afucosylated ipilimumab for use in treating cancer.

Further disclosed herein is a method for enhancing, optimizing or maximizing the anti-tumor efficacy of an Fc fusion protein which binds specifically to a target, *e.g.,* an immunomodulatory target, on a T cell in a subject afflicted with a cancer or a disease caused by an infectious agent and alters the activity of the target, thereby potentiating an endogenous immune response against cells of the cancer or the infectious agent, wherein the method comprises selecting, designing or modifying the Fc region of the Fc fusion protein so as to enhance the binding of said Fc region to an activating Fc receptor. According to the invention, the Fc fusion protein is an anti-CTLA-4 antibody. Other antibodies are, for example, an anti-GITR, anti-OX40, anti-ICOS or anti-CD 137 antibody. In preferred embodiments, the target is expressed on T_{regs} at the tumor site at a higher level than on T_{effs} at a tumor site.

Also disclosed herein is an Fc fusion protein that binds specifically to a target, *e.g.,* an immunomodulatory receptor protein, on a T cell in a subject afflicted with a cancer or a disease caused by an infectious agent and alters the activity of the target, thereby potentiating an endogenous immune response against cells of the cancer or the infectious agent, wherein the ability of the antibody to potentiate an endogenous immune response has been enhanced, optimized or maximized by a method comprising selecting, designing or modifying the Fc region of the Fc fusion protein so as to enhance the binding of said Fc region to an activating Fc receptor.

Further disclosed herein is a method for potentiating an endogenous immune response in a subject afflicted with a cancer or a disease caused by an infectious agent so as to thereby treat the subject, which method comprises administering to the subject a therapeutically effective amount of an Fc fusion protein, wherein the Fc region of the Fc fusion protein has been selected, designed or modified so as to enhance the binding of said Fc region to an activating Fc receptor.

Additionally disclosed herein is a method for immunotherapy of a subject afflicted with cancer or a disease caused by an infectious agent, which method comprises: (a) selecting a subject that is a suitable candidate for immunotherapy, the selecting comprising (i) assessing the presence of myeloid-derived suppressor cells (MDSCs) in a test tissue sample, and (ii) selecting the subject as a suitable candidate based on the presence of MDSCs in the test tissue sample; and (b) administering a therapeutically effective amount of an immunomodulatory Fc fusion protein to the selected subject.

Other features and advantages of the instant disclosure will be apparent from the following detailed description and examples, which should not be construed as limiting.

### BRIEF DESCRIPTION OF THE FIGURES

Figure 1 shows the binding of different isotypes of the mouse anti-mouse CTLA-4 antibody, 9D9, to CTLA-4⁺ cells.
Figure 2 shows a pharmacokinetic analysis of serum concentrations of anti-CTLA-4 isotypes in C57BL/6 mice.
Figure 3 shows the effects of different isotypes of the mouse anti-mouse CTLA-4 antibody, 9D9, on anti-tumor activity in a syngeneic CT26 adenocarcinoma mouse model: A, control mouse IgG (human anti-diphtheria toxin antibody with a mouse IgG1 isotype, also used as the control in other experiments); B, anti-CTLA-4-γ1D265A; C, anti-CTLA-4-γ2b; D, anti-CTLA-4-γ2a. The number of tumor-free (TF) mice per group is shown for each group of 10 mice.
Figure 4 shows an analysis of intratumoral T cells as a percentage of CD45⁺ cells in anti-CTLA-4 treated mice bearing CT26 tumors: A, CD8⁺ T cells, B, CD4⁺ T cells, C, T_{regs}.
Figure 5 shows the intratumoral ratios of CD8⁺ T_{effs} to T_{regs} (A), and CD4⁺ T_{effs} to T_{regs} (B), in anti-CTLA-4 treated mice bearing CT26 tumors.
Figure 6 shows an analysis of peripheral (lymph node) T_{regs} in anti-CTLA-4 treated mice bearing CT26 tumors.
Figure 7 shows the anti-tumor activity of four different mouse anti-CTLA-4 isotypes, as measured by changes in the tumor volumes in individual mice treated with these isotypes, in a MC38 colon adenocarcinoma tumor model: A, control mouse IgG1 antibody; B, anti-CTLA-4 IgG1; C, anti-CTLA-4 IgG1D265A; D, anti-CTLA-4 IgG2a; D, anti-CTLA-4 IgG2b.
Figure 8 shows the changes in mean tumor volumes (A) and median tumor volumes (B) of syngeneic MC38 colon adenocarcinoma tumors in groups of mice treated with mouse anti-CTLA-4 antibodies of different isotypes.
Figure 9 shows a flow cytometric analysis of MC38 tumor-infiltrating lymphocytes (TILs) from mice treated with specified anti-CTLA-4 antibodies. A, Percentage of CD45⁺ cells that are also CD4⁺; B, Percentage of CD45⁺ cells that are also CD8⁺; C, Percentage of CD4⁺ cells that are also Foxp3⁺.
Figure 10 shows CD8 and CD4 T_{eff} to T_{reg} ratios in MC38 tumors of mice treated with specified anti-CTLA-4 IgG isotypes. A, Ratio of CD8 T_{effs} to T_{regs} from TILs (CD8⁺ cells/CD4⁺ Foxp3⁺ cells); B, Ratio of CD4 T_{effs} to T_{regs} from TILs (CD4⁺ Foxp3⁻ cells/CD4⁺ Foxp3⁺ cells).
Figure 11 shows the anti-tumor activity of different mouse anti-CTLA-4 isotypes in a syngeneic Sa1N fibrosarcoma mouse model as measured by the changes in tumor volumes of individual mice treated with these isotypes: A, control mouse IgG1 antibody; B, anti-CTLA-4 IgG2a; C, anti-CTLA-4 IgG1D265A.
Figure 12 shows the changes in mean (A) and median tumor volumes (B) of syngeneic Sa1N fibrosarcoma tumors in groups of mice treated with mouse anti-CTLA-4 antibodies of different isotypes.
Figure 13 shows a flow cytometric analysis of Sa1N TILs from mice treated with specified anti-CTLA-4 antibodies. A, Percentage of CD45⁺ cells that are also CD8⁺; B, Percentage of CD45⁺ cells that are also CD4⁺; C, Percentage of CD4⁺ cells that are also Foxp3⁺.
Figure 14 shows CD8 and CD4 T_{eff} to T_{reg} ratios in syngeneic Sa1N tumor grafts of mice treated with specified anti-CTLA-4 IgG isotypes. A, Ratio of CD8 T_{effs} to T_{regs} from TILs (CD8⁺ cells/CD4⁺ Foxp3⁺ cells); B, Ratio of CD4 T_{effs} to T_{regs} from TILs (CD4⁺ Foxp3⁻ cells/CD4⁺ Foxp3⁺ cells).
Figure 15 shows isotype-dependent recruitment of MDSCs (A) and IL-1α production (B) in tumors of MC38 tumor-bearing mice treated with different anti-CTLA-4 antibody isotypes.
Figure 16 shows the effects of anti-CTLA-4 isotypes on intratumoral Th1/2 cytokine secretion. A, IFN-γ; B, IL-13; C, TNF-α; D, IL-10.
Figure 17 shows the effects of different isotypes of the rat anti-mouse GITR antibody, DTA-1, on anti-tumor activity as measured by changes in the tumor volumes in individual mice treated with these isotypes in a MC38 colon adenocarcinoma model: A, control mouse IgG1 antibody; B, anti-GITR mouse IgG1; C, anti-GITR rat IgG2b; D, anti-GITR mouse IgG2a. The number of TF mice per group is shown for each group of 10 mice.
Figure 18 shows the changes in mean (A) and median tumor volumes (B) of MC38 tumors in groups of mice treated with anti-GITR antibodies of different isotypes.
Figure 19 shows a flow cytometric analysis of spleens (A-C) and TILs (D-F) from MC38 tumor-bearing mice treated with the different anti-GITR (DTA-1) and anti-CTLA-4 (9D9) isotypes and control antibody indicated. A, Percentage of CD8⁺ T cells in spleen; B, Percentage of CD4⁺ cells in spleen; C, Percentage of CD4⁺ cells that are also Foxp3⁺ in spleen; D, Percentage of CD8⁺ T cells in TILs; E, Percentage of CD4⁺ cells in TILs; F, Percentage of CD4⁺ cells that are also Foxp3⁺ in TILs.
Figure 20 shows the effects of different isotypes of the rat anti-mouse GITR antibody, DTA-1, re-engineered to minimize aggregation, on anti-tumor activity as measured by changes in the tumor volumes in individual mice treated with these isotypes in a MC38 model: A, control mouse IgG1 antibody; B, anti-GITR mouse IgG1; C, anti-GITR mouse IgG1-D265A; D, anti-GITR mouse IgG2a; E, anti-GITR mouse IgG2b; F, anti-GITR rat IgG2b. The number of TF mice per group is shown for each group of 9 mice.
Figure 21 shows the changes in mean (A) and median tumor volumes (B) of MC38 tumors in groups of mice treated with re-engineered anti-GITR antibodies of different isotypes.
Figure 22 shows a flow cytometric analysis of the effects of different anti-GITR (reengineered "mGITR" DTA-1 or the originally engineered "DTA-1" antibodies) and anti-CTLA-4 (9D9) isotypes on Foxp3⁺/CD4⁺ T_{regs} in spleens (A) and TILs (B) from MC38 tumor-bearing mice.
Figure 23 shows the anti-tumor activity of different mouse anti-GITR isotypes in a Sa1N fibrosarcoma mouse model as measured by the changes in tumor volumes of individual mice treated with these isotypes: A, control mouse IgG1 antibody; B, anti-GITR mouse IgG2a; C, anti-GITR rat IgG2b; D, anti-GITR mouse IgG1; E, anti-GITR mouse IgG1-D265A. The number of TF mice per group is shown for each group of up to 10 mice.
Figure 24 shows the changes in mean (A) and median tumor volumes (B) of Sa1N tumors in groups of mice treated with anti-GITR (DTA-1) antibodies of different isotypes.
Figure 25 shows the effects of different anti-GITR (DTA-1) and anti-CTLA-4 (9D9) isotypes on Foxp3⁺/CD4⁺ T_{regs} in spleens (A) and TILs (B) from Sa1N tumor-bearing mice.
Figure 26 shows the effects of afucosylation of anti-CTLA-4 (9D9) antibodies on anti-tumor activity as measured by changes in the tumor volumes in individual mice treated with these antibodies in a MC38 tumor model: A, control mouse IgG1 antibody; B, anti-CTLA-4 IgG1D265A; C, anti-CTLA-4 IgG2b; D, nonfucosylated (NF) anti-CTLA-4 IgG2b; E, anti-CTLA-4 IgG2a; F, anti-CTLA-4 IgG2a-NF. The number of TF mice per group is shown for each group of 12 mice.
Figure 27 shows the changes in mean (A) and median tumor volumes (B) of MC38 tumors in groups of mice treated with anti-CTLA-4 antibodies of different isotypes and nonfucosylated variants.
Figure 28 shows the anti-tumor activity of different anti-OX40 isotypes in a syngeneic CT26 colon adenocarcinoma mouse model as measured by the changes in tumor volumes of individual mice treated with these isotypes: A, control mouse IgG1 antibody; B, anti-OX40 rat IgG1; C, anti-OX40 mouse IgG1; D, anti-OX40 mouse IgG2a. The number of TF mice per group is shown for each group of 10 mice.
Figure 29 shows the anti-tumor activity of different anti-OX40 isotypes in a staged syngeneic CT26 mouse tumor model as measured by the changes in tumor volumes of individual mice treated with these isotypes: A, control mouse IgG1 antibody; B, anti-OX40 mouse IgG1-D265A; C, anti-OX40 mouse IgG1; D, anti-OX40 mouse IgG2a. The number of TF mice per group is shown for each group of 8 mice in groups that contained any TF mice.
Figure 30 shows the anti-tumor activity of different isotypes of anti-ICOS Fc fusion proteins in a syngeneic Sa1N sarcoma mouse model as measured by the changes in tumor volumes of individual mice treated with these isotypes: A, control mouse IgG1 antibody; B, ICOSL-mouse IgG1 fusion protein; C, ICOSL-human IgGlfusion protein; D, rat IgG2b anti-mouse ICOS antibody, 17G9. The number of TF mice per group is shown for each group of up to 10 mice.
Figure 31 shows the effects of anti-mouse ICOS antibody, 17G9, on Foxp3⁺/CD4⁺ (A) and Foxp3⁺/CD45⁺ (B) T_{regs} compared to IgG1 control antibody in tumors from MC38 tumor-bearing mice.
Figure 32 shows a first study (Experiment #1) of the anti-tumor activity of different isotypes of an anti-mouse PD-1 antibody in a syngeneic MC38 tumor mouse model as measured by the changes in tumor volumes of individual mice treated with these isotypes: A, control mouse IgG1 antibody; B, anti-PD-1 IgG1; C, anti-PD-1 IgG1D265A; D, anti-PD-1 IgG2a. The number of TF mice per group is shown for each group of 11 mice.
Figure 33 shows the changes in mean (A) and median tumor volumes (B) of MC38 tumors in groups of mice treated with anti-PD-1 antibodies of different isotypes (Experiment #1).
Figure 34 shows a flow cytometric analysis of the effects of different anti-PD-1 antibody isotypes on the percentage of T cell subsets that infiltrate a MC38 tumor in tumor-bearing mice: A. CD8⁺ T_{effs}; B, CD4⁺ T_{effs}; C, FoxP3⁺/CD4⁺ T_{regs}.
Figure 35 shows the second study (Experiment #2) of the anti-tumor activity of different isotypes of an anti-mouse PD-1 antibody in a syngeneic MC38 tumor mouse model as measured by the changes in tumor volumes of individual mice treated with these isotypes: A, control mouse IgG1 antibody; B, anti-PD-1 IgG1; C, anti-PD-1 IgG1D265A; D, anti-PD-1 IgG2a. The number of TF mice per group is shown for each group of 11 mice.

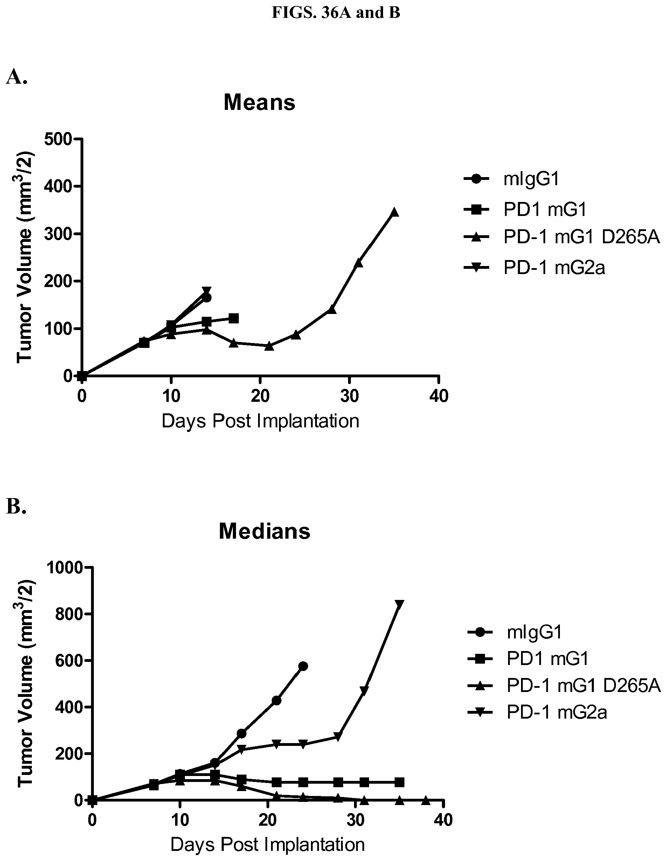

Figure 36 shows the changes in mean (A) and median tumor volumes (B) of MC38 tumors in groups of mice treated with anti-PD-1 antibodies of different isotypes (Experiment #2).

### DETAILED DESCRIPTION OF THE INVENTION

Certain aspects of the present disclosure relate to methods for enhancing optimizing or maximizing the anti-tumor efficacy of an Fc fusion protein, such as an antibody, which binds specifically to a target, *e,g.,* an immunomodulatory target such as CTLA-4, GITR or ICOS, on a T cell in a patient afflicted with cancer or an infectious disease. However, the target need not be an immunomodulatory target that is involved in regulating an immune response; more importantly, it is a target that is expressed at a high level on T_{regs} at the tumor site compared to the level of expression on T_{effs} at the tumor site. Additionally, the target is preferably expressed at a high level on T_{regs} at the tumor site compared to the level of expression on T_{regs} and T_{effs} in the periphery. In certain cases, the target is an immunomodulatory receptor or ligand and binding of the Fc fusion protein alters the activity of the target, thereby potentiating an endogenous immune response against cells of the cancer.

The disclosed methods comprise selecting, designing or modifying the Fc region of the Fc fusion protein so as to increase the binding of said Fc region to an activating Fc receptor (FcR). In certain cases, this increased binding to the activating FcR mediates a reduction of T_{regs} selectively at the tumor site, for example, by ADCC. This mechanism of action, involving the selective depletion of T_{regs} selectively at the tumor site, was first exemplified in a variety of mouse tumor models using anti-mouse CTLA-4 antibodies comprising variant Fc regions corresponding to different IgG isotypes. The mechanism was also shown to be operative with Fc fusion proteins that bind to other immunomodulatory receptors including the co-stimulatory receptors GITR, OX40 and ICOS. Thus, the present methods are not limited to anti-CTLA-4 antibodies but also apply to antibodies and other Fc fusion proteins that bind to diverse receptors including GITR, OX40 and ICOS. The underlying mechanism of this T_{reg} depletion phenomenon suggests that CD137 and TIGIT are also good targets, whereas certain receptors including, PD-1, LAG-3, TIM-3 and CD27 are unlikely to be suitable targets.

Disclosed herein is an Fc fusion protein that binds specifically to an immunomodulatory target on a T cell in a subject afflicted with a cancer or a disease caused by an infectious agent, the anti-tumor or anti-infectious agent activity of which Fc fusion protein has been enhanced by the methods disclosed herein.

Also disclosed herein are methods for potentiating an endogenous immune response in a patient afflicted with a cancer or a disease caused by an infectious agent so as to thereby treat the patient, which methods comprise administering to the patient a therapeutically effective amount of an anti-tumor or anti-infectious agent Fc fusion protein, wherein the ability of the Fc fusion protein to potentiate an endogenous immune response against cells of the cancer or anti-infectious agent has been enhanced by any of the methods disclosed herein.

### Terms

In order that the present disclosure may be more readily understood, certain terms are first defined. As used in this application, except as otherwise expressly provided herein, each of the following terms shall have the meaning set forth below. Additional definitions are set forth throughout the application.

"Administering" refers to the physical introduction of a composition comprising a therapeutic agent to a subject, using any of the various methods and delivery systems known to those skilled in the art. Preferred routes of administration for antibodies of the invention include intravenous, intraperitoneal, intramuscular, subcutaneous, spinal or other parenteral routes of administration, for example by injection or infusion. The phrase "parenteral administration" as used herein means modes of administration other than enteral and topical administration, usually by injection, and includes, without limitation, intravenous, intraperitoneal, intramuscular, intraarterial, intrathecal, intralymphatic, intralesional, intracapsular, intraorbital, intracardiac, intradermal, transtracheal, subcutaneous, subcuticular, intraarticular, subcapsular, subarachnoid, intraspinal, epidural and intrasternal injection and infusion, as well as *in vivo* electroporation. Alternatively, an antibody of the invention can be administered via a non-parenteral route, such as a topical, epidermal or mucosal route of administration, for example, intranasally, orally, vaginally, rectally, sublingually or topically. Administering can also be performed, for example, once, a plurality of times, and/or over one or more extended periods.

An "antibody" (Ab) shall include, without limitation, a glycoprotein immunoglobulin which binds specifically to an antigen and comprises at least two heavy (H) chains and two light (L) chains interconnected by disulfide bonds, or an antigen-binding portion thereof. Each H chain comprises a heavy chain variable region (abbreviated herein as V_{H}) and a heavy chain constant region. The heavy chain constant region comprises three domains, C_{H1}, C_{H2} and C_{H3}. Each light chain comprises a light chain variable region (abbreviated herein as V_{L}) and a light chain constant region. The light chain constant region is comprised of one domain, C_{L}. The V_{H} and V_{L} regions can be further subdivided into regions of hypervariability, termed complementarity determining regions (CDRs), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen.

Antibodies typically bind specifically to their cognate antigen with high affinity, reflected by a dissociation constant (K_{D}) of 10⁻⁵ to 10⁻¹¹ M⁻¹ or less. Any K_{D} greater than about 10⁻⁴ M⁻¹ is generally considered to indicate nonspecific binding. As used herein, an antibody that "binds specifically" to an antigen refers to an antibody that binds to the antigen and substantially identical antigens with high affinity, which means having a K_{D} of 10⁻⁷ M or less, preferably 10⁻⁸ M or less, even more preferably 5 x 10⁻⁹ M or less, and most preferably between 10⁻⁸ M and 10⁻¹⁰ M or less, but does not bind with high affinity to unrelated antigens. An antigen is "substantially identical" to a given antigen if it exhibits a high degree of sequence identity to the given antigen, for example, if it exhibits at least 80%, at least 90%, preferably at least 95%, more preferably at least 97%, or even more preferably at least 99 sequence identity to the sequence of the given antigen. By way of example, an antibody that binds specifically to human CTLA-4 may also have cross-reactivity with CTLA-4 antigens from certain primate species but may not cross-react with CTLA-4 antigens from certain rodent species or with an antigen other than CTLA-4, *e.g.,* a human PD-1 antigen.

The immunoglobulin may derive from any of the commonly known isotypes, including but not limited to IgA, secretory IgA, IgG and IgM. The IgG isotype may be divided in subclasses in certain species: IgG1, IgG2, IgG3 and IgG4 in humans, and IgG1, IgG2a, IgG2b and IgG3 in mice. "Isotype" refers to the antibody class (*e.g.,* IgM or IgG1) that is encoded by the heavy chain constant region genes. "Antibody" includes, by way of example, both naturally occurring and non-naturally occurring antibodies; monoclonal and polyclonal antibodies; chimeric and humanized antibodies; human or nonhuman antibodies; wholly synthetic antibodies; and single chain antibodies.

An "isolated antibody" refers to an antibody that is substantially free of other antibodies having different antigenic specificities (*e.g.,* an isolated antibody that binds specifically to CTLA-4 is substantially free of antibodies that bind specifically to antigens other than CTLA-4). An isolated antibody that binds specifically to CTLA-4 may, however, have cross-reactivity to other antigens, such as CTLA-4 molecules from different species. Moreover, an isolated antibody may be substantially free of other cellular material and/or chemicals. By comparison, an "isolated" nucleic acid refers to a nucleic acid composition of matter that is markedly different, *i.e.,* has a distinctive chemical identity, nature and utility, from nucleic acids as they exist in nature. For example, an isolated DNA, unlike native DNA, is a free-standing portion of a native DNA and not an integral part of a larger structural complex, the chromosome, found in nature. Further, an isolated DNA, unlike native DNA, can be used as a PCR primer or a hybridization probe for, among other things, measuring gene expression and detecting biomarker genes or mutations for diagnosing disease or predicting the efficacy of a therapeutic. An isolated nucleic acid may also be purified so as to be substantially free of other cellular components or other contaminants, *e.g.,* other cellular nucleic acids or proteins, using standard techniques well known in the art.

The phrases "an anti-antigen antibody", "an antibody recognizing an antigen", and "an antibody specific for an antigen" are used interchangeably herein with the term "an antibody which binds specifically to an antigen."

The term "monoclonal antibody" ("mAb") refers to a preparation of antibody molecules of single molecular composition, *i.e.,* antibody molecules whose primary sequences are essentially identical, and which exhibits a single binding specificity and affinity for a particular epitope. Monoclonal antibodies may be produced by hybridoma, recombinant, transgenic or other techniques known to those skilled in the art.

A "human" antibody (HuMAb) refers to an antibody having variable regions in which both the framework and CDR regions are derived from human germline immunoglobulin sequences. Furthermore, if the antibody contains a constant region, the constant region also is derived from human germline immunoglobulin sequences. The human antibodies of the invention may include amino acid residues not encoded by human germline immunoglobulin sequences (*e.g.,* mutations introduced by random or site-specific mutagenesis *in vitro* or by somatic mutation *in vivo*). However, the term "human antibody", as used herein, is not intended to include antibodies in which CDR sequences derived from the germline of another mammalian species, such as a mouse, have been grafted onto human framework sequences. The terms "human" antibodies and "fully human" antibodies and are used synonymously.

A "humanized" antibody refers to an antibody in which some, most or all of the amino acids outside the CDR domains of a non-human antibody are replaced with corresponding amino acids derived from human immunoglobulins. In one case of a humanized form of an antibody, some, most or all of the amino acids outside the CDR domains have been replaced with amino acids from human immunoglobulins, whereas some, most or all amino acids within one or more CDR regions are unchanged. Small additions, deletions, insertions, substitutions or modifications of amino acids are permissible as long as they do not abrogate the ability of the antibody to bind to a particular antigen. A "humanized" antibody retains an antigenic specificity similar to that of the original antibody.

A "chimeric antibody" refers to an antibody in which the variable regions are derived from one species and the constant regions are derived from another species, such as an antibody in which the variable regions are derived from a mouse antibody and the constant regions are derived from a human antibody.

An "antibody fragment" refers to a portion of a whole antibody, generally including the "antigen-binding portion" ("antigen-binding fragment") of an intact antibody which retains the ability to bind specifically to the antigen bound by the intact antibody, or the Fc region of an antibody which retains FcR binding capability.

"Antibody-dependent cell-mediated cytotoxicity" ("ADCC") refers to an *in vitro* or *in vivo* cell-mediated reaction in which nonspecific cytotoxic cells that express FcRs (e.g., natural killer (NK) cells, macrophages, neutrophils and eosinophils) recognize antibody bound to a surface antigen on a target cell and subsequently cause lysis of the target cell. In principle, any effector cell with an activating FcR can be triggered to mediate ADCC.

A "binding protein" refers to a protein that binds specifically to a particular moiety or target with high affinity. Examples of binding proteins include, but are not limited to, antibodies, antigen-binding fragments of an antibody, adnectins, minibodies, affibodies, affilins, the target-binding region of a receptor, cell adhesion molecules, ligands, enzymes, cytokines, and chemokines. In preferred cases of the present invention, a binding protein comprises an Fc region of an antibody.

A "cancer" refers a broad group of various diseases characterized by the uncontrolled growth of abnormal cells in the body. Unregulated cell division and growth divide and grow results in the formation of malignant tumors or cells that invade neighboring tissues and may also metastasize to distant parts of the body through the lymphatic system or bloodstream.

A "cell surface receptor" refers to molecules and complexes of molecules capable of receiving a signal and transmitting such a signal across the plasma membrane of a cell. Examples of cell surface receptors of the present disclosure include CTLA-4, GITR, OX40, ICOS, PD-1, CD127, TIGIT and FcRs.

An "effector cell" refers to a cell of the immune system that expresses one or more FcRs and mediates one or more effector functions. Preferably, the cell expresses at least one type of an activating Fc receptor, such as, for example, human FcγRIII and performs ADCC effector function. Examples of human leukocytes which mediate ADCC include peripheral blood mononuclear cells (PBMCs), NK cells, monocytes, macrophages, neutrophils and eosinophils.

An "effector function" refers to the interaction of an antibody Fc region with an Fc receptor or ligand, or a biochemical event that results therefrom. Exemplary "effector functions" include Clq binding, complement dependent cytotoxicity (CDC), Fc receptor binding, FcyR-mediated effector functions such as ADCC and antibody dependent cell-mediated phagocytosis (ADCP), and downregulation of a cell surface receptor (*e.g.,* the B cell receptor; BCR). Such effector functions generally require the Fc region to be combined with a binding domain (*e.g.,* an antibody variable domain).

An "Fc fusion protein," used interchangeably herein with a "binding protein comprising an Fc region," refers to a protein that includes within its structure a binding protein operably linked to an Fc region. The non-Fc part of an Fc fusion protein mediates target binding and is functionally analogous to, for example, the variable regions of an antibody. Well known examples of binding proteins comprising an Fc region include antibodies and immunoadhesins.

An "Fc receptor" or "FcR" is a receptor that binds to the Fc region of an immunoglobulin. FcRs that bind to an IgG antibody comprise receptors of the FcγR family, including allelic variants and alternatively spliced forms of these receptors. The FcγR family consists of three activating (FcγRI, FcγRIII, and FcγRIV in mice; FcγRIA, FcγRIIA, and FcγRIIIA in humans) and one inhibitory (FcγRIIB) receptor. Various properties of human FcγRs are summarized in Table 1. The majority of innate effector cell types coexpress one or more activating FcγR and the inhibitory FcγRIIB, whereas natural killer (NK) cells selectively express one activating Fc receptor (FcγRIII in mice and FcγRIIIA in humans) but not the inhibitory FcγRIIB in mice and humans.

An "Fc region" (fragment crystallizable region) or "Fc domain" or "Fc" refers to the C-terminal region of the heavy chain of an antibody that mediates the binding of the immunoglobulin to host tissues or factors, including binding to Fc receptors located on various cells of the immune system (*e.g.,* effector cells) or to the first component (C1q) of the classical complement system. Thus, the Fc region is a polypeptide comprising the constant region of an antibody excluding the first constant region immunoglobulin domain. In IgG, IgA and IgD antibody isotypes, the Fc region is composed of two identical protein fragments, derived from the second (C_{H2}) and third (C_{H2}) constant domains of the antibody's two heavy chains; IgM and IgE Fc regions contain three heavy chain constant domains (C_{H} domains 2-4) in each polypeptide chain. For IgG, the Fc region comprises immunoglobulin domains Cγ2 and Cγ3 and the hinge between Cγ1 and Cγ2. Although the boundaries of the Fc region of an immunoglobulin heavy chain might vary, the human IgG heavy chain Fc region is usually defined to stretch from an amino acid residue at position C226 or P230 to the carboxy-terminus of the heavy chain, wherein the numbering is according to the EU index as in Kabat. The C_{H2} domain of a human IgG Fc region extends from about amino acid 231 to about amino acid 340, whereas the C_{H3} domain is positioned on C-terminal side of a C_{H2} domain in an Fc region, *i.e.,* it extends from about amino acid 341 to about amino acid 447 of an IgG. As used herein, the Fc region may be a native sequence Fc or a variant Fc. Fc may also refer to this region in isolation or in the context of an Fc-comprising protein polypeptide such as a "binding protein comprising an Fc region," also referred to as an "Fc fusion protein" (*e.g.,* an antibody or immunoadhesin).

**Table 1. Properties of human FcγRs**

| Fcγ | Allelic variants | Affinity for human IgG | Isotype preference | Cellular distribution |
|---|---|---|---|---|
| FcγRI | None described | High (K_{D} ∼10 nM) | IgG1=3>4>>2 | Monocytes, macrophages, activated neutrophils, dentritic cells? |
| FcγRIIA | H131 | Low to medium | IgG1>3>2>4 | Neutrophils, monocytes, macrophages, eosinophils, dentritic cells, platelets |
| | R131 | Low | IgG1>3>4>2 | |
| FcγRIIIA | V158 | Medium | IgG1=3>>4>2 | NK cells, monocytes, macrophages, mast cells, eosinophils, dentritic cells? |
| | F158 | Low | IgG1=3>>4>2 | |
| FcγRIIB | 1232 | Low | IgG1=3=4>2 | B cells, monocytes, macrophages, dentritic cells, mast cells |
| | T232 | Low | IgG1=3=4>2 | |

A "hematological malignancy" includes a lymphoma, leukemia, myeloma or a lymphoid malignancy, as well as a cancer of the spleen and the lymph nodes. Exemplary lymphomas include both B cell lymphomas and T cell lymphomas. B-cell lymphomas include both Hodgkin's lymphomas and most non-Hodgkin's lymphomas. Non-limiting examples of B cell lymphomas include diffuse large B-cell lymphoma, follicular lymphoma, mucosa-associated lymphatic tissue lymphoma, small cell lymphocytic lymphoma (overlaps with chronic lymphocytic leukemia), mantle cell lymphoma (MCL), Burkitt's lymphoma, mediastinal large B cell lymphoma, Waldenström macroglobulinemia, nodal marginal zone B cell lymphoma, splenic marginal zone lymphoma, intravascular large B-cell lymphoma, primary effusion lymphoma, lymphomatoid granulomatosis. Non-limiting examples of T cell lymphomas include extranodal T cell lymphoma, cutaneous T cell lymphomas, anaplastic large cell lymphoma, and angioimmunoblastic T cell lymphoma. Hematological malignancies also include leukemia, such as, but not limited to, secondary leukemia, chronic lymphocytic leukemia, acute myelogenous leukemia, chronic myelogenous leukemia, and acute lymphoblastic leukemia. Hematological malignancies further include myelomas, such as, but not limited to, multiple myeloma and smoldering multiple myeloma. Other hematological and/or B cell- or T cell-associated cancers are encompassed by the term hematological malignancy.

An "immune response" refers to a biological response within a vertebrate against foreign agents, which response protects the organism against these agents and diseases caused by them. The immune response is mediated by the action of a cell of the immune system (for example, a T lymphocyte, B lymphocyte, natural killer (NK) cell, macrophage, eosinophil, mast cell, dendritic cell or neutrophil) and soluble macromolecules produced by any of these cells or the liver (including antibodies, cytokines, and complement) that results in selective targeting, binding to, damage to, destruction of, and/or elimination from the vertebrate's body of invading pathogens, cells or tissues infected with pathogens, cancerous or other abnormal cells, or, in cases of autoimmunity or pathological inflammation, normal human cells or tissues.

An "immunomodulator" or "immunoregulator" refers to a component of a signaling pathway that may be involved in modulating, regulating, or modifying an immune response. "Modulating," "regulating," or "modifying" an immune response refers to any alteration in a cell of the immune system or in the activity of such cell. Such modulation includes stimulation or suppression of the immune system which may be manifested by an increase or decrease in the number of various cell types, an increase or decrease in the activity of these cells, or any other changes which can occur within the immune system. Both inhibitory and stimulatory immunomodulators have been identified, some of which may have enhanced function in a tumor microenvironment. In preferred cases of the disclosure, the immunomodulator is located on the surface of a T cell. An "immunomodulatory target" or "immunoregulatory target" is an immunomodulator that is targeted for binding by, and whose activity is altered by the binding of, a substance, agent, moiety, compound or molecule. Immunomodulatory targets include, for example, receptors on the surface of a cell ("immunomodulatory receptors") and receptor ligands ("immunomodulatory ligands").

An "immunomodulatory Fc fusion protein" or "immunoregulatory Fc fusion protein" refers to an Fc fusion protein that binds to an immunomodulator and, as a result of this binding, either increases or inhibits the quantity or activity of the immunomodulator.

"Immunotherapy" refers to the treatment of a subject afflicted with, or at risk of contracting or suffering a recurrence of, a disease by a method comprising inducing, enhancing, suppressing or otherwise modifying an immune response.

"Potentiating an endogenous immune response" means increasing the effectiveness or potency of an existing immune response in a subject. This increase in effectiveness and potency may be achieved, for example, by overcoming mechanisms that suppress the endogenous host immune response or by stimulating mechanisms that enhance the endogenous host immune response.

A "protein" refers to a chain comprising at least two consecutively linked amino acid residues, with no upper limit on the length of the chain. One or more amino acid residues in the protein may contain a modification such as, but not limited to, glycosylation, phosphorylation or disulfide bond formation. The term "protein" is used interchangeable herein with "polypeptide."

A "signal transduction pathway" or a "signaling pathway" refers to two or more chemical agents and the biochemical relationship between them that play a role in the transmission of a signal from one cell to another cell, or from one portion of a cell to another portion of the cell.

A "subject" includes any human or nonhuman animal. The term "nonhuman animal" includes, but is not limited to, vertebrates such as nonhuman primates, sheep, dogs, rabbits, rodents such as mice, rats and guinea pigs, avian species such as chickens, amphibians, and reptiles. In preferred embodiments, the subject is a mammal such as a nonhuman primate, sheep, dog, cat, rabbit, ferret or rodent. In more preferred embodiments of any aspect of the disclosed invention, the subject is a human. The terms, "subject" and "patient" are used interchangeably herein.

A "therapeutically effective amount" or "therapeutically effective dosage" of a drug or therapeutic agent, such as an Fc fusion protein of the invention, is any amount of the drug that, when used alone or in combination with another therapeutic agent, promotes disease regression evidenced by a decrease in severity of disease symptoms, an increase in frequency and duration of disease symptom-free periods, or a prevention of impairment or disability due to the disease affliction. A therapeutically effective amount or dosage of a drug includes a "prophylactically effective amount" or a "prophylactically effective dosage", which is any amount of the drug that, when administered alone or in combination with another therapeutic agent to a subject at risk of developing a disease or of suffering a recurrence of disease, inhibits the development or recurrence of the disease. The ability of a therapeutic agent to promote disease regression or inhibit the development or recurrence of the disease can be evaluated using a variety of methods known to the skilled practitioner, such as in human subjects during clinical trials, in animal model systems predictive of efficacy in humans, or by assaying the activity of the agent in *in vitro* assays.

By way of example, an anti-cancer agent promotes cancer regression in a subject. In preferred embodiments, a therapeutically effective amount of the drug promotes cancer regression to the point of eliminating the cancer. "Promoting cancer regression" means that administering an effective amount of the drug, alone or in combination with an antineoplastic agent, results in a reduction in tumor growth or size, necrosis of the tumor, a decrease in severity of at least one disease symptom, an increase in frequency and duration of disease symptom-free periods, a prevention of impairment or disability due to the disease affliction, or otherwise amelioration of disease symptoms in the patient. In addition, the terms "effective" and "effectiveness" with regard to a treatment includes both pharmacological effectiveness and physiological safety. Pharmacological effectiveness refers to the ability of the drug to promote cancer regression in the patient. Physiological safety refers to the level of toxicity, or other adverse physiological effects at the cellular, organ and/or organism level (adverse effects) resulting from administration of the drug.

By way of example for the treatment of tumors, a therapeutically effective amount or dosage of the drug preferably inhibits cell growth or tumor growth by at least about 20%, more preferably by at least about 40%, even more preferably by at least about 60%, and still more preferably by at least about 80% relative to untreated subjects. In the most preferred embodiments, a therapeutically effective amount or dosage of the drug completely inhibits cell growth or tumor growth, *i.e.,* preferably inhibits cell growth or tumor growth by 100%. The ability of a compound to inhibit tumor growth can be evaluated in an animal model system, such as the CT26 colon adenocarcinoma, MC38 colon adenocarcinoma and Sa1N fibrosarcoma mouse tumor models described herein, which are predictive of efficacy in human tumors. Alternatively, this property of a composition can be evaluated by examining the ability of the compound to inhibit cell growth, such inhibition can be measured *in vitro* by assays known to the skilled practitioner. In other preferred embodiments of the invention, tumor regression may be observed and continue for a period of at least about 20 days, more preferably at least about 40 days, or even more preferably at least about 60 days.

"Treatment" or "therapy" of a subject refers to any type of intervention or process performed on, or administering an active agent to, the subject with the objective of reversing, alleviating, ameliorating, inhibiting, slowing down or prevent the onset, progression, development, severity or recurrence of a symptom, complication, condition or biochemical indicia associated with a disease.

### Effects of Isotype of Anti-CTLA-4 Antibodies on Anti-Tumor Efficacy and T Cell Subsets

Most therapeutic antibodies that have been commercialized are of the human IgG1 isotype, which can induce strong ADCC and CDC when compared with other human antibody isotypes. Additionally, therapeutic IgG1 antibodies have long-term stability in blood mediated via binding to the neonatal Fc receptor (FcRn). The activity of several therapeutic antibodies, including anti-CD20 rituximab (RITUXAN®) (Dall'Ozzo *et al.,* 2004)), anti-Her2 trastuzumab (HERCEPTIN®) (Gennari *et al.,* 2004), anti-tumor necrosis factor-α (anti-TNF-a) infliximab (REMICADE®) (Louis *et al.,* 2004), and anti-RhD (Miescher *et al.,* 2004) is mediated, at least in part, by ADCC. CDC is also considered a possible anti-tumor mechanism of rituximab (Idusogie *et al.,* 2000) and alemtuzumab (CAMPATH®) (Crowe *et al.,* 1992). Not surprisingly, therefore, efforts to improve the efficacy of therapeutic antibodies have recently focused on enhancement of effector functions, especially ADCC and CDC (Natsume *et al.,* 2009). Successful approaches have been reported, in particular, involving improving the binding activity of the Fc region of antibodies to FcγRIIIa or C1q by introducing amino acid mutations into the Fc regions or through modification of Fc-linked oligosaccharides.

However, in the case of agents, *e.g.,* antibodies, that bind to immunomodulatory targets on T cells and augment a T cell response, it is arguably undesirable to utilize an antibody that is cytotoxic to T cells via, for example, ADCC, CDC or ADCP, since this runs counter to the objective of boosting the numbers and activity of these T cells in upregulating the immune response. For example, U.S. Patent No. 6,682,736, which discloses the human anti-CTLA-4 antibody, tremelimumab, teaches that it is "not preferred to utilize an antibody that kills the cells," and it is instead desirable "to simply inhibit CTLA-4 binding with its ligands to mitigate T cell down regulation." The patent further identifies antibody isotypes, including human IgG1 and IgG3, that are capable of CDC, and other isotypes, including human IgG2 and IgG4, that do not mediate CDC. It further discloses that undesirable antibody isotypes, *e.g.,* human IgG1 or IgG3, can be switched to the desirable IgG2 or IgG4 isotype using conventional techniques well known in the art. Consistent with these teachings, U.S. Patent No. 6,682,736 also discloses that the majority of the CTLA-4 antibodies discussed therein, including tremelimumab, are of the desirable human IgG2 isotype, which can be readily isotype switched to generate the also desirable IgG4 isotype.

It is now recognized that CTLA-4 exerts its physiological function primarily through two distinct effects on the two major subsets of CD4⁺ T cells: (1) downmodulation of helper T cell activity, and (2) enhancement of the immunosuppressive activity of regulatory T cells (T_{regs}) (Lenschow *et al.,* 1996; Wing *et al.,* 2008; Peggs *et al.,* 2009). T_{regs} are known to constitutively express high levels of surface CTLA-4, and it has been suggested that this molecule is integral to their regulatory function (Takahashi *et al.,* 2000; Birebent *et al.,* 2004). Accordingly, the T_{reg} population may be most susceptible to the effects of CTLA-4 blockade.

CTLA-4 blockade results in a broad activation of immune responses that are dependent on helper T cells and, conversely, CTLA-4 engagement on T_{regs} enhances their suppressive function. Thus, in considering the mechanism of action for CTLA4 blockade, both enhancement of effector CD4⁺ T cell activity and inhibition of T_{reg}-dependent immunosuppression are probably important factors. One mechanism could be that CTLA-4 blockade acts directly on CD4⁺ and/or CD8⁺ cells to remove the inhibitory effects of CTLA-4 and thereby enhance effector functions. Alternatively, or additionally, the constitutive expression of CTLA-4 on T_{regs} suggests the possibility that the clinical effect of CTLA-4 blockade could be mediated by depletion or blockade of T_{regs}. In a study aimed at resolving these alternative mechanisms, Maker *et al.* (2005) concluded that the antitumor effects of CTLA-4 blockade are due to increased T cell activation rather than inhibition or depletion of T_{regs}. *See, also,* O'Mahony and Janik (2006) and Rosenberg (2006). However, another study to evaluate the independent contributions of CTLA-4 blockade of the T_{eff} or T_{reg} compartment concluded that the combination of direct enhancement of T_{eff} function and concomitant inhibition of T_{reg} activity through blockade of CTLA-4 on both cell types is essential for mediating the full therapeutic effects of anti-CTLA-4 antibodies during cancer immunotherapy (Peggs *et al.,* 2009).

One aspect of the present study evaluated the effect of isotype of a mouse anti-CTLA-4 antibody on the anti-tumor activity of the antibody in a variety of mouse tumor models. First, four variants of a mouse anti-mouse CTLA-4 antibody corresponding to the IgG1, a mutated IgG1D265A, the IgG2b, and the IgG2a isotypes, were generated and shown to bind equivalently to CTLA-4⁺ cells as well as to exhibit similar pharmacokinetic behavior in mouse serum (Example 1). Testing of the anti-tumor activity of the four isotypes of mouse anti-CTLA-4 in a CT26 colon adenocarcinoma tumor model revealed that the IgG2a treatment resulted in complete tumor rejection in 9 of 10 mice treated, whereas the IgG2b isotype produced moderate tumor growth inhibition, and the mutated IgG1D265A isotype showed minimal activity akin to control mouse IgG (Example 2).

The effects of the different anti-CTLA-4 isotypes on subset T cell populations in tumors and tumor draining lymph nodes were then evaluated in the CT26 colon adenocarcinoma mouse tumor model. Treatment of mice with anti-CTLA-4 antibodies resulted in an increase in the population of CD8⁺ cytotoxic T cells at the tumor site, with the greatest increases induced by the IgG2a and IgG2b isotypes (Example 4), while the IgG2a isotype caused a reduction in the population of CD4⁺ T helper cells. Marked differences among the treatment groups were observed regarding the effects on T_{regs}. Treatment with the IgG2a isotype dramatically decreased the population of T_{regs} at the tumor site, while IgG2b showed no change, and IgG1D265 resulted in increases in T_{reg} numbers. The increase in CD8⁺ T_{effs}, coupled with the decrease in T_{regs} mediated by the anti-CTLA-4 antibody having the IgG2a isotype, resulted in an elevated T_{eff} to T_{reg} ratio at the tumor site, which is indicative of potent anti-tumor activity.

In contrast to the changes in T cell subpopulations at the tumor site, all the anti-CTLA-4 isotypes behaved similarly in increasing the numbers of T_{regs} in the tumor draining lymph nodes (Example 4), which was not unexpected in view of earlier studies (Quezada *et al.,* 2006; Maker *et al.,* 2005; Rosenberg, 2006). Thus, the surprising result was the demonstration of T_{reg} loss selectively at the tumor site. This unexpected result was shown not to be a peculiarity of the CT26 tumor model, as the same pattern was also demonstrated in the MC38 colon adenocarcinoma mouse tumor model (Examples 5 and 6) and the immunogenic Sa1N fibrosarcoma tumor model (Examples 7 and 8). In both of these tumor models, the IgG2a isotype produced the most pronounced inhibitory effect on tumor growth, while mediating a marked increase in the percentage of CD8⁺ cells and a concomitant dramatic reduction in the level of T_{regs}. The same phenomena were also observed with other antibodies, including agonistic anti-GITR, OX40 and ICOS antibodies (Examples 10-16) but not with anti-PD-1 antibodies (Example 17). The molecular basis for this differential effect of certain IgG2a antibodies in mediating the depletion of T_{regs} at the tumor site versus inducing an increase in T_{reg} numbers in the lymph nodes can be elucidated from data disclosed herein. As described in Example 18, several T cell targets, including ICOS, GITR, OX40 and CD137 in addition to CTLA-4, are not only more highly expressed on T cells at the tumor site than in the periphery, but are also preferentially expressed on T_{regs} compared to expression levels on CD8 and CD4 T_{effs}. In addition, there is evidence of an increased presence of cells, *e.g.,* macrophages, expressing activating FcRs, particularly FcyRIV, at the tumor site compared to the periphery (Simpson *et al.,* 2013). Such cells have been shown to play a major role in the depletion of tumor infiltrating T_{regs} after anti-CTLA-4 antibody therapy (Simpson *et al.,* 2013). Thus, the mouse IgG2a isotype of a Fc fusion protein, *e.g.,* anti-CTLA-4, which binds to activating FcRs and mediates ADCC, is effective in depleting T cells that preferentially express the target of the antibody, *e.g.,* T_{regs} at the tumor site that differentially express high levels of CTLA-4 compared to expression levels on CD8 and CD4 T_{effs} at the tumor site.

Isotype differences in antibodies have been demonstrated to have profound effects on the biological activity of antibodies (Nimmerjahn and Ravetch, 2005; Nimmerjahn and Ravetch, 2008; Nimmerjahn and Ravetch, 2010). Anti-tumor activity of the TA99 antibody directed against the tumor-specific antigen, tyrosinase-related protein-1 (Tyrp1; gp75), was shown to require binding to the activating receptor FcRIV binding in the B16 murine melanoma model (Nimmerjahn and Ravetch, 2005). Subsequent investigations have reached contradictory conclusions, with Bevaart *et al.* (2006) finding a mandatory role for FcγRI, but no involvement of FcγRIII or FcγRIV, and Albanesi *et al.* (2012) concluding that FcγRI and FcγRIII contributed to TA99 therapeutic effects, whereas FcγRIV did not. Interestingly, the anti-tumor activity of anti-CTLA-4 in the CT26, MC38 and Sa1N tumor models described herein (Examples 2, 5 and 7) implies the requirement for activating Fc receptors for anti-tumor activity. Enhanced binding to activating receptor and reduced binding to the inhibitory receptor correlates with the anti-tumor activity of the anti-CTLA-4 isotypes, with the following hierarchy: mIgG2a >> mIgG2b >> mIgG1D265A. This hierarchy follows the activity ratio of the binding of immunoglobulin Fc regions to activating Fc receptors versus inhibitory Fc receptors (known as the A/I ration) defined by Nimmerjahn and Ravetch (2005) and determined for antibodies mediating ADCC function.

For anti-CTLA-4, maximal anti-tumor activity is achieved by the depletion or elimination of T_{regs} at the tumor site and the concomitant activation of T_{effs} (Examples 4, 6 and 8). Notably, although activated T cells express CTLA-4 these are not eliminated whereas T_{reg}, which are known to express higher, constitutive levels of anti-CTLA-4 (Read *et al.,* 2000; Takahashi *et al.,* 2000; Birebent *et al.,* 2004), are lost from the tumor site. Thus, the murine anti-CTLA-4 IgG2a isotype is able to maximally reduce T_{reg} numbers when compared to the other isotypes, while sparing activated T_{effs} which mediate the anti-tumor response. Accordingly, the IgG2a isotype is able to both enhance the activity of anti-tumor effector cells while also specifically reducing a population of cells which inhibit the anti-tumor response. Each of these T cell populations, which is differentially affected by the IgG2a anti-CTLA-4 antibody, is central to controlling tumor growth. The differential sensitivity of T_{effs} and T_{regs} to depletion is likely due to lower levels of CTLA-4 expressed at the cell surface of effector cells (*see* Example 18; *see, also* Selby *et al.,* 2013).

This result also suggests that the composition of cells at the tumor microenvironment and the Fc receptors they express are responsible for the anti-tumor activity of anti-CTLA-4. The observation that T_{regs} located specifically at the tumor site are reduced in number while those in the lymph node are activated by all isotypes of anti-CTLA-4 clearly demonstrates a tissue-specific difference in the activity of the different isotypes of anti-CTLA-4.

As noted, anti-CTLA-4-IgG2a, and to a lesser extent anti-CTLA-4-IgG2b, mediate the elimination or depletion of T_{regs} from the tumor site, consistent with their ability to bind to activating FcyRs. This occurs with the concomitant activation and expansion of CD8⁺ T_{effs} (and CD8⁺ T cells), which is likely mediated by inhibiting CTLA-4-B7 interactions. However, the data disclosed herein do not rule out that T_{eff} activation is solely a consequence of T_{reg} depletion. Thus, when compared with the other isotypes, the murine IgG2a isotype of anti-CTLA-4 is able to potently reduce T_{reg} numbers, while sparing activated T_{effs} that mediate the antitumor response. Indeed, the finding of augmented effector cytokine secretion (IFNγ, TNFα, and IL-13, and perhaps IL-10 (Emmerich *et al.,* 2012; Mumm *et al.,* 2011) at the tumor site is consistent with a loss of T_{reg} suppression and an increase in activated CD8 effectors.

The absence of antitumor activity of the IgG1 and IgG1-D265A isotypes in the therapeutic treatment of MC38 and CT26 models is also noteworthy. Inhibiting CTLA-4-B7 interactions with anti-CTLA-4 IgG1 or anti-CTLA-4 IgG1D265A leads to activation and expansion of T_{regs} in the periphery, while blockade of T_{effs} alone (*i.e.,* in the absence of T_{reg} elimination) is insufficient to promote a detectable antitumor response. In addition, blocking CTLA-4 on T_{regs}, while shown to diminish T_{reg} function (Quezada *et al.,* 2006; Onishi *et al.,* 2008) also does not appreciably enhance antitumor activity. In contrast, in a B16 melanoma model, using anti-CTLA-4 (hamster anti-mouse CTLA-4 9H10), GVAX therapy, and reconstitution of irradiated recipient mice with T cell subsets expressing either human or mouse CTLA-4, mouse CTLA-4 expression was required on both T_{effs} and T_{regs} for full antitumor activity (Peggs *et al.,* 2009). However, unlike the studies described herein, anti-CTLA-4 blockade targeted to T_{eff} only resulted in partial antitumor effects in this model.

### Effects of Isotype of Fc Fusion Proteins Other than Anti-CTLA-4 Antibodies on Anti-Tumor Efficacy

Following the demonstration that certain anti-CTLA-4 isotypes, especially the mouse IgG2a isotype, and to a lesser extent mouse IgG2b, mediate the elimination or depletion of T_{regs} from the tumor site, consistent with the ability of the Fc regions to bind to activating FcγRs and correlating with anti-tumor efficacy (Examples 1-7), the effect of isotype on the anti-tumor activity of additional antibodies and other Fc fusion proteins were investigated.

The anti-tumor activity of different anti-GITR isotypes was assessed in syngeneic MC38 colon carcinoma and Sa1N sarcoma mouse models (Examples 10-12). Similar to the results with anti-CTLA-4, it was demonstrated that the anti-GITR IgG1 and IgG1D265A isotypes have essentially no anti-tumor activity, whereas the mouse IgG2a and rat IgG2b (equivalent to mouse IgG2a in binding to mouse activating FcRs) isotypes induced the greatest inhibition of tumor growth (Example 11). The anti-GITR mG2a, mG2b and rG2b isotypes had little effect on, or induced small increases in T_{reg} populations in the periphery versus inducing significant T_{reg} depletion in the tumor environment, which correlated with tumor growth inhibition (Examples 11 and 12). Conversely, the mIgG2a isotype caused an increase in the percentage of CD8⁺ cells at the tumor site, whereas the mIgG1 and rat IgG2b caused no, or only a marginal increase in, the percentage of CD8⁺ cells. None of the isotypes had a major impact on the level of CD8⁺ cells in the periphery. Similar data have recently been reported by Bulliard *et al.* (2013).

Generally similar data were obtained with anti-OX40 isotypes tested in a syngeneic CT26 tumor mouse models (Example 14), and anti-ICOS isotypes tested in Sa1N and MC38 tumor models (Examples 15 and 16). However, assessment of anti-PD-1 isotypes in a MC38 tumor model showed that whereas the anti-PD-1 IgG2a isotype exhibited some anti-tumor activity, this was lower than the activity exhibited by the anti-IgG1 or IgG1D265A isotypes (Example 17). These results, wherein the anti-PD-1 IgG2a isotype did not potentiate anti-tumor activity relative to the IgG1 and IgG1D265A isotypes, were in stark contrast to the results obtained with anti-CTLA-4, GITR, OX40 and ICOS IgG2a antibodies. Furthermore, the anti-PD-1 IgG2a isotype caused a decrease in the percentage of CD8⁺ cells and an increase in the percentage of T_{regs} at the tumor site in contrast to the IgG1 and IgG1D265A isotypes, which induced small increases in CD8⁺ cells at the tumor site and induced smaller increases, relative to the IgG2a isotype, in the percentage of T_{regs} (Example 17).

A study of the expression levels of different receptor on T cell subsets at the tumor site and the periphery helps illuminate the underlying mechanisms. The data show that certain T cell receptors, including ICOS, GITR, CTLA-4, OX40, CD137, CTLA-4 and TIGIT are expressed at relatively high levels on T_{regs} at the tumor site compared to the expression levels on CD8 and CD4 T_{effs} at the tumor site. These receptors are also expressed at higher levels on the T cell subtypes at the tumor site compared to the expression levels on the same types of T cells in the periphery. Conversely, other receptors, including PD-1, LAG-3 and TIM-3 are expressed at higher levels on CD8 and/or CD4 T_{effs} at the tumor site compared to the expression levels on T_{regs} at the tumor site. CD27 shows fairly constant levels of expression on different cell types at the tumor site and in the periphery.

Thus, the mouse IgG2a isotype of a Fc fusion protein, *e.g.,* anti-CTLA-4, which binds to activating FcRs and mediates ADCC, is effective in depleting T cells that preferentially express the target of the antibody, *e.g.,* T_{regs} at the tumor site that differentially express high levels of CTLA-4 compared to expression levels on CD8 and CD4 T_{effs} at the tumor site.

It is noteworthy that the T_{regs} depletion mechanism for potentiating the antitumor efficacy of a Fc fusion protein such as an antibody is operative for both agonistic antibodies that bind to co-stimulatory receptors and antagonistic antibodies that bind to co-inhibitory receptors. The data indicate that any protein expressed on the surface of a T cell, irrespective of its function, can serve as a target for binding to an Fc fusion region that exhibits strong binding to activating FcRs, *e.g.,* IgG2a in mice or IgG1 in humans, for inducing ADCC-mediated depletion of the target cell. In the case of an anti-tumor Fc fusion protein, *e.g.,* an anti-tumor antibody, it is desirable that the target protein is differentially expressed on T_{regs} at the tumor site at a higher level than on T_{effs} at the tumor site, such that there is a selective net depletion of T_{regs} at the tumor site and a concomitant stimulation of the immune response. It is also desirable that the target protein is differentially expressed on T_{regs} at the tumor site at a higher level than on other T cells in the periphery so that the T_{reg} depletion component of stimulating the immune response is largely confined to the tumor site. Fc fusion proteins that target ICOS, GITR, CTLA-4, OX40, CD137, CTLA-4 and TIGIT are, therefore, good candidates for enhancing their anti-tumor efficacy by modifying the Fc region as described herein so as to enhance the binding of the Fc region to an activating FcR. An anti-CTLA-4 Fc fusion protein, e.g., an anti-CTLA-4 antibody, that binds specifically to CTLA-4 but does not block its co-inhibitory activity is a good candidate for engineering enhanced anti-tumor efficacy via the selection, design or modification of the Fc region so as to enhance the binding of said Fc region to an activating Fc receptor (FcR). Such an enhanced Fc fusion protein may exhibit high anti-tumor efficacy without some of the adverse effects of stimulating the immune response in the periphery.

Conversely, Fc fusion proteins that target PD-1, LAG-3, TIM-3 and CD27 are, unlikely to be good candidates for enhancing their anti-tumor efficacy by the methods disclosed described as these receptors are more highly expressed on T_{effs} than on T_{regs} at the tumor site. The data obtained with anti-PD-1 in Example 17 substantiate this view.

### Methods for Enhancing Anti-Tumor Efficacy of Immunomodulatory Fc Fusion Proteins

The data disclosed herein have implications for the activity of anti-CTLA-4 antibodies and other Fc fusion proteins that bind to immunomodulatory targets on T cells including, but not limited to, costimulatory and coinhibitory receptors and receptor ligands, in treating cancer patients. These data also suggest a potential design for higher-potency anti-immunoregulatory antibodies.

Ipilimumab, a human anti-human CTLA-4 monoclonal antibody, has been approved for the treatment of metastatic melanoma and is in clinical testing in other cancers (Hoos *et al.,* 2010; Hodi *et al.,* 2010; Pardoll, 2012a). Ipilimumab has a human IgG1 isotype, which binds best to most human Fc receptors (Table 1; Bruhns *et al.,* 2009) and is considered equivalent to murine IgG2a with respect to the types of activating Fc receptors that it binds. Since IgG1 binds to the activating receptor CD16 (FcγRIIIa) expressed by human NK cells and monocytes, ipilimumab can mediate ADCC. The IgG1-isotype ipilimumab was originally isolated directly from a hybridoma but was subsequently cloned and expressed in Chinese hamster ovary (CHO) cells. Notwithstanding the consideration that an isotype that mediates ADCC and/or CDC might be undesirable in an antibody targeting a receptor on T cells that seeks to upregulate an immune response, the IgG1 isotype of the antibody was retained, in part, because it enhanced vaccine response in cynomolgus monkey and was considered functional. Ipilimumab has been shown to increase the numbers of activated T cells in the blood, as evidenced, for example, by a significant increase in the expression of HLA-DR on the surface of post-treatment CD4⁺ and CD8⁺ cells as well as increases in absolute lymphocyte count (Ku *et al.,* 2010; Attia *et al.,* 2005; Maker *et al.,* 2005; Berman *et al.,* 2009; Hamid *et al.,* 2009), indicating that depletion of T cells does not occur in the periphery in man. Ipilimumab demonstrated only modest levels of ADCC of activated T cells using IL-2-activated PBMCs as effector cells (unpublished); however, use of T_{regs} as targets was not tested. Minor changes in peripheral T_{reg} frequency in the blood of patients treated with ipilimumab have been observed (Maker *et al.,* 2005), but little information of the effect of ipilimumab on intratumoral T_{regs} is available. However, a positive correlation between a high CD8⁺ to T_{reg} ratio and tumor necrosis in biopsies from metastatic melanoma lesions from patients treated with ipilimumab have been described (Hodi *et al.,* 2008). In addition, tumor tissue from ipilimumab-treated bladder cancer patients had lower percentages of CD4⁺ Foxp3⁺ T cells than tumors from untreated bladder cancer patients (Liakou *et al.,* 2008). These results are consistent with the data disclosed herein that ipilimumab mediates T_{reg} reduction at the tumor site.

In contrast, tremelimumab is an IgG2 isotype, which does not bind efficiently to Fc receptors, except for the FcγRIIa variant H131 (Bruhns *et al.,* 2009). While tremelimumab would have the ability to enhance T cell responses by blocking the inhibitory interactions between CTLA-4 and B7, the data disclosed herein suggests that tremelimumab may be limited in mediating depletion of T_{regs} at the tumor and, on that basis, is expected to exhibit reduced anti-tumor activity compared to ipilimumab. It is has been difficult to directly compare the clinical activity of these two antibodies as the dosing regimens for each have been different (*see, e.g.,* Ascierto *et al.,* 2011). Tremelimumab, like ipilimumab, has demonstrable anti-tumor activity (Ribas, 2010). Interestingly, studies on the mechanism of action of tremelimumab show, in a limited number of samples analyzed by immunohistochemistry, that increases in tumor-infiltrating CD8 T cells occur as a result of therapy, while there is no change in the number of Foxp3⁺ cells in the tumor after therapy (Comin-Anduix et al., 2008; Huang *et al.,* 2011). Alternatively, inhibition of T_{reg} function may be accomplished by blocking CTLA-4/B7 interaction.

Based on the experimental data disclosed herein relating to the mechanism of action of antibodies directed at targets on T cells, including anti-CTLA-4 , anti-GITR, anti-OX40 and anti-ICOS antibodies, and the effects of isotype on the activity of these antibodies, the present disclosure provides a method for enhancing, optimizing or maximizing the anti-tumor efficacy of an Fc fusion protein which binds specifically to an target on a T cell in a subject afflicted with a cancer or a disease caused by an infectious agent, wherein the method comprises modifying the Fc region of the Fc fusion protein so as to enhance the binding of said Fc region to an activating Fc receptor (FcR). Such enhanced binding of the antibody to an activating FcR has been shown, with certain targets, to result in depletion of T_{regs} in the tumor environment and increased anti-tumor activity of the antibody. In preferred cases, the target is an immunomodulatory receptor or ligand and binding of the Fc fusion protein alters the activity of the target, thereby potentiating an endogenous immune response against cells of the cancer.

Various aspects of the disclosed invention have been exemplified using anti-CTLA-4, anti-GITR, anti-OX40 and anti-ICOS antibodies. However, the methods disclosed herein are not limited to antibodies or to targeting of immunoregulatory receptors. Instead, the invention is applicable to a wide range of Fc fusion proteins that bind to diverse targets expressed on the surface of T cells. Thus, in certain cases, the Fc fusion protein comprises an Fc region operably linked to a binding protein such as, for example: an antigen-binding fragment of an antibody, including a single chain variable fragments (scFv), divalent or bivalent scFvs (di-scFvs or bi-scFvs), a diabody, a trivalent triabody or tetravalent tetrabody, a minibody or an isolated CDR (*see* Hollinger and Hudson, 2005; Olafsen and Wu, 2010, for further details); an adnectin; an affibody; an affilin; a ligand-binding region of a receptor; a cell adhesion molecule; a receptor ligand; an enzyme; a cytokine; or a chemokine. The use of Fc fusion proteins, such as smaller derivatives of the antigen-binding fragment of an antibody, that clear more rapidly from the circulation than intact antibodies may help mitigate the potential toxicity of an overactive immune response, and/or enable rapid removal of the inducing drug. In certain cases, the Fc fusion protein comprises an Fc region operably linked to a receptor ligand. In preferred cases, the Fc fusion protein is an antibody.

In certain aspects of the present methods, the antibody is of an IgG isotype. In other aspects, the antibody is a monoclonal antibody. In other aspects, the monoclonal antibody is a chimeric, humanized or human antibody. In certain preferred cases, the monoclonal antibody is a human IgG antibody. In other preferred cases, the binding of the human IgG antibody to an activating FcR is enhanced. The activating FcR may be an FcγI, FcγIIa or FcγIIIa receptor. In certain aspects, enhanced binding of the human IgG antibody to the FcγI, FcγIIa or FcγIIIa receptor results mediates a reduction of T_{regs} at the tumor site.

In certain cases of the present methods, enhanced binding of the human IgG antibody to an FcγI, FcγIIa or FcγIIIa receptor (a) does not mediate a reduction of, or (b) mediates an increase in, effector T cells (T_{effs}) at a tumor site. In certain preferred cases, the target is expressed on T_{regs} at the tumor site at a higher level than on T_{effs} at a tumor site. In other cases, the target is expressed on T_{regs} at the tumor site at a higher level than on T_{regs} or T_{effs} in the periphery.

Moreover, the Fc fusion protein employed in the methods disclosed herein may bind to a co-stimulatory or a co-inhibitory immunomodulatory target on a T cell. The Fc fusion protein used for binding specifically to a co-stimulatory target is an agonistic Fc fusion protein. For example, an agonist Fc fusion protein is used to target a co-stimulatory immunomodulator such as GITR, CD134 (OX40), ICOS, CD137 (4-1BB), CD27, CD28 or HVEM on a T cell. Binding of an agonists Fc fusion protein to a co-stimulatory immunomodulator results in upregulation of an immune response, in particular a T cell response. In certain cases of the present methods, the Fc fusion protein is an agonistic antibody that augments the activity of a co-stimulatory immunoregulator target on a T cell. In preferred cases, the co- stimulatory immunoregulator target is GITR, OX40, ICOS or CD137.

Conversely, the Fc fusion protein used for binding specifically to a co-inhibitory target is an inhibitory or antagonistic Fc fusion protein. For example, an inhibitory Fc fusion protein may be used to target a co-inhibitory immunomodulator such as, but not limited to, CTLA-4, PD-1, PD-L1, BTLA, TIM-3, LAG-3, A2aR, KLRG-1, CD244, CD160, or the VISTA receptor on a T cell. In certain cases of the present methods for enhancing the anti-tumor efficacy of an Fc fusion protein, the Fc fusion protein is an antagonistic antibody selected from an anti-CTLA-4 antibody, an anti-BTLA antibody, an anti-VISTA receptor antibody, an anti-A2aR antibody, an anti-KLRG-1 antibody, an anti-CD244 antibody, an anti-CD 160 antibody, and an anti-TIGIT antibody. In preferred cases, the Fc fusion protein is an antagonistic antibody that blocks the activity of a co-inhibitory immunoregulatory target on a T cell. In preferred cases, the co-inhibitory immunoregulatory target is CTLA or TIGIT. Binding of an antagonist Fc fusion protein to a co-inhibitory immunomodulator results in upregulation of an immune response, in particular, a T cell response.

In certain preferred cases, the antibody is an anti-CTLA-4 antibody. In further preferred cases, the anti-CTLA-4 antibody is ipilimumab or tremelimumab, or variants of these antibodies modified to enhance binding of the Fc region to an activating FcR. In other cases, the antibody is an anti-PD-1 antibody. In any of the methods of the disclosure, the subject is preferably a human.

Although an antibody that specifically targets T_{regs} has not yet been produced, many of the immune-checkpoint antibodies in clinical testing may enhance anti-tumor immunity by mechanisms involving blocking the immunosuppressive activity of T_{regs} (Pardoll, 2012b). As demonstrated herein, the IgG2a isotype of mouse anti-CTLA-4, an immune-checkpoint antibody, surprisingly and unexpectedly mediates a depletion of T_{regs} selectively at the tumor site while concomitantly mediating an increase in T_{regs} in tumor draining lymph nodes and an increase in intratumoral CD8⁺ T_{effs} (Examples 4, 6 and 8). Whereas T_{regs} may be targeted by immunomodulatory Fc fusion proteins, in certain aspects of the disclosed invention, the targeted cell is a different type of suppressive cell other than a T_{reg}. Similarly, whereas Fc fusion proteins of the disclosure may target co-inhibitory immunomodulators such as CTLA-4, in certain aspects of the disclosure, the targeted immunomodulator is a co-stimulatory immunomodulator such as GITR, OX40, CD137 or ICOS. Accordingly, in certain cases, the Fc fusion protein, *e.g.,* an agonistic or antagonistic antibody, binds to an immunomodulatory target, be it a co-stimulatory or co-inhibitory target, expressed on a suppressive cell population and mediates a depletion or elimination of that cell population. In preferred cases, the binding of the Fc fusion protein to the immunomodulatory target expressed on a protective cell population enhances the activity of, or has no deleterious effect on, the protective cell population expressing the immunomodulatory target. In certain cases, the target on a T_{reg} or other immunosuppressive T cell bound by the Fc fusion protein is not an immunomodulatory target.

In clinical trials of immunotherapeutic drugs that target immune checkpoints, durable clinical responses have been observed, even in heavily pretreated patients, across multiple tumor types including a substantial proportion of melanoma (MEL), renal cell carcinoma (RCC), squamous non-small cell lung cancer (NSCLC), and non-squamous NSCLC patients and in various sites of metastasis including liver, lung, lymph nodes, and bone (*see, e.g.,* Pardoll, 2012b; Topalian *et al.,* 2012; Brahmer et al., 2012; Mellman *et al.,* 2011; Flies *et al.,* 2011). In certain embodiments of the present invention, the cancer against which the efficacy of an Fc fusion protein is enhanced is selected from bone cancer, pancreatic cancer, skin cancer, cancer of the head or neck, breast cancer, lung cancer, cutaneous or intraocular malignant melanoma, renal cancer, uterine cancer, ovarian cancer, colorectal cancer, colon cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, solid tumors of childhood, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally induced cancers including those induced by asbestos, hematologic malignancies including, for example, multiple myeloma, B-cell lymphoma, Hodgkin lymphoma/primary mediastinal B-cell lymphoma, non-Hodgkin's lymphomas, acute myeloid lymphoma, chronic myelogenous leukemia, chronic lymphoid leukemia, follicular lymphoma, diffuse large B-cell lymphoma, Burkitt's lymphoma, immunoblastic large cell lymphoma, precursor B-lymphoblastic lymphoma, mantle cell lymphoma, acute lymphoblastic leukemia, mycosis fungoides, anaplastic large cell lymphoma, T-cell lymphoma, and precursor T-lymphoblastic lymphoma, and any combinations of said cancers. The present invention is also applicable to treatment of metastatic cancers. In certain preferred embodiments, the cancer is selected from MEL, RCC, squamous NSCLC, non-squamous NSCLC, colorectal cancer (CRC), castration-resistant prostate cancer (CRPC), squamous cell carcinoma of the head and neck, and carcinomas of the esophagus, ovary, gastrointestinal tract and breast, ovarian cancer, gastric cancer, hepatocellular carcinoma, pancreatic carcinoma, and a hematological malignancy. In other preferred embodiments, the cancer is MEL. In other preferred embodiments, the cancer is RCC. In yet other preferred embodiments, the cancer is squamous NSCLC. In still other preferred embodiments, the cancer is non-squamous NSCLC.

The results disclosed herein clearly indicate that the clinical activity of Fc fusion proteins targeting immunomodulatory targets on T cells can be enhanced for use in human patients. Several strategies are available for increasing ADCC and CDC effector functions of Fc fusion proteins for cancer treatment, and particularly, for increasing the binding of IgG1 antibodies to FcRγIIIa for both V and F allotypes (*see, e.g.,* Natsume *et al.,* 2009). Indeed, engineering therapeutic antibodies with the aim of improving specific binding to FcγIIIA and thereby enhancing ADCC is expected to play a key role in the development of next-generation therapeutic antibodies with improved clinical efficacy (Natsume *et al.,* 2009; Albanesi *et al.,* 2012). But while the importance of effector functions such as ADCC, ADCP and CDC for the clinical efficacy of therapeutic Fc fusion proteins is now widely recognized, it was hitherto believed that such effector functions were undesirable in strategies to upregulate a T cell response using Fc fusion proteins that bind to immunomodulatory targets on T cells (*see, e.g.,* U.S. Patent No. 6,682,736).

One approach to engineering human therapeutic Fc fusion proteins is to introduce into the IgG1 Fc region one or more mutations that enhance binding to an activating FcγR (Nimmerjahn and Ravetch, 2012). For example, an IgG1 triple mutant (S298A/E333A/L334A) has been shown to exhibit enhanced FcγRIIIa binding and ADCC activity (Shields *et al.,* 2001). Other IgG1 variants with strongly enhanced binding to FcγRIIIa have been identified, including variants with S239D/I332E and S239D/I332E/A330L mutations which showed the greatest increase in affinity for FcγRIIIa, a decrease in FcyRIIb binding, and strong cytotoxic activity in cynomolgus monkeys (Lazar *et al.,* 2006). Introduction of the triple mutations into antibodies such as alemtuzumab (CD52-specific), trastuzumab (HER2/neu-specific), rituximab (CD20-specific), and cetuximab (EGFR-specific) translated into greatly enhanced ADCC activity *in vitro,* and the S239D/I332E variant showed an enhanced capacity to deplete B cells in monkeys (Lazar *et al.,* 2006). In addition, IgG1 mutants containing L235V, F243L, R292P, Y300L and P396L mutations which exhibited enhanced binding to FcγRIIIa and concomitantly enhanced ADCC activity in transgenic mice expressing human FcγRIIIa in models of B cell malignancies and breast cancer have been identified (Stavenhagen *et al.,* 2007; Nordstrom *et al.,* 2011).

Fc regions can also be mutated to increase the affinity of IgG for the neonatal Fc receptor, FcRn, which prolongs the *in vivo* half-life of antibodies and results in increased anti-tumor activity. For example, introduction of M428L/N434S mutations into the Fc regions of bevacizumab (VEGF-specific) and cetuximab (EGFR-specific) increased antibody half-life in monkeys and improved anti-tumor responses in mice (Zalevsky *et al.,* 2010).

The interaction of antibodies with FcγRs can also be enhanced by modifying the glycan moiety attached to each Fc fragment at the N297 residue. In particular, the absence of branching fucose residues strongly enhances ADCC via improved binding of IgG to activating FcγRIIIA without altering antigen binding or CDC (Natsume *et al.,* 2009). There is convincing evidence that afucosylated tumor-specific antibodies translate into enhanced therapeutic activity in mouse models *in vivo* (Nimmerjahn and Ravetch, 2005; Mossner *et al.,* 2010; *see* Example 13).

Modification of antibody glycosylation can be accomplished by, for example, expressing the antibody in a host cell with altered glycosylation machinery. Cells with altered glycosylation machinery have been described in the art and can be used as host cells in which to express recombinant antibodies of this disclosure to thereby produce an antibody with altered glycosylation. For example, the cell lines Ms704, Ms705, and Ms709 lack the fucosyltransferase gene, FUT8 (α-(1,6) fucosyltransferase; *see* U.S. Publication No. 20040110704; Yamane-Ohnuki *et al.,* 2004), such that antibodies expressed in these cell lines lack fucose on their carbohydrates. As another example, EP 1176195 also describes a cell line with a functionally disrupted FUT8 gene as well as cell lines that have little or no activity for adding fucose to the N-acetylglucosamine that binds to the Fc region of the antibody, for example, the rat myeloma cell line YB2/0 (ATCC CRL 1662). PCT Publication WO 03/035835 describes a variant CHO cell line, Lec13, with reduced ability to attach fucose to Asn(297)-linked carbohydrates, also resulting in hypofucosylation of antibodies expressed in that host cell (*see, also,* Shields, *et al.,* 2002). Antibodies with a modified glycosylation profile can also be produced in chicken eggs, as described in PCT Publication No. WO 2006/089231. Alternatively, antibodies with a modified glycosylation profile can be produced in plant cells, such as *Lemna* (*see, e.g.,* U.S. Publication No. 2012/0276086. PCT Publication No. WO 99/54342 describes cell lines engineered to express glycoprotein-modifying glycosyl transferases (e.g., beta(1,4)-N-acetylglucosaminyltransferase III (GnTIII)) such that antibodies expressed in the engineered cell lines exhibit increased bisecting GlcNac structures which results in increased ADCC activity of the antibodies (*see, also,* Umaña *et al.,* 1999). Alternatively, the fucose residues of the antibody may be cleaved off using a fucosidase enzyme. For example, the enzyme alpha-L-fucosidase removes fucosyl residues from antibodies (Tarentino *et al.,* 1975).

The binding of the C1q component of the complement cascade to the Fc region of cell-bound antibodies also affects the intensity of the subsequent complement activation, and several approaches have succeeded in enhancing CDC by enhancing the binding of the Fc region to C1q. Strategies used include engineering amino acid mutations into the Fc or hinge region, or shuffling IgG1 and IgG3 sequences within a heavy chain constant region (Natsume *et al.,* 2009).

The *in vivo* data disclosed herein indicate that when a variety of Fc fusion proteins (antibodies) that target immunoregulatory receptors on T cells and enhance a T cell response are modified to increase binding of IgG1 to FcγRIIIa, enhanced anti-tumor activity results. Any of the methods disclosed herein for increasing binding of IgG1 to FcγRIIIa may be employed. In one case of the present methods, the Fc fusion protein is not an IgG1 isotype and the modification of the Fc region converts the Fc fusion protein to an IgG1 isotype. By way of example, the anti-tumor efficacy of tremelimumab, an IgG2 anti-CTLA-4 antibody, may be enhanced by a method comprising modifying the Fc region of these antibodies to generate an IgG1 isotype which exhibits increased binding of the modified Fc region to FcγRIIIa. Based on the data disclosed herein, such a modification mediates depletion of T_{regs} at the tumor site and a concomitant increase in CD8⁺ CTLs, resulting in enhanced anti-tumor efficacy.

In another case, the selection, design or modification of the Fc region results in hypofucosylation or nonfucosylation of the Fc region. In yet another case, the selection, design or modification of the Fc region comprises at least one amino substitution that results in enhanced binding of the Fc region to an activating FcR receptor. Thus, for example, certain cases of the present method comprises selecting, designing or modifying the Fc region to include amino acid mutations selected from S298A/E333A/L334A, S239D/I332E, S239D/I332E/A330L, L235V/F243L/R292P/Y300L/ P396L, and M428L/N434S mutations.

By way of example, the anti-tumor efficacy of the IgG1 isotypes of tremelimumab, which may be enhanced by isotype switching as described above, may be further enhanced by a method comprising modifying the Fc region so as to introduce at least one amino substitution that results in enhanced binding of the Fc region to FcγRIIIa, and/or modifying the Fc region to generate a hypofucosylated or nonfucosylated Fc region which exhibits increased binding to FcγRIIIa.

### Anti-Immunomodulator Fc Fusion Proteins

Fc fusion proteins of this disclosure are binding proteins comprising an Fc region that bind specifically and with high affinity to a target on a T cell. In preferred cases, the Fc fusion protein binds to an immunomodulatory target that is expressed at a high level on T_{regs} in the tumor environment relative to the expression level on on T_{regs} in the periphery and on T_{effs}. In certain cases, the anti-immunomodulator Fc fusion protein is an anti-CTLA-4 binding protein, *i.e.,* it binds specifically to CTLA-4. In preferred cases, the anti-CTLA-4 binding protein is a blocking antibody. In other certain cases, the immunomodulator Fc fusion protein is an anti-GITR, anti-OX40 or anti-ICOS binding protein, *i.e.,* it binds specifically to GITR, OX40 or ICOS. In preferred cases, the anti-GITR, anti-OX40 or ICOS binding protein is an agonistic antibody.

Monoclonal antibodies that recognize and bind to the extracellular domain of CTLA-4 are described in U.S. Patent No. 5,977,318. Human monoclonal antibodies of this disclosure can be generated using various methods, for example, using transgenic or transchromosomic mice carrying parts of the human immune system rather than the mouse system, or using *in vitro* display technologies such as phage or yeast display (*see, e.g.,* Bradbury *et al.,* 2011). Transgenic and transchromosomic mice include mice referred to herein as the HUMAB MOUSE® (Lonberg *et al.,* 1994) and KM MOUSE® (WO 02/43478), respectively. The production of exemplary human anti-human CTLA-4 antibodies of this disclosure is described in detail in U.S. Patent Nos. 6,984,720 and 7,605,238. The human IgG1 anti-CTLA-4 antibody identified as 10D1 in these patents is also known as ipilimumab (also formerly known as MDX-010 and BMS-734016), which is marketed as YERVOY®. Other exemplary human anti-CTLA-4 antibodies of this disclosure are described in U.S. Patent No. 6,682,736, including tremelimumab (formerly ticilimumab; CP-675,206), a human IgG2 anti-human CTLA-4 antibody.

Anti-GITR Fc fusion proteins that may be enhanced by the method disclosed herein include the antibodies disclosed in PCT Publication Nos. WO 2006/105021 and WO2011/028683, and Japanese Publication No. 2008278814.

Anti-ICOS Fc fusion proteins that may be enhanced by the method disclosed herein include the antibodies disclosed in U.S. Patent Nos. 7,030,225, 7,932,358, 6,803,039 and 7,722,872 and U.S. Publication No. 2013/0142783.

Anti-OX40 Fc fusion proteins that may be enhanced by the method disclosed herein include the antibodies disclosed in PCT Publication Nos. WO 95/12673, WO 99/42585, WO 03/106498, WO 2007/062245, WO 2009/079335, WO 2010/096418, WO 2012/027328, WO 2013/028231, WO 2013/008171 and WO 2013/038191.

Disclosed herein are methods for enhancing the anti-tumor efficacy of an Fc fusion protein, which comprise modifying the Fc region of the Fc fusion protein so as to increase the binding of said Fc region to an activating Fc receptor. Also disclosed herein are Fc fusion proteins that bind specifically to a co-inhibitory or a co-stimulatory immunomodulatory receptor on a T cell in a subject afflicted with cancer or a disease caused by an infectious agent and blocks the activity of the co-inhibitory receptor or enhances the activity of the co-stimulatory receptor, thereby potentiating an endogenous immune response against cancer cells or the infectious agent, wherein the ability of the antibody to potentiate an endogenous immune response has been enhanced using the methods disclosed herein. In preferred cases, the Fc fusion protein that exhibits an enhanced ability to potentiate an immune response is an antibody (an "enhanced antibody"). In certain cases, this antibody is an IgG isotype. In certain other cases, the enhanced antibody is a monoclonal antibody. In further cases, the enhanced antibody is a chimeric, humanized or human antibody.

In certain aspects of this disclosure, the enhanced Fc fusion protein is a human IgG antibody. In certain cases of this human IgG antibody, binding to an FcγI, FcγIIa or FcγIIIa receptor is enhanced. In other cases, such enhanced binding to the FcγI, FcγIIa or FcγIIIa receptor results in increased ADCC. In certain preferred cases, enhanced binding of the modified Fc to the FcγI, FcγIIa or FcγIIIa receptor mediates a reduction of T_{regs} at the tumor site. This reduction of T_{regs} may be mediated by ADCC or a different mechanism that differentially reduces T_{regs} at the tumor site but not in the periphery.

In certain cases of the instant Fc fusion protein, enhanced binding of the human IgG antibody to an FcγI, FcγIIa or FcγIIIa receptor mediates a reduction in T_{regs} at a tumor site. In other cases, enhanced binding of the human IgG antibody to an FcγI, FcγIIa or FcγIIIa receptor (a) does not mediate a reduction of, or (b) mediates an increase in, T_{effs} at a tumor site. In preferred cases, the target is expressed on T_{regs} at a tumor site at a higher level than on T_{effs} at the tumor site. In further cases, the target is expressed on T_{regs} at a tumor site at a higher level than on T_{regs} or T_{effs} in the periphery. In certain other cases, the Fc fusion protein is an antagonistic antibody that blocks the activity of a co-inhibitory immunoregulatory target on a T cell. In further cases, the co-inhibitory immunoregulatory target is CTLA or TIGIT.

In certain other aspects of this disclosure, the enhanced Fc fusion protein is an agonistic antibody that augments the activity of a co-stimulatory immunoregulatory target on a T cell. In further cases, the co-stimulatory immunoregulatory target is GITR, OX40, ICOS, CD137. In other cases, the enhanced antibody is an anti-CTLA-4 antibody, an anti-GITR antibody, an anti-OX40 antibody, an anti-ICOS receptor antibody, an anti-KLRG-1 antibody, an anti-CD244 antibody, an anti-CD 160 antibody, or an anti-TIGIT antibody. In certain preferred cases, the enhanced antibody is an anti-CTLA-4 antibody, an anti-GITR antibody, an anti-OX40 antibody, an anti-ICOS receptor antibody. The anti-CTLA-4 antibody may be, for example, an enhanced variant of ipilimumab or an enhanced variant of tremelimumab. In certain cases, such enhanced variants are afucosylated or hypofucosylated variants of ipilimumab or tremelimumab. In other cases, such enhanced variants of ipilimumab or tremelimumab comprise amino acid mutations selected from S298A/E333A/L334A, S239D/I332E, S239D/I332E/A330L, L235V/F243L/R292P/Y300L/ P396L, and M428L/N434S mutations.

In other aspects of this disclosure, the enhanced Fc fusion protein is an antagonistic antibody that augments the activity of a co-inhibitory immunoregulatory target on a T cell. In certain cases, the antibody selected from an anti-CTLA-4 antibody, an anti-BTLA antibody, an anti-VISTA receptor antibody, an anti-A2aR antibody, an anti-KLRG-1 antibody, an anti-CD244 antibody, an anti-CD160 antibody, and an anti-TIGIT antibody. In preferred cases, the antibody is an anti-CTLA-4 antibody or an anti-TIGIT antibody.

In certain other aspects of the enhanced Fc fusion protein, the subject is afflicted with a cancer selected from bone cancer, pancreatic cancer, skin cancer, cancer of the head or neck, breast cancer, lung cancer, cutaneous or intraocular malignant melanoma, renal cancer, uterine cancer, ovarian cancer, colorectal cancer, colon cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, solid tumors of childhood, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally induced cancers including those induced by asbestos, hematologic malignancies including, for example, multiple myeloma, B-cell lymphoma, Hodgkin lymphoma/primary mediastinal B-cell lymphoma, non-Hodgkin's lymphomas, acute myeloid lymphoma, chronic myelogenous leukemia, chronic lymphoid leukemia, follicular lymphoma, diffuse large B-cell lymphoma, Burkitt's lymphoma, immunoblastic large cell lymphoma, precursor B-lymphoblastic lymphoma, mantle cell lymphoma, acute lymphoblastic leukemia, mycosis fungoides, anaplastic large cell lymphoma, T-cell lymphoma, and precursor T-lymphoblastic lymphoma, and any combinations of said cancers. The present invention is also applicable to treatment of metastatic cancers. In preferred embodiments, the cancer is selected from MEL, RCC, squamous NSCLC, non-squamous NSCLC, CRC, CRPC, squamous cell carcinoma of the head and neck, carcinomas of the esophagus, ovary, gastrointestinal tract and breast, ovarian cancer, gastric cancer, hepatocellular carcinoma, pancreatic carcinoma, and a hematological malignancy.

### Nucleic Acid Molecules Encoding Antibodies of the Invention

Another aspect of the present disclosure pertains to isolated nucleic acid molecules that encode any of the Fc fusion proteins of the disclosure that bind to targets, e.g., immunomodulatory receptors or ligands, on T cells. In preferred embodiments, these isolated nucleic acid molecules encode antibodies that target and block inhibitory immunomodulatory receptors. The nucleic acids may be present in whole cells, in a cell lysate, or in a partially purified or substantially pure form. The nucleic acid can be, for example, DNA or RNA, and may or may not contain intronic sequences. In certain embodiments, the DNA is genomic DNA, cDNA, or synthetic DNA, *i.e.,* DNA synthesized in a laboratory, *e.g.,* by the polymerase chain reaction or by chemical synthesis. In preferred embodiments, the nucleic acid is a cDNA.

Nucleic acids of the disclosure can be obtained using standard molecular biology techniques. Disclosed herein is an isolated nucleic acid encoding any of the Fc fusion proteins disclosed herein. Also disclosed herein is an expression vector comprising said isolated nucleic acid. Further disclosed herein is a host cell comprising any of the disclosed expression vectors. Such a host cell may be used for producing any of the Fc fusion proteins described herein using methods well known in the art, *e.g.,* by culturing the host cells for a period of time sufficient to allow for expression of the Fc fusion protein in the host cells or, more preferably, secretion of the antibody into the culture medium in which the host cells are grown. The Fc fusion protein can be recovered from the culture medium using standard protein purification methods that are well known in the art.

### Pharmaceutical Compositions

Fc fusion proteins of the present invention may be constituted in a composition, *e.g.,* a pharmaceutical composition, containing the binding protein, for example an antibody or a fragment thereof, and a pharmaceutically acceptable carrier. As used herein, a "pharmaceutically acceptable carrier" includes any and all solvents, dispersion media, coatings, antibacterial and antifungal agents, isotonic and absorption delaying agents, and the like that are physiologically compatible. Preferably, the carrier is suitable for intravenous, subcutaneous, intramuscular, parenteral, spinal or epidermal administration (*e.g.,* by injection or infusion). A pharmaceutical composition of the invention may include one or more pharmaceutically acceptable salts, anti-oxidant, aqueous and nonaqueous carriers, and/or adjuvants such as preservatives, wetting agents, emulsifying agents and dispersing agents.

Dosage regimens are adjusted to provide the optimum desired response, *e.g.,* a therapeutic response or minimal adverse effects. Typically, the dosage of an enhanced antibody of the disclosure required to achieve a certain level of anti-cancer efficacy is lower than for the unmodified antibody. Further, such a lower dosage typically results in a lower incidence or severity of adverse effects. For administration of an antibody of the disclosure that binds specifically to a target on a T cell, the dosage ranges from about 0.00001 to about 100 mg/kg, usually from about 0.0001 to about 20 mg/kg, and more usually from about 0.001 to about 10 mg/kg, of the subject's body weight. Preferably, the dosage is within the range of 0.01-10 mg/kg body weight. For example, dosages can be 0.01, 0.05, 0.1, 0.3, 1, 3, or 10 mg/kg body weight, and more preferably, 0.1, 0.3, 1, or 3 mg/kg body weight. The dosing schedule is typically designed to achieve exposures that result in sustained receptor occupancy based on typical pharmacokinetic properties of an antibody. An exemplary treatment regime entails administration once per week, once every two weeks, once every three weeks, once every four weeks, once a month, once every 3 months or once every three to 6 months.

Actual dosage levels of the active ingredients in the pharmaceutical compositions of the present invention may be varied so as to obtain an amount of the active ingredient that is effective to achieve the desired therapeutic response for a particular patient, composition, and mode of administration, without being unduly toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts. One of ordinary skill in the art would be able to determine appropriate dosages based on such factors as the subject's size, the severity of the subject's symptoms, and the particular composition or route of administration selected. A composition of the present invention can be administered via one or more routes of administration using one or more of a variety of methods well known in the art.

In certain embodiments, the dose of the Fc fusion protein is a flat-fixed dose that is fixed irrespective of the size or weight of the patient. For example, the Fc fusion protein may be administered at a fixed dose of 5, 20, 35, 75, 200, 350, 750 or 1500 mg, without regard to the patient's weight. As used herein, the terms "fixed dose", "flat dose" and "flat-fixed dose" are used interchangeably and refer to a dose that is administered to a patient without regard to the weight or body surface area of the patient. The fixed or flat dose is therefore not provided as a mg/kg dose, but rather as an absolute amount of the Fc fusion protein (*e.g.,* an anti-CTLA-4 antibody). As will be appreciated by the skilled artisan, the dosage, route and/or mode of administration will vary depending upon the desired results.

### Therapeutic Uses and Methods of the Invention

The Fc fusion proteins, compositions and methods of the present disclosure have numerous therapeutic utilities, including the treatment of cancers and infectious diseases.

### Cancer immunotherapy

Disclosed herein is a method for potentiating an endogenous immune response in a subject afflicted with a cancer so as to thereby treat the subject, which method comprises administering to the subject a therapeutically effective amount of any of the Fc fusion proteins described herein, wherein the Fc region of the Fc fusion protein has been selected, designed or modified so as to enhance the binding of said Fc region to an activating Fc receptor. In the methods disclosed herein, the Fc region is not necessarily modified; for example, the appropriate Fc region may be selected or designed to bind to activating FcRs. Similarly, binding to the FcR is not necessarily increased but may be selected or designed to be high. In certain cases, the Fc fusion protein is an antibody. In preferred cases, the antibody is an IgG isotype. In certain other cases, the antibody is a monoclonal antibody. In further cases, the monoclonal antibody is a chimeric, humanized or human antibody. In yet other cases, the monoclonal antibody is a human IgG antibody. In certain preferred aspects of the present methods, binding of the human IgG antibody to an FcγI, FcγIIa or FcγIIIa receptor is enhanced. Preferably, enhanced binding of the human IgG antibody to the FcγI, FcγIIa or FcγIIIa receptor results in increased ADCC. In certain cases, the antibody is an antagonistic antibody that blocks the activity of a co-inhibitory immunoregulatory target on a T cell. In preferred cases, the co-inhibitory immunoregulatory target is CTLA or TIGIT.

Fc fusion proteins that bind to a wide variety of T cell localized targets, for example, both co-stimulatory and co-inhibitory immunomodulatory targets on T cells, preferably T_{regs} or other immune suppressive cell, may be employed in the present immunotherapeutic methods. In certain cases, the Fc fusion protein is an antagonist antibody, *e.g.,* an anti-CTLA-4 antibody or an anti-TIGIT antibody. In certain preferred cases, the antibody is an anti-CTLA-4 antibody. In further preferred cases, the anti-CTLA-4 antibody is ipilimumab or tremelimumab. In even more preferred cases, the anti-CTLA-4 antibody is an antibody wherein the Fc region of the Fc fusion protein has been selected, designed or modified so as to enhance the binding of the Fc region to an activating Fc receptor, for example, an enhanced variant of ipilimumab or tremelimumab. In certain cases, such enhanced variants are afucosylated or hypofucosylated variants of ipilimumab or tremelimumab. In other cases, such enhanced variants of ipilimumab or tremelimumab comprise amino acid mutations selected from S298A/E333A/L334A, S239D/I332E, S239D/I332E/A330L, L235V/F243L/R292P/Y300L/ P396L, and M428L/N434S mutations.

In other cases, the antibody the antibody is an agonistic antibody that augments the activity of a co-stimulatory immunoregulator target on a T cell. In preferred cases, the co-stimulatory immunoregulator target is GITR, OX40, ICOS or CD137. In further cases, the anti-GITR, OX40, ICOS or CD137 antibody is an afucosylated or hypofucosylated variant. In further cases, the anti-GITR, OX40, ICOS or CD137 antibody is an enhanced variant comprising one or more amino acid mutations selected from S298A/E333A/L334A, S239D/I332E, S239D/I332E/A330L, L235V/F243L/R292P/Y300L/ P396L, and M428L/N434S mutations.

In preferred cases of the present immunotherapeutic methods, the subject is a human.

Examples of other cancers that may be treated using the immunotherapeutic methods of the disclosure include bone cancer, pancreatic cancer, skin cancer, cancer of the head or neck, breast cancer, lung cancer, cutaneous or intraocular malignant melanoma, renal cancer, uterine cancer, ovarian cancer, colorectal cancer, colon cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, a hematological malignancy, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally induced cancers including those induced by asbestos, metastatic cancers, and any combinations of said cancers. In preferred embodiments, the cancer is selected from MEL, RCC, squamous NSCLC, non-squamous NSCLC, CRC, CRPC, squamous cell carcinoma of the head and neck, and carcinomas of the esophagus, ovary, gastrointestinal tract and breast. The present methods are also applicable to treatment of metastatic cancers.

Fc fusion proteins of the disclosure can be combined with an immunogenic agent, such as cancerous cells, purified tumor antigens (including recombinant proteins, peptides, and carbohydrate molecules), cells, and cells transfected with genes encoding immune stimulating cytokines (He *et al.,* 2004; Mellman *et al.,* 2011). Non-limiting examples of tumor vaccines that can be used include peptides of melanoma antigens, such as peptides of gp100, MAGE antigens, Trp-2, MART1 and/or tyrosinase, or tumor cells transfected to express the cytokine GM-CSF.

In certain cases of these methods for treating a cancer patient, the Fc fusion protein is administered to the subject as monotherapy, whereas in other cases, stimulation or blockade of immunomodulatory targets may be effectively combined with standard cancer treatments, including chemotherapeutic regimes, radiation, surgery, hormone deprivation and angiogenesis inhibitors. The Fc fusion protein can be linked to an antineoplastic agent (as an immunoconjugate) or can be administered separately from the agent. In the latter case (separate administration), the antibody can be administered before, after or concurrently with the agent or can be co-administered with other known therapeutic agents. Chemotherapeutic drugs include, among others, doxorubicin (ADRIAMYCIN®), cisplatin, carboplatin, bleomycin sulfate, carmustine, chlorambucil (LEUKERAN®), cyclophosphamide (CYTOXAN®; NEOSAR®), lenalidomide (REVLIMID®), bortezomib (VELCADE®), dexamethasone, mitoxantrone, etoposide, cytarabine, bendamustine (TREANDA®), rituximab (RITUXAN®), ifosfamide, vincristine (ONCOVIN®), fludarabine (FLUDARA®), thalidomide (THALOMID®), alemtuzumab (CAMPATH®), ofatumumab (ARZERRA®), everolimus (AFINITOR®, ZORTRESS®), and carfilzomib (KYPROLIS™). Co-administration of anti-cancer agents that operate via different mechanisms can help overcome the development of resistance to drugs or changes in the antigenicity of tumor cells.

In other embodiments, the patient can be additionally treated with an agent that modulates, *e.g.,* enhances or inhibits, the expression or activity of an FcγR by, for example, treating the subject with a cytokine. Preferred cytokines for administration during treatment with the Fc fusion protein include granulocyte colony-stimulating factor (G-CSF), granulocyte- macrophage colony-stimulating factor (GM-CSF), interferon-y (IFN-y) and tumor necrosis factor (TNF).

One aspect of this invention is the use of any Fc fusion protein of the disclosure for the preparation of a medicament for immunotherapy of a subject afflicted with cancer. Uses of any Fc fusion protein of the disclosure for the preparation of medicaments are broadly applicable to the full range of cancers disclosed herein. Also disclosed herein is an Fc fusion protein of the invention for use in any method of treatment employing an Fc fusion protein described herein.

### Treatment of Infectious Diseases

Other methods disclosed herein are used to treat patients that have been exposed to particular toxins or pathogens. Accordingly, another aspect of the disclosure provides a method of treating an infectious disease in a subject, for example by potentiating an endogenous immune response in a subject afflicted with an infectious disease, comprising administering to the subject a therapeutically effective amount of an Fc fusion protein of the disclosure, wherein the Fc region of the Fc fusion protein has been selected, designed or modified so as to enhance the binding of said Fc region to an activating Fc receptor.

In certain preferred cases, the Fc fusion protein is an antibody. Examples of infectious diseases for which this therapeutic approach may be particularly useful include diseases caused by pathogens for which there is currently no effective vaccine, or pathogens for which conventional vaccines are less than completely effective. These include, but are not limited to HIV, Hepatitis (A, B, and C), Influenza, Herpes, Giardia, Malaria, Leishmania, Staphylococcus aureus, and Pseudomonas aeruginosa. In certain preferred cases, the pathogen is a viral pathogen. The immunotherapeutic approaches described herein are particularly useful against established infections by agents such as HIV that present altered antigens over the course of the infections.

Similar to its application to tumors discussed above, the immunotherapeutic approaches described herein can be used alone, or as an adjuvant, in combination with vaccines, to stimulate the immune response to pathogens, toxins, and self-antigens. These approaches can be combined with other forms of immunotherapy such as cytokine treatment (*e.g.,* administration of interferons, GM-CSF, G-CSF or IL-2).

### Potential Biomarkers for Anti-CTLA-4 Immunotherapy

The data disclosed herein also suggest potential biomarkers for predicting the suitability of candidate patients for immunotherapy with an anti-CTLA-4 antibody and/or predicting anti-tumor efficacy of such an antibody. For example, the observation that the IgG1 isotype for anti-CTLA-4 mediates T_{reg} depletion suggests that FCRG3A (CD16) polymorphisms may be related to the activity of ipilimumab as has been observed for the activity of rituximab (Weng and Levy, 2003; Cartron *et al.,* 2004) and trastuzumab (HERCEPTIN®) Musolino *et al.,* 2008; Tamura *et al.,* 2011). If binding to FcγRIIIa is required for the activity of ipilimumab, then individuals with homozygous FcγRIIIa V158 variants, which bind IgG1 with higher affinity than the F158 variant, would be expected to show better survival and/or response (Cartron *et al.,* 2004). In addition, if binding to FcγRIIa is required for activity of ipilimumab, then individuals with FcγRIIa H131 variants, which bind IgG1 with higher affinity than the R131 variant, would be expected to show better survival and/or response (Weng and Levy, 2003).

The present data also suggest that anti-CTLA-4 functions best in tumors containing an increased number of T_{regs} at the tumor site. The presence of T_{regs} is likely to be the consequence of an ongoing immune response to tumor antigens. Indeed, response to ipilimumab has been associated with the presence of tumor-infiltrating lymphocytes (Hamid *et al.,* 2011; Ji *et al.,* 2012). Ipilimumab therapy is also more effective in patients with preexisting responses to tumor antigen, such as for NY-ESO-1 (Yuan *et al.,* 2011). Moreover, T_{reg} elimination may depend on the presence of specific cell types in the tumor microenvironment. The present data indicate that macrophage-like cells or other cells of myeloid lineage present in the tumor, such as monocytes, macrophages or NK cells which bear the relevant Fcγ receptors, are required for the antitumor effect of anti-CTLA-4.

Accordingly, disclosed herein is a method for predicting the suitability of candidate patients for immunotherapy with an Fc fusion protein such as an anti-CTLA-4 antibody, and/or predicting anti-tumor efficacy of such an antibody, comprising screening for the presence of macrophage-like cells or other cells of myeloid lineage in the tumor.

Also disclosed herein is a method for immunotherapy of a subject afflicted with cancer, which method comprises: (a) selecting a subject that is a suitable candidate for immunotherapy, the selecting comprising (i) assessing the presence of macrophage-like cells or other cells of myeloid lineage in a test tissue sample, and (ii) selecting the subject as a suitable candidate based on the presence of macrophage-like cells or other cells of myeloid lineage in a test tissue sample; and (b) administering a therapeutically effective amount of an immunomodulatory Fc fusion protein to the selected subject. In certain preferred cases, the immunomodulatory Fc fusion protein is an anti-CTLA-4, an anti-TIGIT, an anti-GITR, an anti-OX40, an anti CD137 or an anti-ICOS antibody.

### Kits

Also within the scope of the present disclosure are kits comprising any Fc fusion protein or composition thereof of this disclosure and instructions for use. Accordingly, disclosed herein is a kit for treating a cancer or a disease caused by an infectious agent in a subject, the kit comprising (a) one or more doses of any Fc fusion protein of the disclosure that exhibits an enhanced ability to potentiate an endogenous immune response against cells of the cancer or the infectious agent in the subject, and (b) instructions for using the Fc fusion protein in any of the therapeutic methods described herein. For example, in certain cases the Fc fusion protein in the kit is an antagonistic Fc fusion protein that blocks the activity of a co-inhibitory immunoregulatory target on a T cell. In further cases, the co-inhibitory immunoregulatory target is CTLA or TIGIT. In other cases, the antagonistic Fc fusion protein is an antibody. In further cases, the antibody is an anti-CTLA-4 or anti-TIGIT antibody. In further cases, the anti-CTLA-4 antibody is an enhanced variant of ipilimumab or tremelimumab.

In certain other cases, the Fc fusion protein is an agonistic protein that augments the activity of a co-stimulatory immunoregulator target on a T cell. In further cases, the co-stimulatory immunoregulatory target is GITR, OX40, ICOS or CD137. In other cases, the agonistic Fc fusion protein is an antibody. In further cases, the antibody is an anti-GITR, OX40, ICOS or CD137 antibody.

The kit may further contain one or more additional therapeutic reagents. For example, for the treatment of a cancer, the one or more additional agents may be an immunosuppressive reagent, a chemotherapeutic agent or a radiotoxic agent, or one or more additional Fc fusion proteins that target different antigens.

Kits typically include a label indicating the intended use of the contents of the kit and instructions for use. The term label includes any writing, or recorded material supplied on or with the kit, or which otherwise accompanies the kit. In certain cases of a pharmaceutical kit, the Fc fusion protein may be co-packaged with other therapeutic agents in unit dosage form.

The present invention is further illustrated by the following examples, which should not be construed as limiting.

### EXAMPLE 1

### Generation of Different Anti-mCTLA-4 Antibody Isotypes

To determine the relative potency of different isotypes of anti-CTLA-4 in antitumor activity, four isotypic variants of the mouse anti-mouse CTLA-4 antibody, 9D9, were generated and purified from CHO transfectants or the parental hybridoma. These anti-CTLA-4 variants included the IgG1 isotype containing a D265A mutation (IgG1-D265A), which is a non-FcγR-binding mutant (Clynes *et al.,* 2000), IgG1, IgG2b (original isotype of 9D9, derived from a hybridoma), and IgG2a. The 9D9 hybridoma (kindly supplied by J. Allison, University of Texas, MD Anderson, Houston, Texas) is a mouse anti-mouse CTLA-4 antibody derived by immunization of human CTLA-4 transgenic mice with mouse CTLA-4 (Peggs *et al.,* 2009). 9D9 blocks the binding of murine CTLA-4-Ig to B7-1-positive cells (data not shown).

To generate 9D9 isotypes, total RNA was prepared from 9D9 hybridoma cells using the RNeasy Mini Kit (Qiagen, Valencia, CA, USA). cDNA was prepared by the 5'-RACE protocol using the SMARTer RACE cDNA Amplification and Advantage 2 PCR kits (Clontech Laboratories, Inc., Mountain View, CA, USA). Variable regions were amplified using a 3' murine-specific constant region primer, paired with the 5' RACE universal primer mix. PCR products containing the V-region were cloned into the pCR4-TOPO vector (Invitrogen, Carlsbad, CA, USA) and transformed into *E. coli* strain TOP10 (Invitrogen). Templiphi (GE Healthcare Biosciences, Piscataway, NJ, USA) samples were prepared and subjected to DNA sequencing (Sequetech, Mountain View, CA, USA).

For expression of recombinant antibodies (mouse IgG2a, mouse IgG1, and mouse IgG1-D265A isotypes), the 9D9 variable regions were amplified by PCR to introduce cloning sites and cloned into UCOE expression vectors (EMD Millipore, Billerica, MA, USA) that contain the osteonectin signal sequence and the desired constant region. Heavy and light chain vectors were linearized and cotransfected into CHO-S cells (Invitrogen). Stable pools and/or clones were selected. For mouse IgG2a, the BALB/c allotype, IgG2a^{a} (haplotype Igh-1^{a}) sequence was used.

Culture supernatants from 9D9 hybridoma or CHO cell transfectants were harvested for antibody production. Antibodies were purified using Protein A or Protein G by standard methods and dialyzed into PBS. All antibodies were free of endotoxin (< 0.05 EU/mg) and shown to have < 5% aggregates as determined by size exclusion chromatography/HPLC. Each of the CTLA-4 isotypes was assessed for binding to cells constitutively expressing CTLA-4 (58α-β-CTLA-4/CD3ζ) by flow cytometry. 58α-β-CTLA-4/CD3ζ is a murine T-cell hybridoma that expresses murine CTLA-4 fused to CD3ζ and is analogous to a similar construct for human CTLA-4 (Keler *et al.,* 2003). Cells (1 x 10⁵ per well) in FACS buffer (1x DPBS [CellGro], 0.02% sodium azide, 2% FBS [Hyclone], and 1 mM EDTA) were stained with serial dilutions of antibodies starting at 20 µg/ml and incubated for 30 min at 4°C. The cells were washed and secondary antibody (R-PE Donkey anti-mouse IgG [Jackson ImmunoLabs]) was added and incubated for 30 min at 4°C. Cells were then washed and resuspended in FACS buffer and analyzed on a BD FACS Canto flow cytometer.

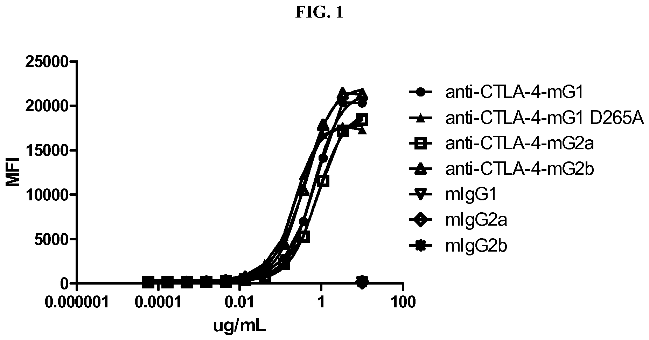

As shown in Figure 1, each of the anti-CTLA-4 isotype variants binds equivalently to cells constitutively expressing mouse CTLA-4. Antibodies labeled mIgG1, mIgG2a and mIgG2b are commercially available mouse isotype control antibodies.

The general procedures described above were also used to construct different isotypic variants of agonistic antibodies that bind to mouse GITR, OX40 and ICOS receptors, and an antagonistic antibody that binds to mouse PD-1.

### Pharmacokinetic analysis

For characterization of pharmacokinetics of the anti-CTLA-4 antibodies, nine female C57BL/6 mice were injected intraperitoneally with 10 mg/kg of each isotype of anti-CTLA-4 (IgG1, IgG1-D265A, IgG2a, or IgG2b). Blood samples were taken at 1, 6, 24, 48, 72, 120, 168, 336, and 504 h and the sera were analyzed by ELISA. Chemiluminescent ELISA was used to measure serum levels of anti-CTLA-4 monoclonal antibodies. Recombinant mouse CTLA-4-Ig was used as a capture in combination with an HRP conjugate of goat anti-mouse IgG (light chain specific) polyclonal antibody. Standards, controls, and samples were diluted 100-fold with 1% BSA/PBS/0.05% Tween 20. Concentrations of anti-CTLA-4 antibodies in mouse serum samples were calculated from luminescence intensity as measured by M5 plate reader (Molecular Devices, Sunnyvale, CA) using a 5-parameter logistic (5-PL) calibration curve generated from corresponding anti-CTLA-4 antibody calibrators.

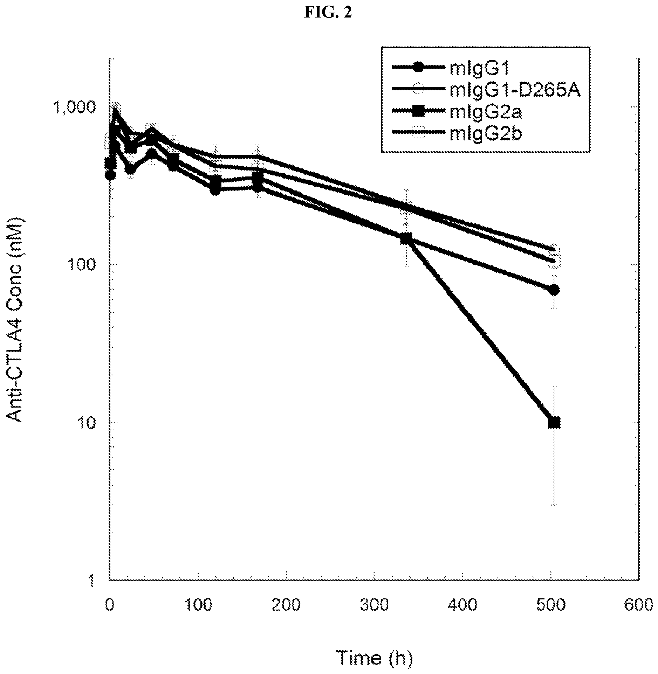

As shown in Table 2 and Figure 2, systemic exposure of the four isotypes were largely similar, although the area under the concentration vs. time curve (AUC) of IgG1-D265A (185 µM·h) and IgG2b (170 µM·h) were slightly higher than IgG1 (119 µM·h) and IgG2a (125 µM·h). The terminal half lives of the antibodies were also similar (156-174 h), although there was an accelerated terminal decay observed only for IgG2a from week 2 to week 3; this was presumably due to the formation of anti-drug antibody as a consequence of allotypic differences between the Balb/c IgG2a^{a} constant region and that of C57BL/6 mice tested here (Schreier *et al.,* 1981). Thus, the differences in anti-tumor efficacy of the anti-CTLA-4 isotypes cannot be explained by differences in drug exposure.

**Table 2. Summary of pharmacokinetic parameters of anti-CTLA4 isotypes following a single intraperitoneal injection at 10 mg/kg in non-tumor bearing C57BL/6 mice**

| PK Parameters | IgG1 | IgG1-D265A | IgG2a | IgG2b |
|---|---|---|---|---|
| Cmax (µM) | 0.56 | 0.92 | 0.71 | 0.96 |
| Tmax (h) | 6 | 6 | 6 | 6 |
| AUC0-504h (µM·h) | 119 | 185 | 125 | 170 |
| T_{1/2} (h) | 156 | 171 | 164* | 174 |

| | | | | |
|---|---|---|---|---|
| For IgG2a, an accelerated terminal decay was observed from 336 h to 504 h, presumably due to the formation of anti-drug antibody (see below). Note that the IgG2a allotype is derived from BALB/c mice. The 504-h time point was excluded from the estimation of T_{1/2}. | | | | |

### Binding affinities

Each of the anti-CTLA-4 isotypes was characterized for binding to soluble forms of FcγRI, FcγRIIB, FcγRIII and FcγRIV, and FcRn, by surface plasmon resonance. Affinities of the different anti-CTLA-4 isotypes for FcγRs were determined to be as previously described (Nimmerjahn and Ravetch, 2005). FcγRs were procured from R&D Systems with the exception of FcγRI. For expression of FcγRI, the extracellular domain was amplified by PCR and cloned into a UCOE expression vector (EMD Millipore, USA) in-frame with an osteonectin signal sequence and a C-terminal 6xHis-tag and stop codon. CHO-S cells (Invitrogen) were transfected using Amaxa Nucleofector II (Lonza Group, AG), and stable pools and clones were selected and expanded and the subsequent supernatants collected for purification. The soluble recombinant proteins were purified using standard techniques through immobilized metal nickel affinity chromatography(IMAC Life Technologies Corporation) nickel-charged resin columns.

FcγR interactions were determined by coating the antibodies directly on a CM5 chip to a density of about 1500 RUs and flowing 8 concentrations of mFcRs over the immobilized antibodies until equilibrium was attained. The equilibrium response unit (RU) was plotted as a function of FcR concentration using GraphPad Prism and equilibrium K_{D} was obtained. Alternatively, the 6xHis-tagged FcRs were captured (to about 200 RUs) on an anti-His antibody-coated CM5 surface and flowing 8 concentrations of the antibody over the FcγR captured surface. The equilibrium K_{D} obtained by this approach was lower by about 4-fold due to the absence of multivalent binding present when antibodies are directly coated on the surface. FcRn interaction was characterized by coating 500 RUs of mo FcRn on a CM5 chip and flowing 8 concentrations of antibodies over the FcRn coated surface at pH 6.0, in 50mM 2-(N-morpholino)ethanesulfonic acid, 150mM NaCl running buffer. The antibody-bound surface was regenerated using pH 8.0 Tris buffer. The binding affinities are shown in Table 3.

**Table 3. Binding affinities of anti-CTLA-4 isotypes to murine FcγR proteins as assessed by surface plasmon resonance.**

| | Affinity K_{D} (nM) | | | | |
|---|---|---|---|---|---|
| Antibody | FcγRIII | FcγRIV | FcγRIIB | FcγRI | FcRn |
| 9D9-IgG1 | 1878 | NB | 479 | NB | 22.4 |
| 9D9-IgG1-D256A | NB | NB | NB | NB | 24.6 |
| 9D9-IgG2a | 1497 | 29.45 | 1414 | 32.67 | 13.46 |
| 9D9-IgG2b | 2445 | 56.26 | 748.4 | NB | 19.67 |

| | | | | | |
|---|---|---|---|---|---|
| NB = no binding. | | | | | |

### EXAMPLE 2

### Anti-Tumor Activity of Variant Anti-CTLA-4 Isotypes in Murine CT26 Colon Adenocarcinoma Tumor Model

Many different antibodies have been used to demonstrate activity of anti-CTLA-4 antibodies, including hamster anti-CTLA-4 antibodies, 9H10 (Krummel and Allison, 1995) and 4F10 (Walunas *et al.,* 1994), and the mouse anti-mouse CTLA-4 antibody, 9D9 (Peggs *et al.,* 2009). In order to determine the relative potency of different isotypes of anti-CTLA-4 in anti-tumor activity, three of the four isotypic variants of anti-CTLA-4 antibody 9D9 generated (anti-CTLA-4-γ1D265A, anti-CTLA-4-y2b, and anti-CTLA-4-γ2a, which bind equally well to CTLA-4⁺ cells as described in Example 1) were tested together with a mouse IgG1 isotype control for anti-tumor activity in a syngeneic CT26 colon adenocarcinoma model. The control antibody used for the studies is a recombinant human anti-diphtheria toxin antibody with a mouse IgG1 isotype.

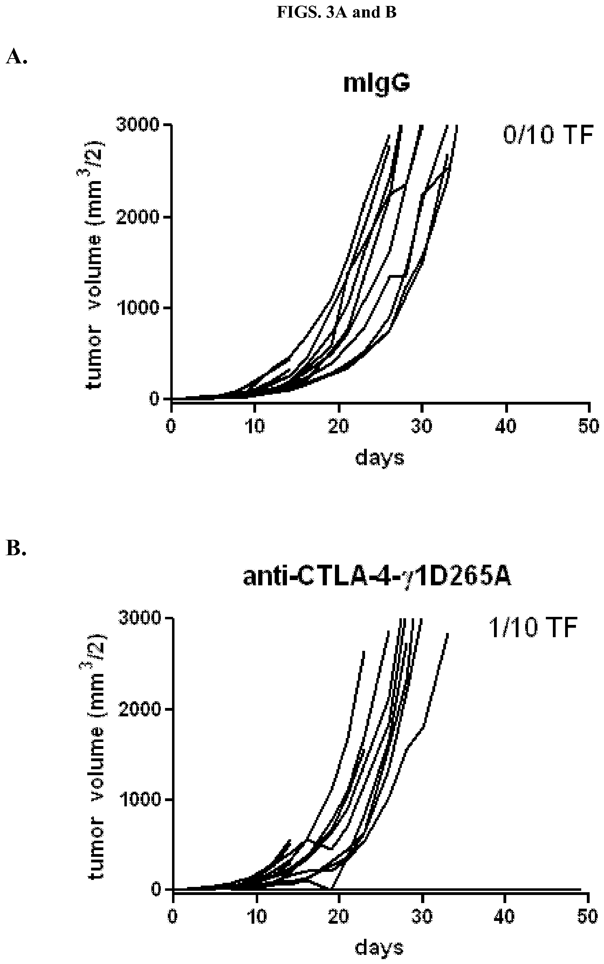

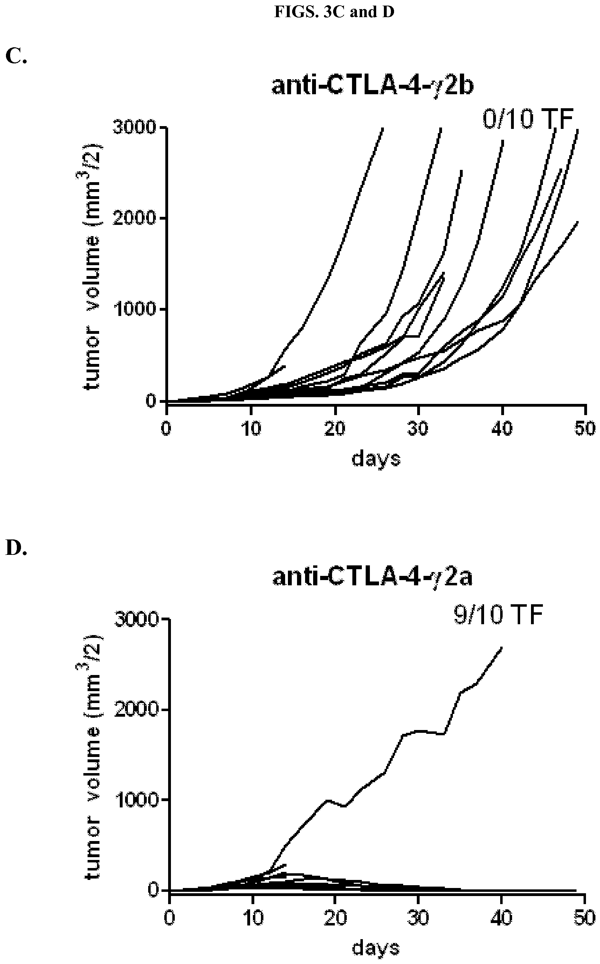

Ten BALB/c mice were subcutaneously injected with 1 x 10⁶ CT26 tumor cells on day 0. Treatment was begun at Day 7 after implantation. Tumors were measured, randomized into treatment groups so as to have comparable mean tumor volumes (45-50 mm³/2), and then treated intraperitoneally (IP) with the designated antibody (200 µg/dose) and again on Days 10, 14 and 17. Tumor volumes were measured twice weekly. As shown in Figure 3, anti-CTLA-4 9D9-IgG2a resulted in tumor rejection in 9 of 10 mice treated, while 9D9-IgG2b showed moderate tumor growth inhibition, with none of ten treated mice being tumor-free after up to 50 days. Surprisingly, the anti-CTLA-4 IgG1D265A isotype showed little activity and was comparable to control IgG (Figure 3).

### EXAMPLE 3

### Effects of Anti-CTLA-4 Isotypes on CT26 Intratumoral T Cell Subsets and Peripheral T Cell Populations

### Lymphocyte staining analysis

In order to measure the effect of different anti-CTLA-4 isotypes on T cell populations, T cells isolated from mice treated with the different antibodies were stained for the presence of CD8, CD4, CD45 and Foxp3 markers. All mice were sacrificed and tumor and draining lymph node were harvested for analysis on Day 15 after tumor implantation. Single cell suspensions were prepared by dissociating tumor and lymph node with the back of a syringe in a 24-well plate. Cell suspensions were passed through 70 µm filters, pelleted, resuspended, and counted. Cells were then plated in 96-well plates with 1 x 10⁶ cells per well for staining. Cells were treated with 24G.2 (BioXcell), which blocks Fc binding to FcγRIIB and FcγRIII, and subsequently stained with antibodies against CD8 (clone 53-6.7; Biolegend), CD4 (clone GK1.5; Biolegend), and CD45 (clone 30-F11; Biolegend) or antibodies to CD11c (clone N418; eBioscience), CD45, CD8, CD11b (clone M1/70 Biolegend), and Gr-1 (clone Rb6-8C5; eBioscience). For intracellular staining, samples were fixed, permeabilized, and stained with antibodies to Foxp3 (clone FJK-16s; eBioscience), Ki67 (clone SolA15; eBioscience), and CTLA-4 (clone 4F10; BD Pharmingen). Samples were then analyzed on a FACS Canto flow cytometer (BD). This general flow cytometric procedure was also used to determine the effects of different Fc fusion proteins on populations of different T cells as described in the following Examples.

CD45 is a type I transmembrane protein that is expressed at high levels in various forms on all differentiated hematopoietic cells except erythrocytes and plasma cells. CD8, a co-receptor for the T cell receptor (TCR), is predominantly expressed on the surface of cytotoxic T cells, though it may also be found on NK cells, cortical thymocytes, and dendritic cells, whereas CD4 is a glycoprotein found predominantly on T helper cells, but also on the surface of other immune cells such as monocytes, macrophages, and dendritic cells. Foxp3, a transcriptional repression factor of the forkhead or winged helix family of transcription factors, serves as a specific marker of T_{regs} which had previously been identified by non-specific markers such as CD25 or CD45RB (an isoform of CD45, with exon 5 splicing, that encodes B determinant). Foxp3 has been found to be expressed in all CD4⁺ T_{regs} that have regulatory activity, but staining for this marker requires fixation and permeabilization of the cells.

### Effects of anti-CTLA-4 isotype on T cell populations

As CTLA-4 is expressed by, and has a functional role in, both activated T_{eff}/T memory cells and T_{reg} subsets, multiple cell populations from different locations were monitored. Previous data demonstrated that anti-CTLA-4 antibody blockade results in expansion of T_{regs} in the lymph nodes (LN) of treated mice (Quezada *et al.,* 2006). The effect of CTLA-4 antibody isotype on peripheral T_{reg} expansion was evaluated in CT26 colon adenocarcinoma tumor-bearing mice by analyzing T cell subsets in the tumor and tumor draining lymph nodes of the mice at Day 16 after antibody treatment. Statistical analyses were performed using GraphPad Prism. Error bars represent the standard error of the mean calculated using Prism. Specific statistical tests used were unpaired t tests and 1-way analysis of variance. *P* values of < 0.05, 0.01. and 0.001 were noted as *, **, and ***, respectively, in each figure.

All antibodies enhanced the numbers of T_{regs} in the spleen or at other sites in the periphery, such as LNs or blood for representative FACS plots). Additionally, T_{regs} in animals treated with anti-CTLA-4 also have higher expression of Ki-67, a marker for proliferation, suggesting that CTLA-4 blockade is removing an inhibitory signal, regardless of the antibody isotype. Similar results were obtained in an analysis of LNs from non-tumor-bearing mice treated with each of the anti-CTLA-4 isotypes (data not shown).

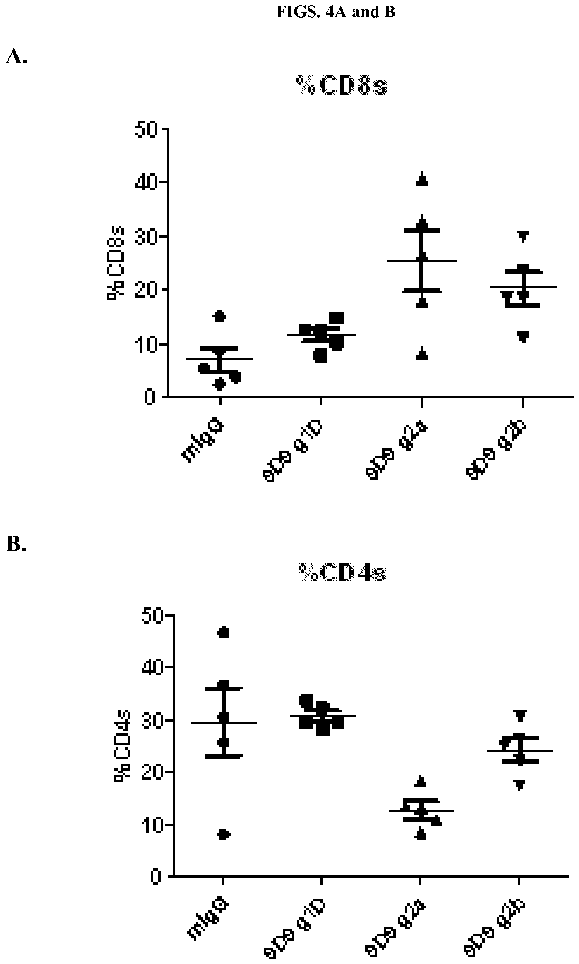

As expected, treatment of mice with anti-CTLA-4 antibodies resulted in an increase in the percentage of CD45⁺ cells at the tumor site that were CD8⁺, with the greatest increases induced by the 2a and 2b isotypes and a slight increase induced by the G1D265A group (Figure 4A). Total CD8⁺ T cell numbers at the tumor were consistent with the percentage changes (data not shown).

Analysis of the percentage of intratumoral CD4⁺ cells revealed that anti-CTLA-4 with a 2a isotype resulted in a reduction of CD4⁺ T cells (Figure 4B).

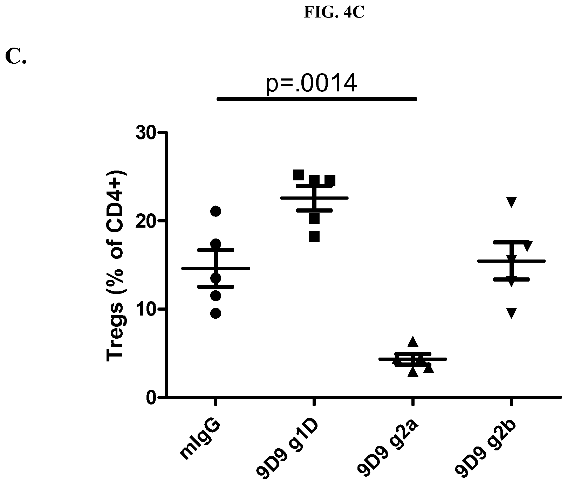

When intratumoral T cells were analyzed for T_{regs} by staining for Foxp3 and CD4, profound differences in each of the treatment groups were observed (Figure 4C). Treatment with anti-CTLA-4 of the 2a isotype resulted in dramatic decreases in the number of T_{regs} at the tumor, while 2b showed no change, and IgG1D265 resulted in increases in T_{reg} numbers.

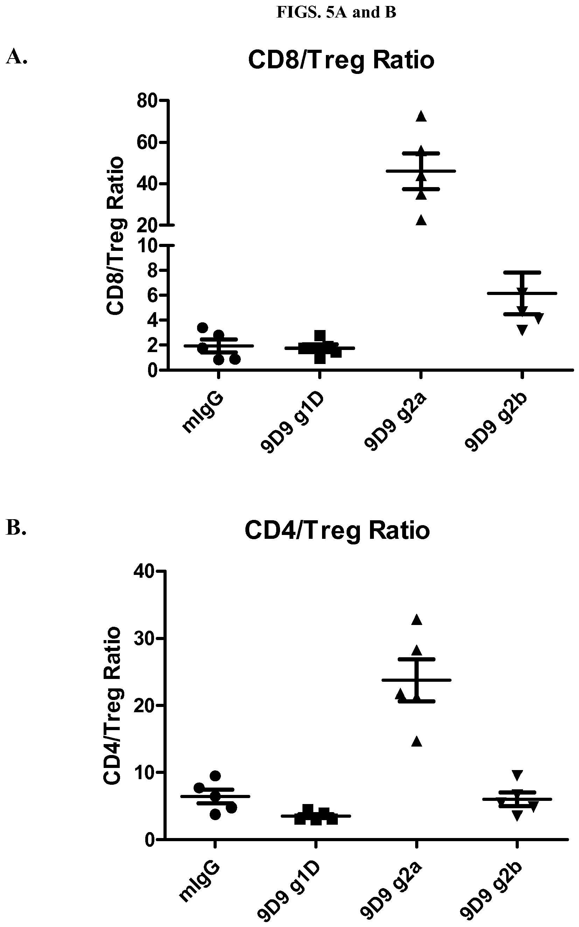

The changes in T effector cell (T_{eff}) and T_{reg} numbers mediated by each of these anti-CTLA-4 isotypes result in dramatic differences in the intratumoral CD8⁺ T cell to T_{reg} ratio as well as CD4⁺ T_{eff} to T_{reg} ratio (Figures 5A and B). Anti-CTLA-IgG2a isotype showed the highest T_{eff} to T_{reg} ratio. A high ratio of CD8⁺ T cells to T_{regs} is considered to be reflective of potent anti-tumor activity.

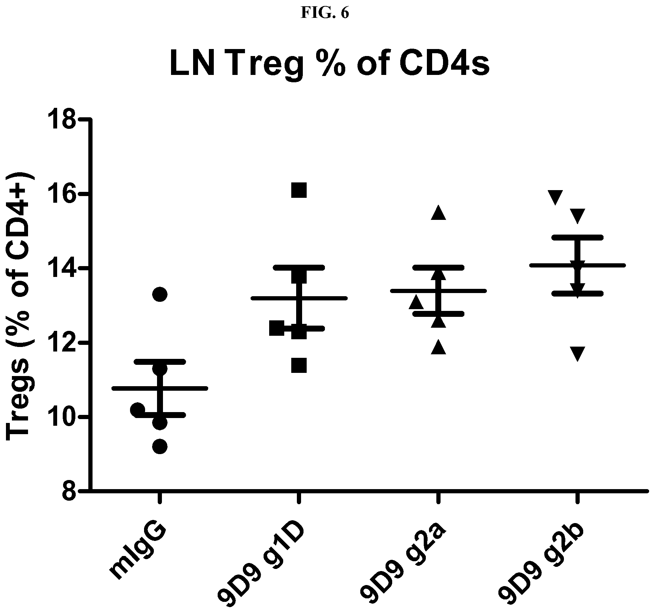

In contrast to the analysis of intratumoral T cells, evaluation of T cell subsets in the periphery showed little differences between the isotypes. All antibodies enhanced the numbers of T_{regs} in the tumor draining lymph nodes (Figure 6). These data are consistent with earlier observations that anti-CTLA-4 results in expansion of T_{regs} in the LNs of anti-CTLA-4-treated mice (Quezada *et al.*, 2006), and generally consistent with earlier conclusions that the anti-tumor effects of CTLA-4 blockade are not due to depletion of peripheral T_{regs} (Maker *et al.*, 2005; Rosenberg, 2006). An increase in the percentage of CD4⁺ cells that express ICOS was also observed in animals treated with all CTLA-4 isotypes (data not shown), suggesting that increased activation of T_{effs} is not dependent on antibody isotype. Collectively, these data demonstrate the T_{reg} loss is restricted to the tumor site.

### EXAMPLE 4

### Anti-Tumor Activity of Variant Anti-CTLA-4 Isotypes in MC38 Murine Colon Adenocarcinoma Tumor Model

In addition to the CT26 tumor model described in Example 3, the anti-tumor activity of different anti-CTLA-4 isotypes was assessed in a MC38 colon adenocarcinoma tumor model. C57BL/6 mice were each subcutaneously injected with 2 x 10⁶ MC38 tumor cells. After 7 days, tumor volumes were determined and mice were randomized into treatment groups so as to have comparable mean tumor volumes (44.7-49.2 mm³/2). Anti-CTLA-4 antibodies of four different isotypes (IgG1, IgG1D265A, IgG2a and IgG2b), formulated in PBS, were administered IP on Days 7, 10 and 14 at 200 µg per dose in a volume of 200 µl. Tumor volumes were recorded three times weekly.

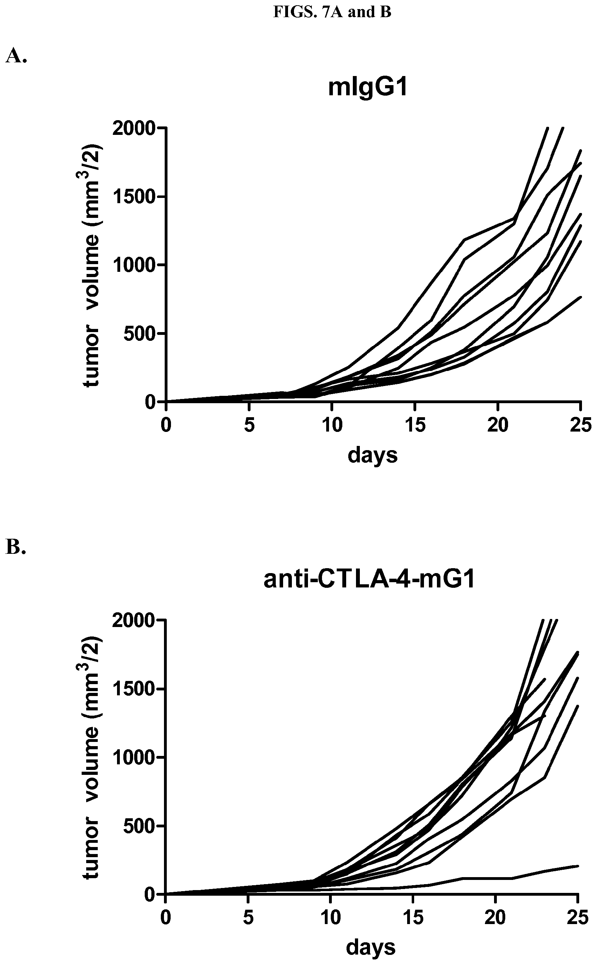

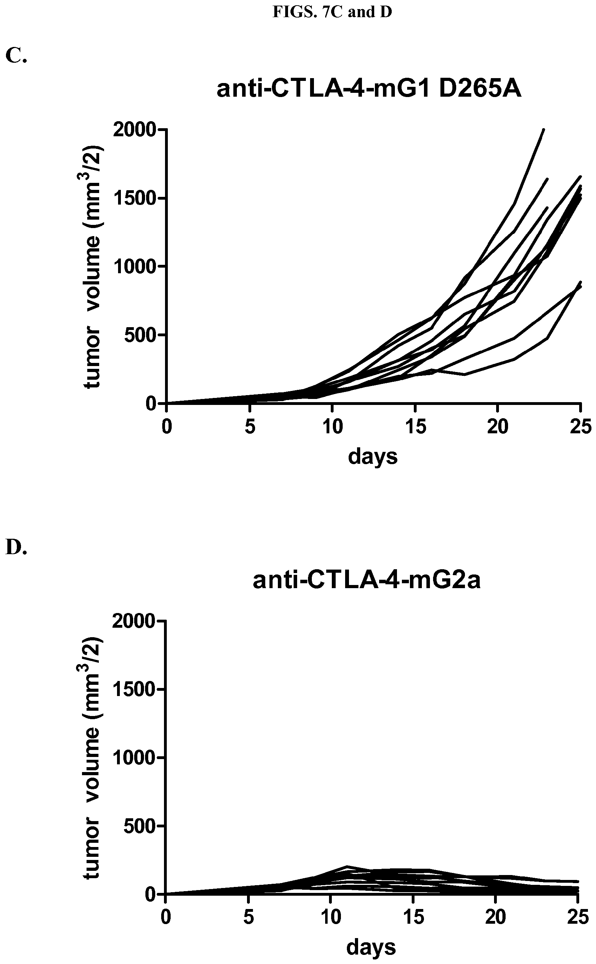

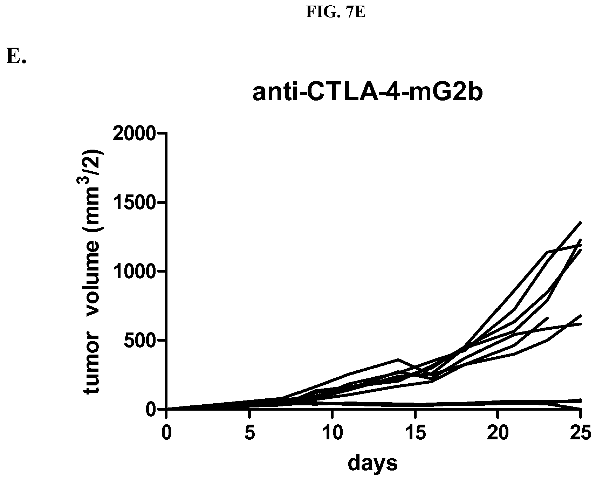

Figures 7B and C show that the IgG1 and IgG1D265A anti-CTLA-4-treated tumors grew rapidly at similar rates comparable to the rate of growth of tumors treated with a mouse IgG1 control (Figure 7A). In contrast, treatment of mice with the IgG2a anti-CTLA-4 antibody (Figure 7D) dramatically reduced the rate of tumor growth to levels approaching complete inhibition. The IgG2b anti-CTLA-4 antibody also significantly inhibited tumor growth (Figure 7E), though to a lesser extent than the IgG2a isotype.

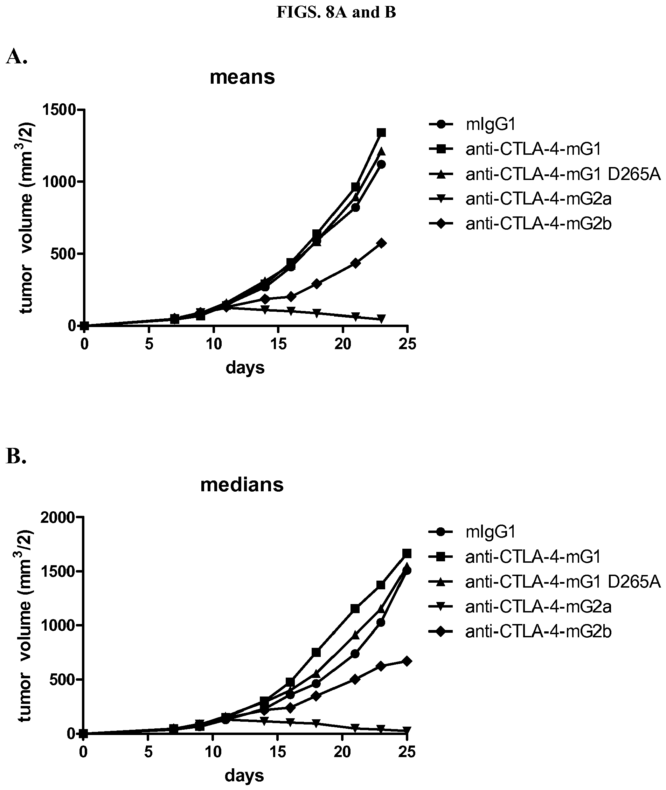

The changes in mean tumor volumes and median tumor volumes of the mice of groups treated with the different anti-CTLA-4 isotypes are plotted in Figures 8A and B. These plots confirm the individual mouse data shown in Figure 7 and clearly reveal that the IgG2a isotype of the anti-CTLA-4 antibody exhibits the most potent inhibitory effect on MC38 tumor growth, followed by the effect of the IgG2b isotype. The IgG1 and IgG1D265A isotypes show little or no inhibition of tumor growth, similar to the mouse IgG1 control. The percentage mean tumor growth inhibition effected by the four anti-CTLA-4 isotypes at different time points post tumor-implantation is shown in Table 4.

Collectively, the data in Figures 7 and 8 and in Table 4 demonstrate that the IgG1 and the mutated mouse IgG1 anti-CTLA-4 isotypes exhibit no anti-tumor activity compared to a mouse IgG isotype control in this staged (therapeutic) MC38 tumor model. In contrast, significant anti-tumor activity is apparent with the IgG2a and IgG2b isotypes, with the former, achieving virtually complete inhibition of tumor growth, being much more potent than the latter.

**Table 4. Percentage mean inhibition of MC38 tumor growth post tumor implantation**

| Day | % Mean Tumor Growth Inhibition | | | |
|---|---|---|---|---|
| | Anti-CTLA-4-mIgG1 | Anti-CTLA-4-mIgG1D265A | Anti-CTLA-4-mIgG2a | Anti-CTLA-4-mIgG2b |
| 7 | 8.2 | 2.0 | -0.1 | -1.0 |
| 9 | 7.3 | -13.6 | -17.7 | -18.5 |
| 11 | 3.8 | -8.5 | 14.9 | 11.5 |
| 14 | -8.1 | -15.6 | 59.8 | 31.3 |
| 16 | -7.5 | -3.6 | 75.5 | 50.9 |
| 18 | -7.8 | 0.8 | 85.4 | 50.6 |
| 21 | -17.3 | -8.9 | 92.6 | 47.0 |

### EXAMPLE 5

### Effects of Anti-CTLA-4 Isotypes on MC38 Intratumoral T Cell Subsets

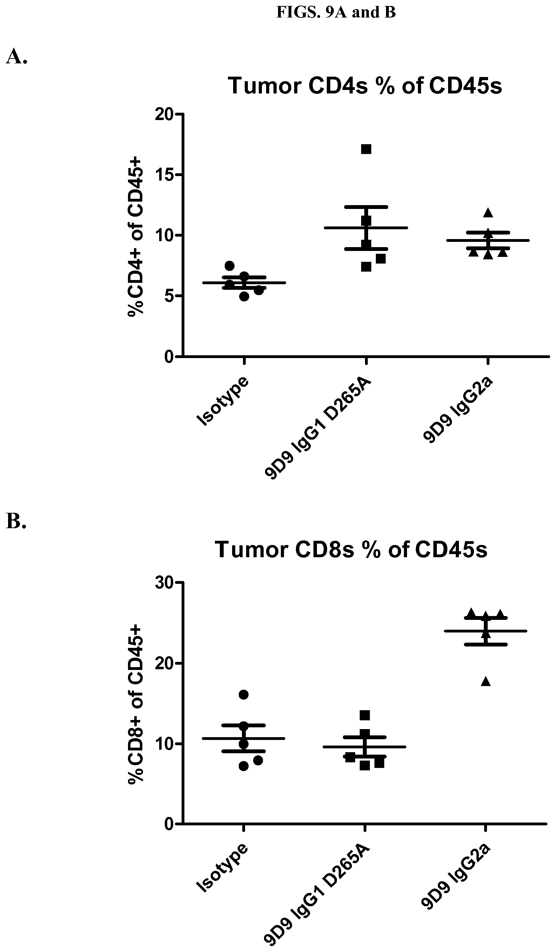

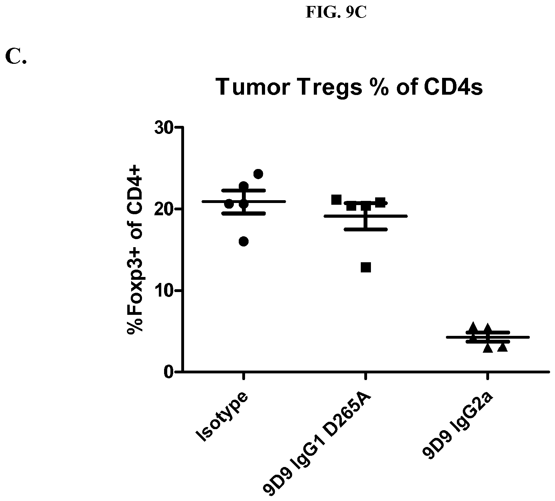

T cell subsets were analyzed in MC38 tumor-infiltrating lymphocytes (TILs) from mice treated with the different anti-CTLA-4 isotypes. The IgG2a and mutated IgG1D265A isotypes caused a marginal increase in the percentage of CD4⁺ cells compared to the mouse IgG1 isotype control (Figure 9A). However, when the level of CD8⁺ cells was analyzed, treatment with the IgG2a anti-CTLA-4 antibody resulted in a significant (about 2.5-fold) increase in the percentage of CD8⁺ cells compared to both the mouse IgG1 isotype and the mutated IgG1 anti-CTLA-4 (Figure 9B). The IgG2a anti-CTLA-4 antibody also induced an approximately 5-fold reduction in the level of T_{regs} compared to the IgG1 isotype and the mutated IgG1 anti-CTLA-4 (Figure 9C).

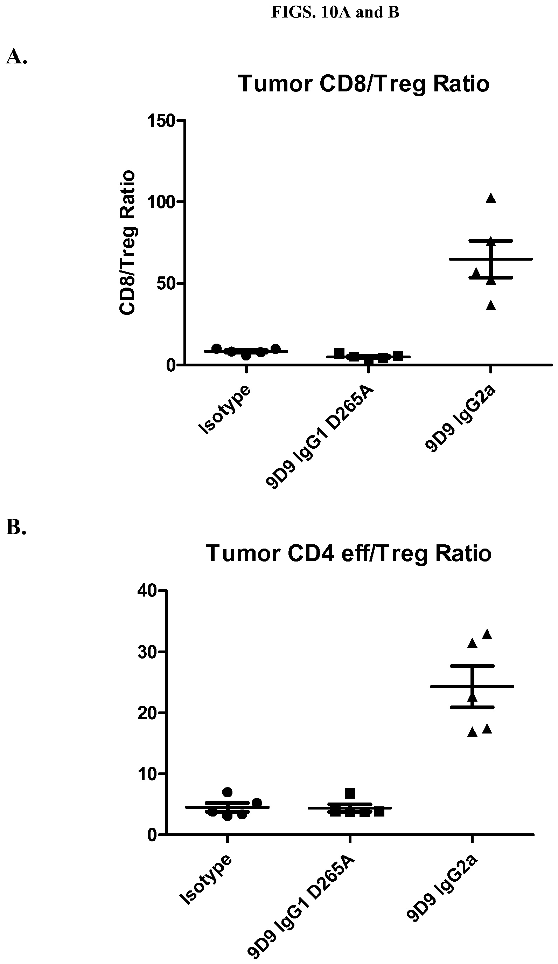

The combined effects of the increase in CD8⁺ T_{effs} and decrease in T_{regs} mediated by the IgG2a anti-CTLA-4 isotype translated into a T_{eff} to T_{reg} ratio (Figure 10A) that was much (more than 8-fold) higher than the T_{eff} to T_{reg} ratios resulting from treatment with the IgG1 isotype or IgG1D265A anti-CTLA-4 antibody (Figure 10A). As in the CT26 colon adenocarcinoma model, this high T_{eff} to T_{reg} ratio reflects robust anti-tumor activity. The CD4⁺ T_{eff} to T_{reg} ratio resulting from treatment with the IgG2a antibody was also about 5-fold higher than the T_{eff} to T_{reg} ratios induced by the isotype or IgG1D265A antibody (Figure 10B).

### EXAMPLE 6

### Anti-Tumor Activity of Variant Anti-CTLA-4 Isotypes in an Immunogenic SalN Murine Fibrosarcoma Tumor Model

The anti-tumor activity of anti-CTLA-4 was also assessed in an immunogenic SalN fibrosarcoma tumor model. A/J mice were subcutaneously injected with 2 x 10⁶ SalN tumor cells. After 7 days, tumor volumes were determined and mice were randomized into treatment groups so as to have comparable mean tumor volumes (132.4-146.5 mm³/2). Anti-CTLA-4 (9D9) antibodies having the IgG1, mutated IgG1D265A, and IgG2a isotypes were formulated in PBS and administered IP on Days 7, 11 and 14 at 200 µg per dose in a volume of 200 µl. Tumor volumes were recorded twice weekly.

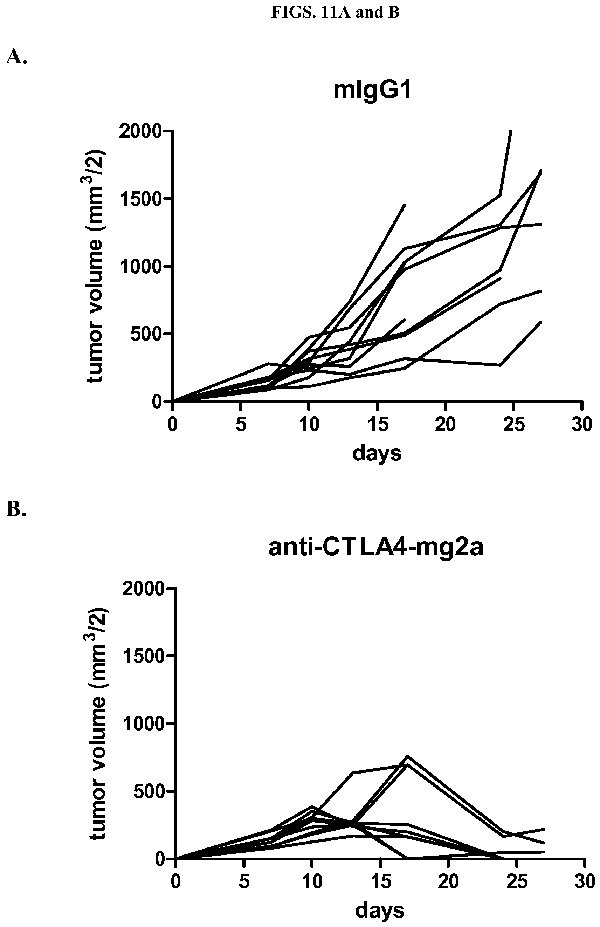

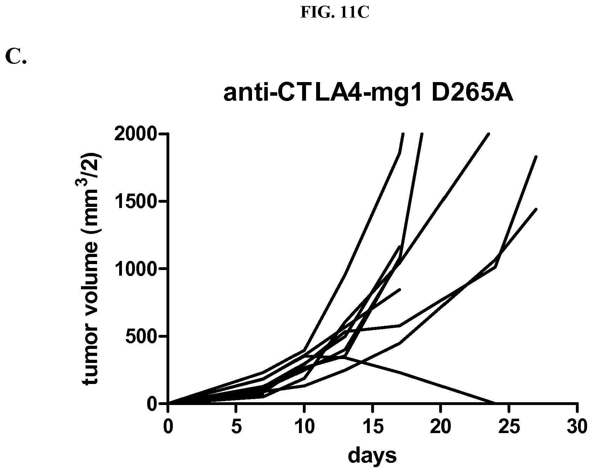

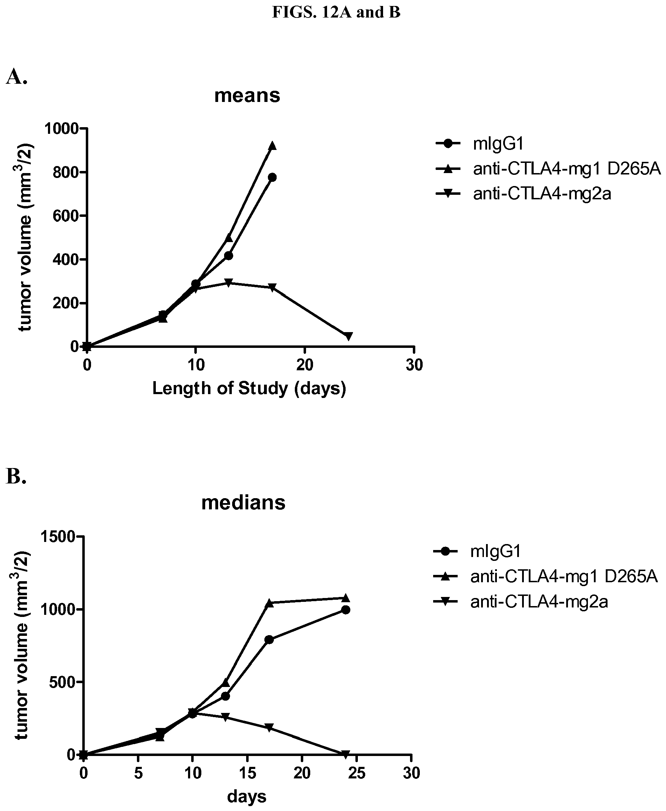

As shown in Figure 11, treatment of mice with the IgG2a anti-CTLA-4 antibody significantly inhibited tumor growth (Figure 11B), whereas IgG1D265A-treated tumors (Figure 11C) continued rapid growth, comparable to the uninhibited growth of IgG1 isotype control-treated tumors (Figure 11A). The changes in mean tumor volumes and median tumor volumes of the mice of groups treated with the different anti-CTLA-4 isotypes and the control, shown in Figures 12A and B, confirm the pronounced inhibitory effect of the IgG2a antibody on tumor growth, compared to the relative lack of inhibition of tumor growth exhibited by the IgG1D265A isotype and the mouse IgG1 control. A comparison of the percentage tumor growth inhibition effected by the IgG2a and mutated IgGlD265A isotypes at various time points post tumor-implantation is shown in Table 5.

**Table 5. Percentage mean inhibition of SalN tumor growth post tumor implantation**

| Day | % Mean tumor growth inhibition | |
|---|---|---|
| | Anti-CTLA-4-mIgG 1D265A | Anti-CTLA-4-mIgG2a |
| 7 | 9.8 | 3.1 |
| 10 | 2.1 | 8.2 |
| 13 | -20.0 | 30.2 |
| 17 | -18.8 | 65.3 |

Collectively, the data in Figures 11 and 12 and in Table 5 demonstrate that the IgG2a isotype of the anti-CTLA-4 antibody exhibits potent anti-tumor activity in this staged (therapeutic) SalN tumor model, in contrast to the mutated IgG1 anti-CTLA-4 antibody which lacks anti-tumor activity, similar to an IgG1 isotype control.

### EXAMPLE 7

### Effects of Anti-CTLA-4 Isotypes on SalN Intratumoral T Cell Subsets

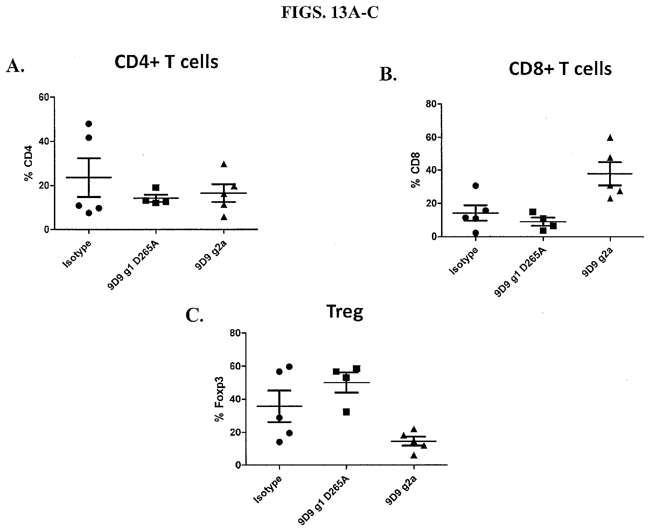

T cell subsets were analyzed in SalN tumor TILs from mice treated with the IgG2a and mutated IgG1 anti-CTLA-4 isotypes, along with an IgG1 isotype control. None of the antibodies tested caused any significant change in the percentage of CD4⁺ cells (Figure 13A). In contrast, treatment with anti-CTLA-4 having the 2a isotype resulted in a marked increase in the percentage of CD8⁺ cells (Figure 13B) and a concomitant significant reduction in the level of T_{regs} (Figure 13C).

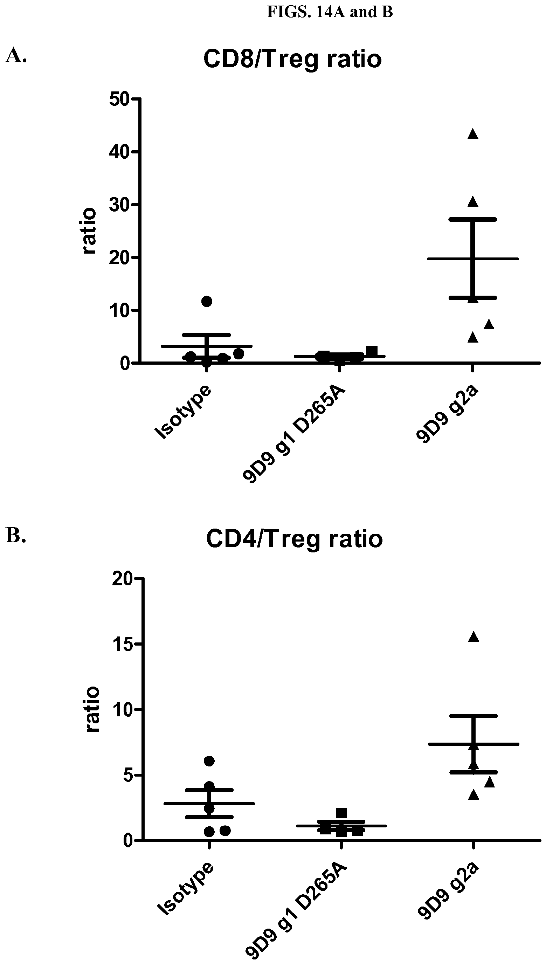

The increase in CD8⁺ T_{effs} and decrease in T_{regs} mediated by the IgG2a anti-CTLA-4 isotype translated into a T_{eff} to T_{reg} ratio (Figure 14A) that was significantly higher (at least about 6-fold higher) than the T_{eff} to T_{reg} ratios resulting from treatment with the IgG1 isotype or IgG1D265A anti-CTLA-4 antibody (Figure 14A). Consistent with the CT26 and MC38 tumor models, this high T_{eff} to T_{reg} ratios is indicative of robust anti-tumor activity. The CD4⁺ T_{eff} to T_{reg} ratio resulting from treatment with the IgG2a antibody was also higher than the T_{eff} to T_{reg} ratios induced by the isotype control or IgG1D265A anti-CTLA-4 antibody (Figure 14B). However, because the IgG2a antibody did not cause an increase in CD4⁺ cells compared to the IgG1 control or IgG1D265A, the increase in the T_{eff} to T_{reg} ratio was not as pronounced as for the CD8⁺ T_{eff} to T_{reg} ratio.

As antibodies to CTLA-4 (hamster; Leach *et al.,* 1996) and 9D9 (isotype 2b (our unpublished data)) were previously shown to be effective in the SalN fibrosarcoma as monotherapy even with a large tumor burden, it was expected that activation of T_{effs} may be solely responsible for the anti-tumor effect of anti-CTLA-4 antibody therapy in highly immunogenic tumors. However, the above data indicate that the activity of anti-CTLA-4 in the Sa1N model is mediated, at least in part, by the ability of certain isotypes, in particular the IgG2a isotype, to reduce the number of T_{regs} at the tumor site. A concomitant increase in CD8⁺ T_{effs} results in a marked increase in the T_{eff} to T_{reg} ratio. Nevertheless, preliminary data (not shown) also suggest that in mice bearing a low burden of an immunogenic tumor, *e.g.,* a syngeneic SalN tumor, activation of an anti-tumor response by anti-CTLA-4 antibodies, irrespective of isotype, may be mediated solely by the blocking of CTLA-4. In this setting, activation of T_{effs} alone may be sufficient to eliminate tumors without any concomitant reduction in T_{reg} numbers.

### EXAMPLE 8

### Effect of Anti-CTLA-4 Treatment on Myeloid Derived Suppressor Cells

In addition to T cells, myeloid derived suppressor cells (MDSC), defined by expression of the surface markers CD11b and Gr-1, were analyzed in tumors and spleens of anti-CTLA-4 antibody-treated MC38 tumor-bearing mice. MC38 colon tumor cells (2 x 10⁶) were implanted subcutaneously into C57BL/6 mice. At Day 7 post-implantation, tumor-bearing mice were randomized and received 3 doses of antibody by intraperitoneal injection (10 mg/kg) every 3 days. On Day 15 post-implantation, tumors were harvested, manually dissociated into single cell suspensions, and levels of intratumoral cytokines were assessed via bead-based cytokine arrays (FlowCytomix; Ebioscience, San Diego, CA).

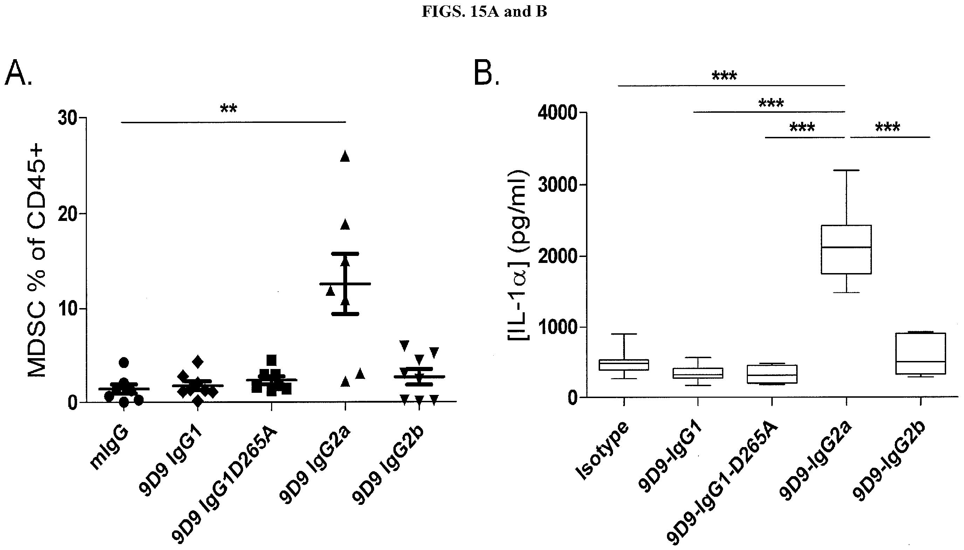

Figure 15 shows the assessment of numbers of MDSCs (CD11b⁺Gr-1⁺) among CD45⁺ cells (Figure 15A), as well as levels of interleukin 1-alpha (IL-1α) (Figure 15B). Data are representative of (A) two independent experiments with ≥ 3 mice per group or (B) three independent experiments with ≥5 mice /group/experiment. No changes were observed in splenic MDSC for any of the anti-CTLA-4 isotypes (data not shown). In contrast, however, MDSC numbers were substantially increased in tumors of mice treated with the IgG2a isotype (Figure 15A).

### EXAMPLE 9

### Intratumoral Cytokine Expression in Response to Anti-CTLA-4 Treatment

To determine whether the decrease in tumor-infiltrating T_{regs} and concomitant increase in effector CD8 numbers was associated with changes in T-cell function, cytokine levels present within the tumor microenvironment in each of the treatment groups in MC38 tumor-bearing mice were measured. Tumors were harvested into 1 ml of complete T cell medium (RPMI-1640 supplemented with 10% heat-inactivated fetal bovine serum (FBS), penicillin/streptomycin, and β-mercaptoethanol (Life Technologies, Grand Island, NY) in 24-well plates and manually dissociated into single cell suspensions. Cells and debris were spun down and supernatant was harvested and frozen to allow for batch processing of samples. Upon thawing, 25 µl of supernatant from each sample were assessed for concentrations of intratumoral IL-1α, IL-2, IL-4, IL-5, IL-6, IL-10, IL-13, IL-17A, IL-21, IL-22, IL-27, IP-10, GM-CSF, TNF-α, and IFN-γ in duplicate using a bead-based cytokine array according to the manufacturer's instructions (FlowCytomix; eBioscience, San Diego, CA).

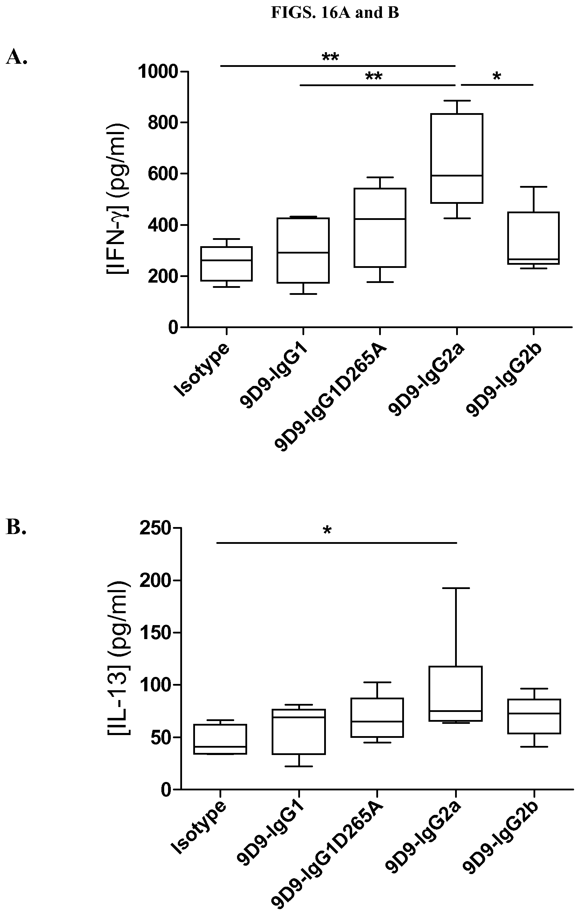

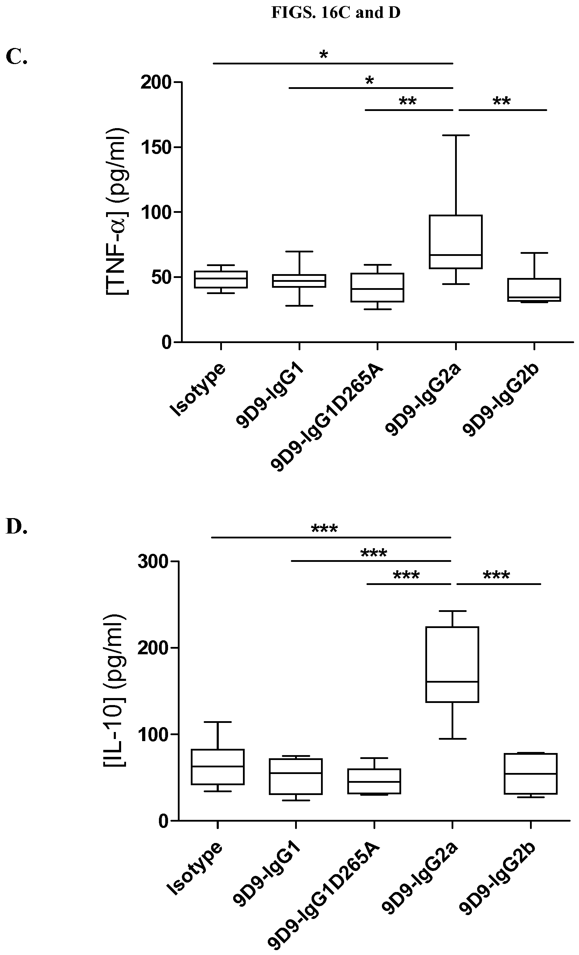

Anti-CTLA-4-IgG2a treatment resulted in the most pronounced increases in intratumoral levels of both T-helper (T_{H})1 and T_{H}2 cytokines, with significant enhancement of BFN-γ, TNF-α, IL-13, and IL-10 compared with each of the other isotype variants (Figure 16). Interestingly, the levels of IL-1α were also both specifically and significantly higher in tumors of mice that had been treated with CTLA-4-IgG2a (Figure 15B) compared with all other isotypes. This upregulation was unique to treatment with the IgG2a variant and not simply associated with tumor destruction and regression since this increase in IL-1α was not observed with MC38 tumor-bearing mice treated with the combination of anti-PD-1 and anti-CTLA-4-IgG2b antibodies, which leads to similar antitumor efficacy and expansion of T_{effs} (data not shown).

### REFERENCE EXAMPLE 10

### Generation of Different Anti-mGITR Antibody Isotypes

GITR (glucocorticoid-induced tumor necrosis factor (TNF) receptor), a type I transmembrane protein with homology to other TNF receptor family members such as OX40, CD27, and 4-1BB (Nocentini and Riccardi, 2005). In humans, GITR is normally expressed at low levels on resting CD4⁺Foxp3⁻ and CD8⁺ T cells, but is constitutively expressed at high levels on CD4⁺CD25⁺Foxp3⁺ T_{regs}. Expression increases on all 3 subpopulations following T cell activation (Cohen *et al.*, 2010). Our own data show that in mice, GITR is constitutively expressed at high levels on all T cell subsets *(see* Example 18).

DTA-1 is an agonistic rat anti-mouse GITR antibody (Shimizu *et al.*, 2002; eBioscience, San Diego, CA). This IgG2b antibody has been shown to modulate both T_{regs} and T_{effs} during treatment of B16 melanoma. In addition, GITR expression by both T_{effs} and T_{regs} was needed for the full effects of DTA-1. Cohen *et al.* (2010) have suggested that while GITR ligation by DTA-1 does not globally abrogate T_{reg} suppressive activity, it impairs T_{reg} tumor infiltration and leads to loss of Foxp3 expression within intra-tumor T_{regs}, implying a localized abrogation of suppression. The net result is an augmented intra-tumor T_{eff}:T_{reg} ratio and greater T_{eff} activation and function within the tumor.

DTA-1 blocks the interaction between GITR and GITR ligand (GITRL) and the soluble antibody is effective in promoting a cell response *in vitro.* It is also efficacious in various tumor models in inhibiting tumor growth (*see, e.g.,* Turk *et al.*, 2004; Cohen *et al.*, 2010). As described in Example 1 for 9D9, four isotypic variants of DTA-1 were generated, *i.e.,* mIgG1, mIgG1-D265A, mIgG2a, and rIgG2b (the original rat isotype, which is equivalent to mouse IgG2a).

### REFERENCE EXAMPLE 11

### Anti-Tumor Activity of Variant Anti-GITR Isotypes in MC38 Tumor Model

### Experiment MC38 #1

The anti-tumor activity of the different anti-GITR (DTA-1) isotypes was assessed in a staged MC38 colon adenocarcinoma tumor model as described in Example 4. C57BL/6 mice were each subcutaneously injected with 2 x 10⁶ MC38 tumor cells. After 7 days, the mice were randomized into 5 treatment groups and test antibodies were administered IP on Days 7, 10 and 14 at 200 µg per dose in a volume of 200 µl as follows: Group 1: mouse IgG1 control (IgG); Group 2: anti-CTLA-4 mouse IgG2a Ab (9D9-m2a); Group 3: anti-GITR rat IgG2b Ab (DTA-r2b); Group 4: anti-GITR mouse IgG1 Ab (DTA-mg1); and Group 5: anti-GITR mouse IgG 2a Ab (DTA-m2a). Tumors and spleens were harvested on Day 15. (Biophysical analysis (by SEC) demonstrated that with the exception of the DTA-r2b antibody, all the reengineered DTA-1 monoclonal antibodies were highly aggregated, which later prompted the repetition of this experiment as Experiment MC38 #2 described below.)

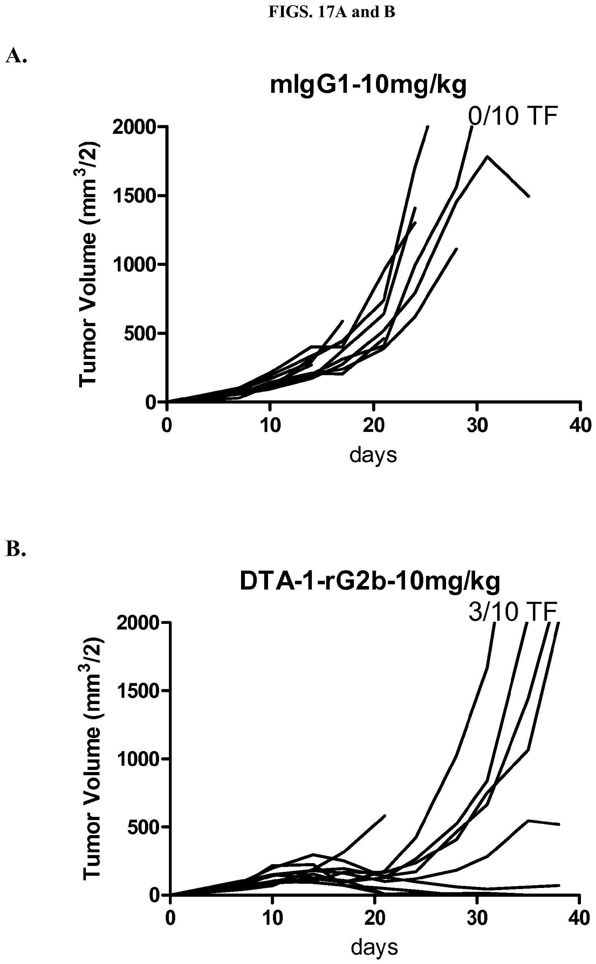

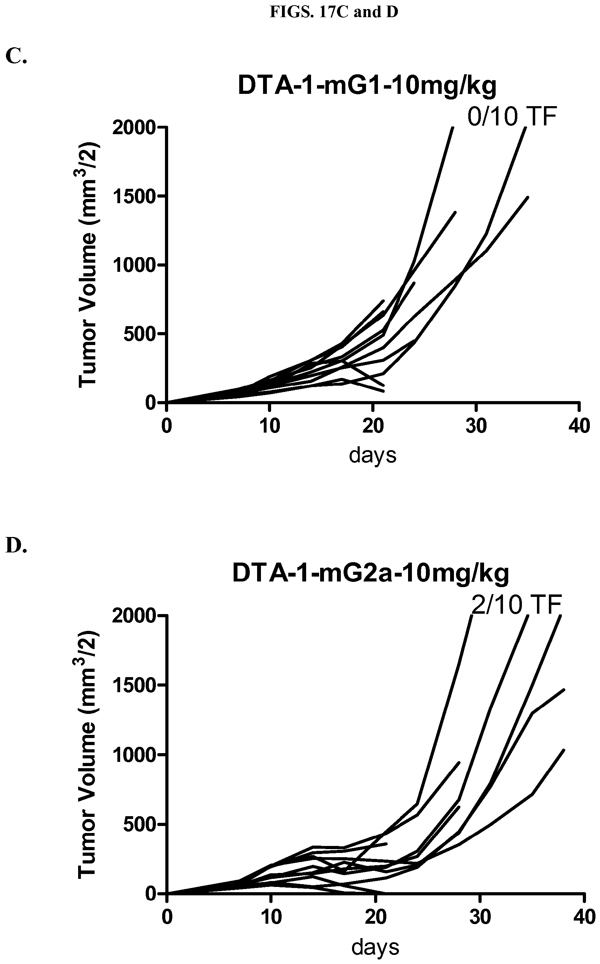

Figure 17B shows that the IgG1 anti-GITR-treated tumors grew at a comparable rate to that of tumors treated with the mouse IgG1 control (Figure 17A), none of the 10 mice being tumor free (TF) by the end of monitoring the mice. However, similar to the pattern seen with the IgG2a and IgG2b anti-CTLA-4 antibodies, DTA-r2b (Figure 17C) and DTA-m2a (Figure 17D) significantly reduced the rate of tumor growth, with 3 and 2 out of 10 mice, respectively, being TF.

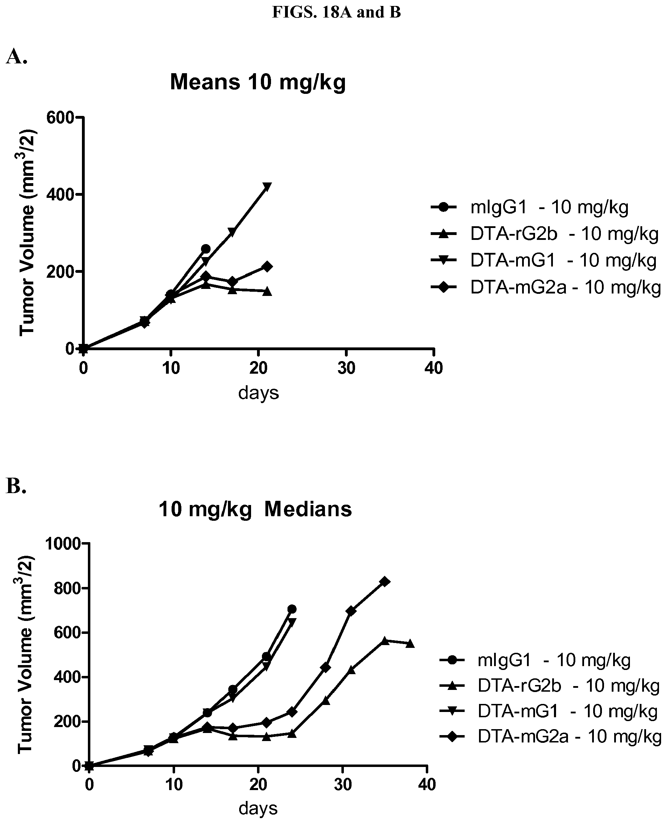

The changes in mean tumor volumes and median tumor volumes of the mice of groups treated with the different anti-GITR isotypes are plotted in Figures 18A and B. These plots confirm the individual mouse data shown in Figure 17 that the IgG2b isotype of the anti-GITR antibody exhibits the most potent inhibitory effect on MC38 tumor growth, with the IgG2a isotype only slightly less potent. The IgG1 isotype shows little inhibition of tumor growth, with the mean and median tumor volumes being similar to those in mice treated with the mouse IgG control.

### Effects of anti-GITR isotypes on MC38 T cell subsets in TILs and spleen

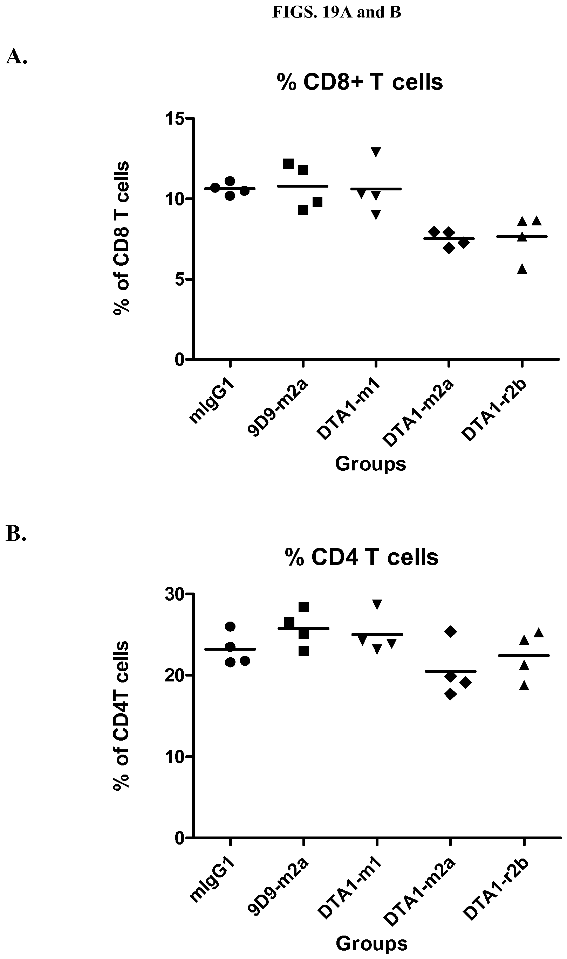

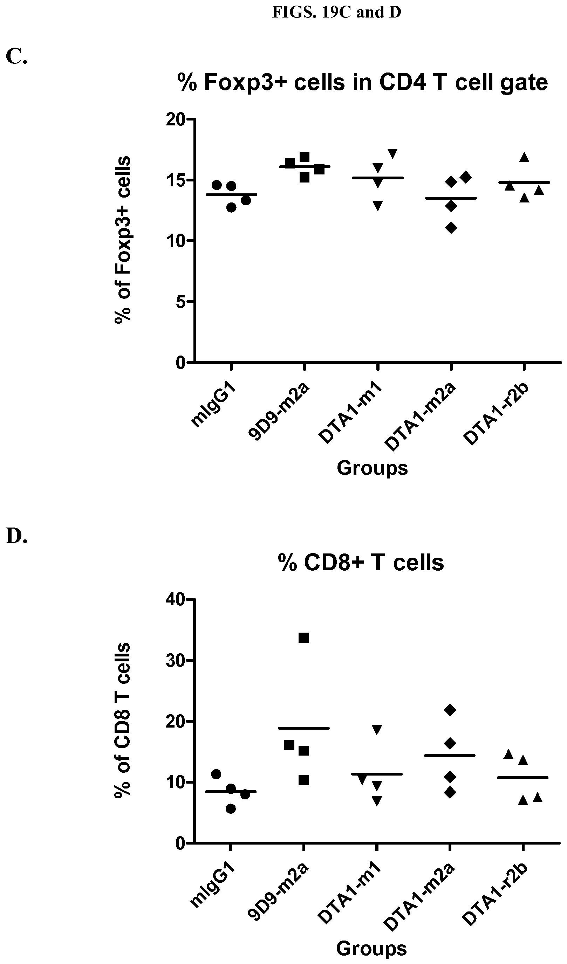

The populations of T cell subsets in MC38 TILs and spleens from mice treated with the different anti-GITR isotypes were compared. In the spleen, DTA-m2a and DTA-r2b caused a slight reduction in the level of CD8⁺ cells whereas 9D9-m2a and DTA-m1 did not alter CD8⁺ T cell levels (Figure 19A). None of the isotype variants tested had a significant effect on the percentage of CD4⁺ or CD4⁺Foxp3⁺ cells in the spleen (Figures 19B and C).

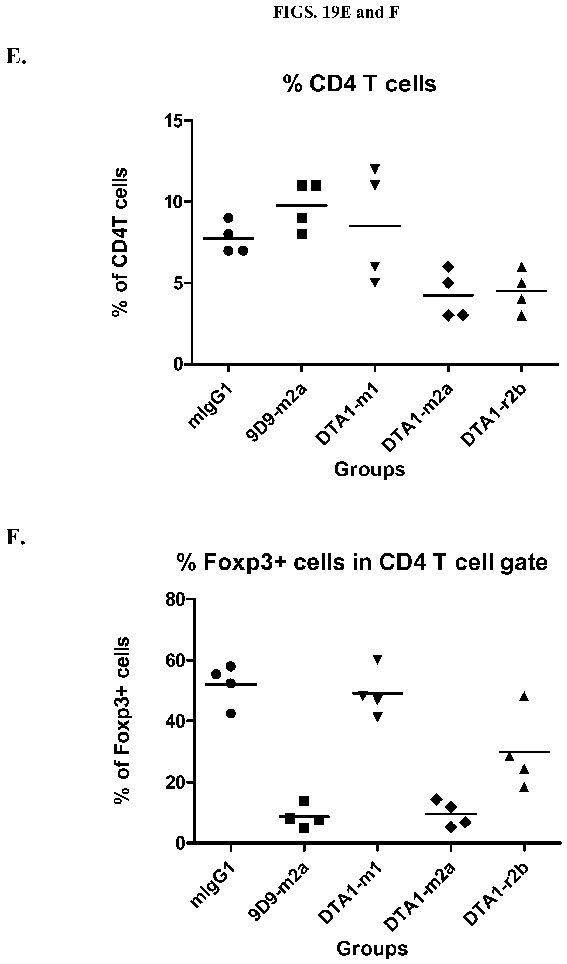

In TILs, 9D9-m2a caused at least a 2-fold increase in the percentage of CD8⁺ cells compared to both the mouse IgG1 control (Figure 19D), consistent with the results in Example 5. DTA-m2a had a less pronounced effect, increasing the percentage of CD8⁺ cells about 50%, whereas DTA-m1 and DTA-r2b caused no, or only a marginal increase in, the percentage of CD8⁺ cells compared to the mouse IgG1 isotype control (Figure 19D). 9D9-m2a caused a small increase in the percentage of CD4⁺ cells compared to the mouse IgG1 isotype control, whereas DTA-m1 caused no change in CD4⁺ (Figure 19E). In contrast, both DTA-m2a and DTA-r2b reduced CD4⁺ percentages by 40-50% compared to both the mouse IgG1 isotype (Figure 19E).

The most dramatic effects were seen with the levels of CD4⁺Foxp3⁺ T_{regs} among the TILs. While DTA-m1 had no effect on this population of T cells, 9D9-m2a and DTA-m2a induced an approximately 6-fold reduction in the level of CD4⁺Foxp3⁺ T_{regs} compared to the IgG1 isotype and DTA-m1 (Figure 19F). These data confirm the effect seen in Example 5 for the IgG2a anti-CTLA-4 isotype, and demonstrate that the IgG2a variant of anti-GITR similarly reduces the level of T_{regs} specifically in the tumor environment. Thus, similar to the IgG2a anti-CTLA-4 isotype, the IgG2a anti-GITR isotype also induces an increase in CD8⁺ T_{effs} and decrease in T_{regs} at the tumor site which translates into an elevated T_{eff} to T_{reg} ratio that is indicative of robust anti-tumor activity. DTA-r2b also induced significant reduction in the level of CD4⁺Foxp3⁺ T_{regs} compared to the IgG1 control, though not as pronounced a reduction as that induced by 9D9-m2a and DTA-m2a, consistent with the lower binding of the rat IgG2b Fc region to murine activating FcyRs. These data show that the agonist anti-GITR antibody behaves similarly to the antagonistic anti-CTLA-4 antibody in requiring engagement of activating FcyRs for depletion activity.

Flow cytometric measurement of the level of GITR expression on different subsets of T cells in MC38 TILs and spleen showed that GITR was most highly expressed on T_{regs} at the tumor site, that level of expression being higher than on T_{regs} in the periphery or CD8⁺ T_{effs} at the tumor site, which in turn exhibited higher expression than CD8⁺ or CD4⁺ T_{effs} in the periphery *(see* Example 18). The lowest relative level of GITR expression was seen on CD4⁺ T_{effs} at the tumor site. These data suggest a mechanism whereby T cell depletion activity assists in stimulating a T cell response and thereby enhance anti-tumor efficacy of a Fc fusion protein if the target of the of the Fc fusion protein is highly expressed on T_{regs} at the tumor site relative to expression of the target on T_{effs} at the tumor site, and the Fc fusion protein binds to an activating FcR that mediates depletion of the target cell.

### Experiment MC38 #2

Because of the aggregation encountered with the DTA-1 variants (except the commercially obtained original form of DTA-r2b), a new set of isotypic variants were reengineered to obtain DTA-1 antibodies that do not aggregate. The aggregation observed was traced to an extra amino acid that had inadvertently been incorporated into the light chain of the engineered isotypic variants, and the problem was alleviated by removal of this extraneous amino acid. The reengineered antibodies were used in this Experiment #2. The anti-tumor activity of the reengineered anti-GITR (DTA-1; GITR.7 series) isotypes was assessed using a staged MC38 model. C57BL/6 mice were each subcutaneously implanted with 2 x 10⁶ MC38 cells. After 7 days, the mice were randomized into 7 treatment groups so as to have comparable mean tumor volumes of about 148 mm³/2), and test antibodies were administered IP on Days 7, 10 and 14 at 200 µg per dose (except for the mIgG control which was administered at a dose of 200 µg) as follows: Group 1: mouse IgG1 control (mIgG or "isotype"); Group 2: anti-GITR mouse IgG1Ab (mGITR.7.mgl); Group 3: anti-GITR mouse IgG1D265A isotype (mGITR.7.mg1-D265A); Group 4: anti-GITR mouse IgG2a Ab (mGITR.7.mg2a); Group 5: anti-GITR mouse IgG2b Ab (mGITR.7.mg2b); Group 6: anti-GITR rat IgG2b Ab (mGITR.7.r2b or DTA-1-rG2b); and Group 7: anti-CTLA-4 mouse IgG2a Ab (9D9-mg2a). Tumors and spleens were harvested on Day 15.

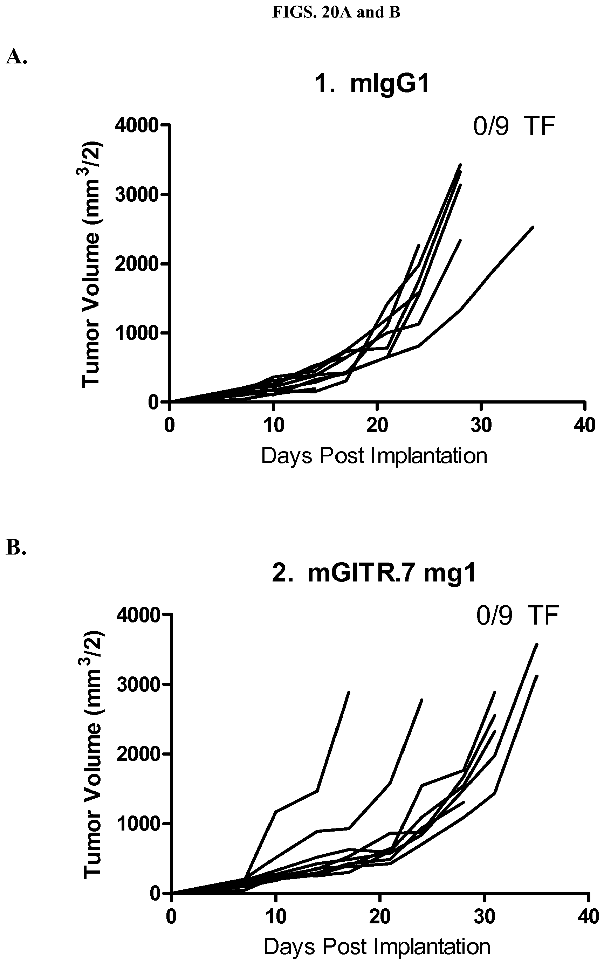

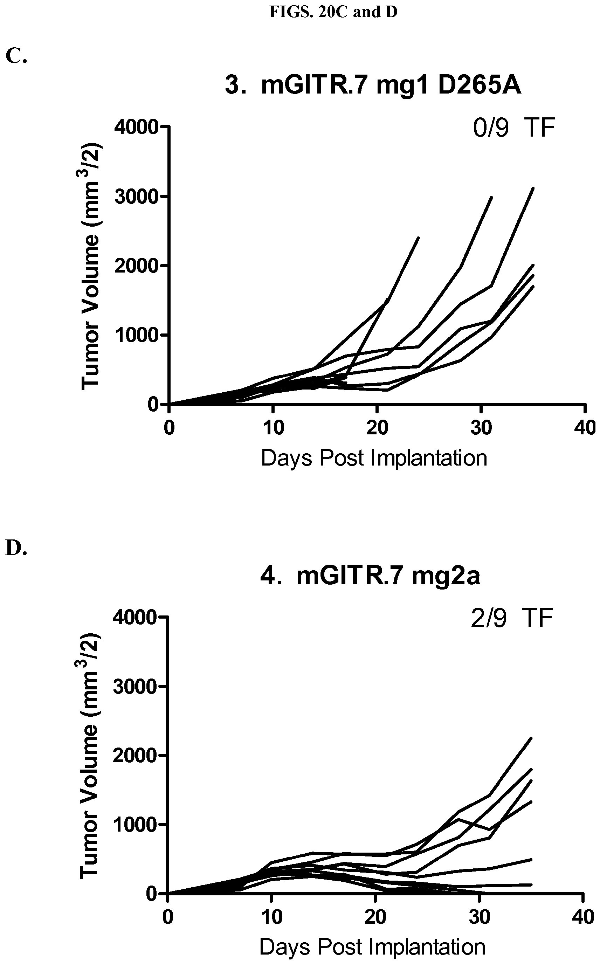

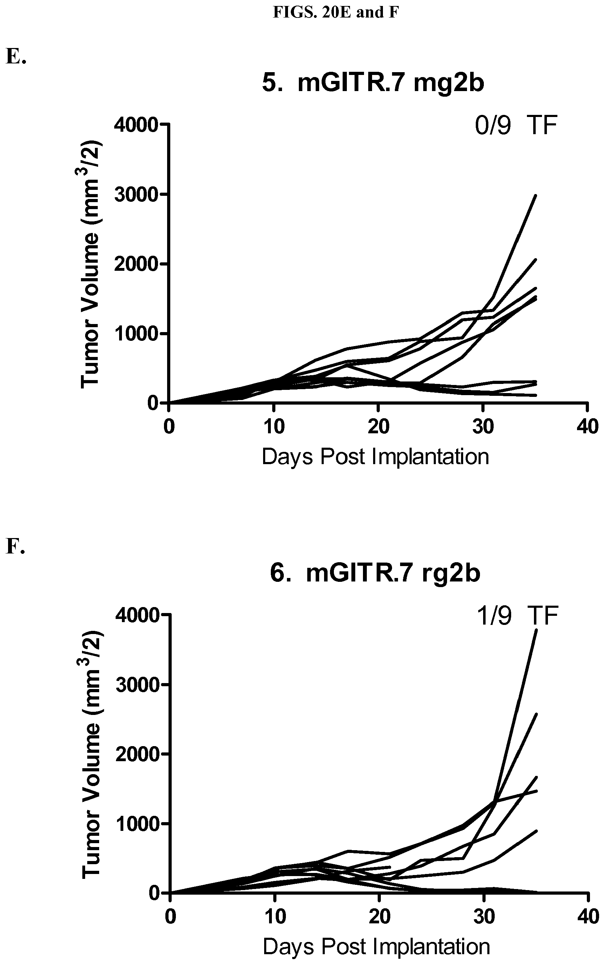

Figures 20B and C show that the IgG1 and IgG1-D265A anti-GITR-treated tumors grew at a comparable rate to that of tumors treated with the mouse IgG1 control (Figure 20A). In each case none of the 9 mice being TF by the end of monitoring the mice 35 days post-implantation. However, similar to the results in Experiment MC38 #1, mGITR.7.mg2a (Figure 20D) induced the greatest inhibition of tumor growth, with 2 out of the 9 mice being TF. The mouse and rat anti-GITR-2b antibodies also significantly reduced the rate of tumor growth to similar extents (Figures 20E and F), though the rat 2b antibody produced 1 TF mouse while the mouse 2b antibody did not produced any TF mice 35 days post-implantation.

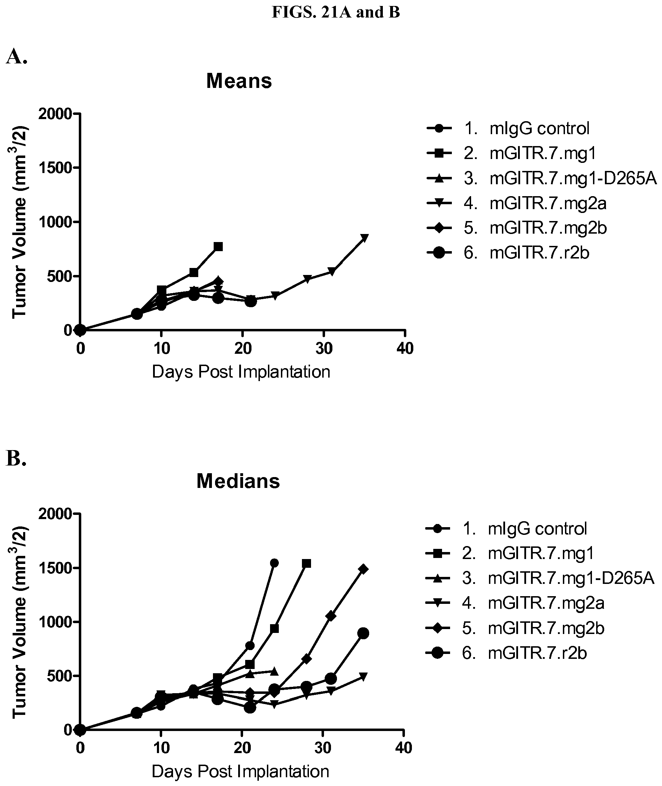

Changes in mean tumor volumes and median tumor volumes are shown in Figures 21A and B. The trends are similar to those seen in MC38 Experiment 1 except that, similar to the data obtained with anti-CTLA-4 antibodies, the IgG2a anti-GITR isotype is the most potent inhibitor of MC38 tumor growth, while the IgG2b isotype exhibits significant, but lower, potency in inhibiting tumor growth. The IgG1 and IgG1-D265A isotypes showed a low-level inhibition of tumor growth compared to the mouse IgG control.

### Effects of anti-GITR isotypes on T_{reg} populations in MC38 tumor model

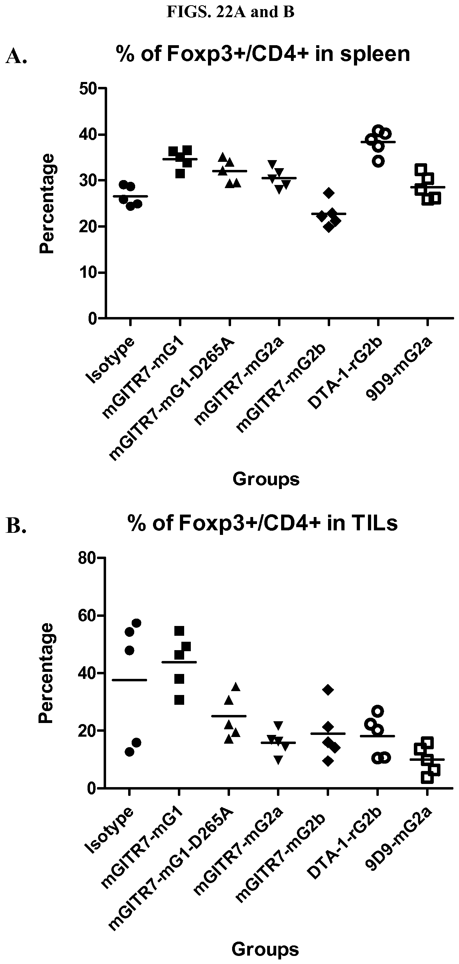

The effects of the different anti-GITR isotypes on the populations of T_{regs} in TILs and spleens from the treated mice are shown in Figure 22. As observed in Experiment #1, none of the isotype variants tested had a huge effect on the percentage of CD4⁺Foxp3⁺ T_{regs} in the spleen: the strongest effect was a less that 40% increase induced by treatment with the rat anti-GITR IgG2b isotype, whereas the mouse anti-GITR IgG2b isotype marginally reduced the percentage of CD4⁺Foxp3⁺ T_{regs}. The other anti-GITR isotypes tested and the anti-CTLA-4 IgG2a antibody marginally increased the percentage of T_{regs} (Figure 22A).

In contrast, in the TILs, with the exception of the IgG1 isotype, which caused no change compared to the isotype control, all of the antibodies tested induced significant reductions in the percentage of T_{regs}. Anti-CTLA-4 antibody 9D9-mG2a cause an approximately 4-fold reduction in the level of CD4⁺Foxp3⁺ T_{regs} compared to the IgG1 isotype; the anti-GITR mouse 2a and 2b isotypes and the rat 2b isotype all lowered the level of T_{regs} about 2-fold, and the IgG1-D265A mutant caused a slightly lower reduction (Figure 22B). These data confirm the effects seen in Experiment #1 in demonstrating that anti-GITR mG2a, mG2b and rG2b isotypes induce significant T_{reg} depletion in the tumor environment, which correlates with tumor growth inhibition.

The data obtained in Experiment MC38 #2 are largely consistent with those obtained in Experiment #1, which suggests that aggregation of the antibodies did not unduly interfere with the activities of the antibodies. Possibly, the aggregated antibodies are rapidly flushed in the mice and, thus, antibody aggregation may not be a significant problem in the present *in vivo* assays.

### REFERENCE EXAMPLE 12

### Anti-Tumor Activity of Variant Anti-GITR Isotypes in a Staged SalN Tumor Model

The anti-tumor activity of anti-GITR was also assessed in a SalN sarcoma model in A/J mice. The mice were subcutaneously injected with 2 x 10⁶ SalN cells per implant. After 7 days, tumor volumes were determined and mice were randomized into treatment groups so as to have comparable mean tumor volumes (about 75 mm³/2). Anti-GITR (DTA-1) antibodies engineered to have different isotypes as described in Example 11, Experiment MC38 #1, were administered IP on Days 7, 10 and 12 at 200 µg per dose.

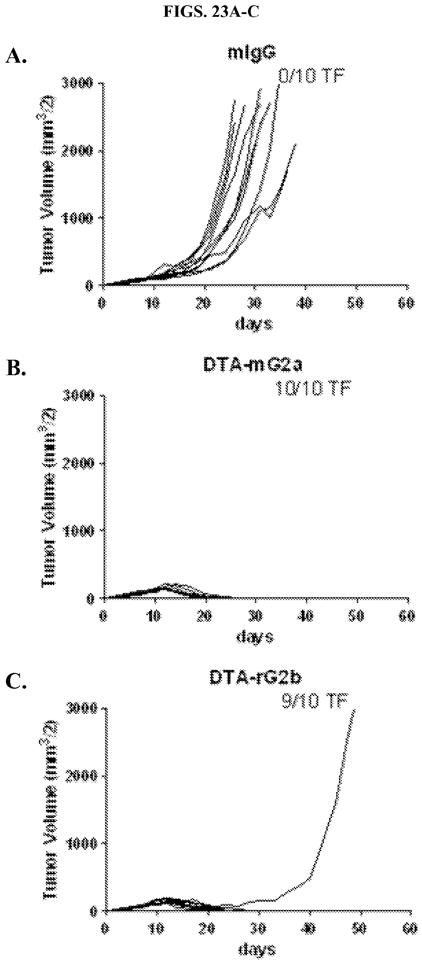

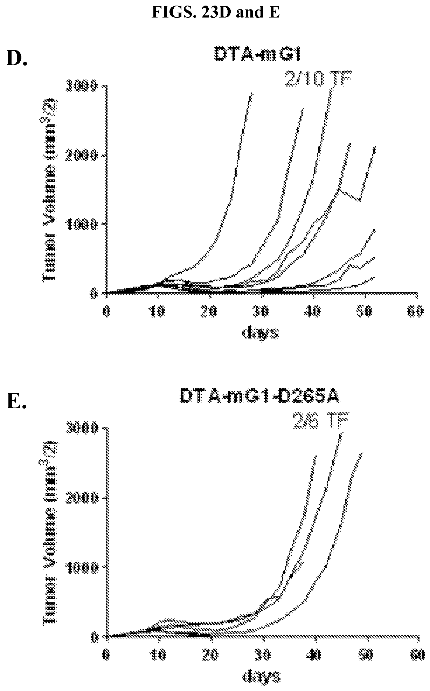

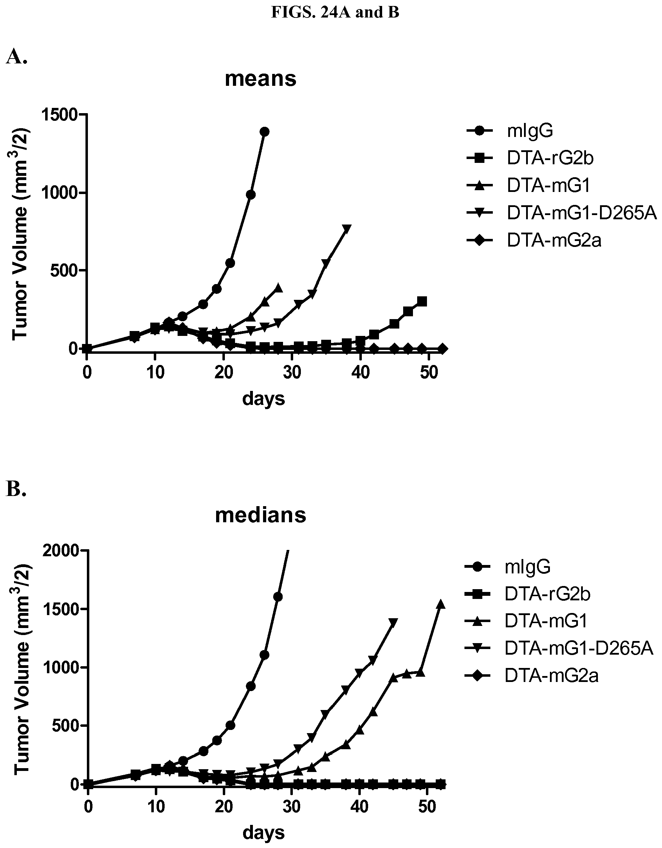

The effects on tumor growth are shown in Figure 23. Treatment with the IgG2a anti-GITR antibody completely inhibited tumor growth and all 10 mice were TF by about Day 20 post-implantation (Figure 23B), and the rat IgG2b isotype had a similar effect with 9 out of 10 mice TF by about Day 20 (Figure 23C). The IgG1 (Figure 23D) and IgG1D265A (Figure 23E) isotypes inhibited tumors to some extent compared to the uninhibited growth of IgG1 isotype control-treated tumors (Figure 23A) but this was much less than the inhibition seen with the mIgG2a and rIgG2b isotypes. The changes in mean tumor volumes and median tumor volumes, shown in Figures 24A and B, confirm the virtually complete inhibitory effect of the mIgG2a and rIgG2b antibodies on tumor growth, compared to much lower inhibition of tumor growth exhibited by the mIgG1 and mIgG1-D265A isotypes.

Collectively, the data in Figures 23 and 24 confirm the data obtained with the MC38 tumor model (Example 11) showing that the anti-GITR mIgG2a and rIgG2b isotypes exhibit potent anti-tumor activity in contrast to the mIgG1 (and mIgG1-D265A) isotypes which exhibit much lower anti-tumor activity.

### Effects of anti-GITR isotypes on T_{reg} populations in SalN tumor model

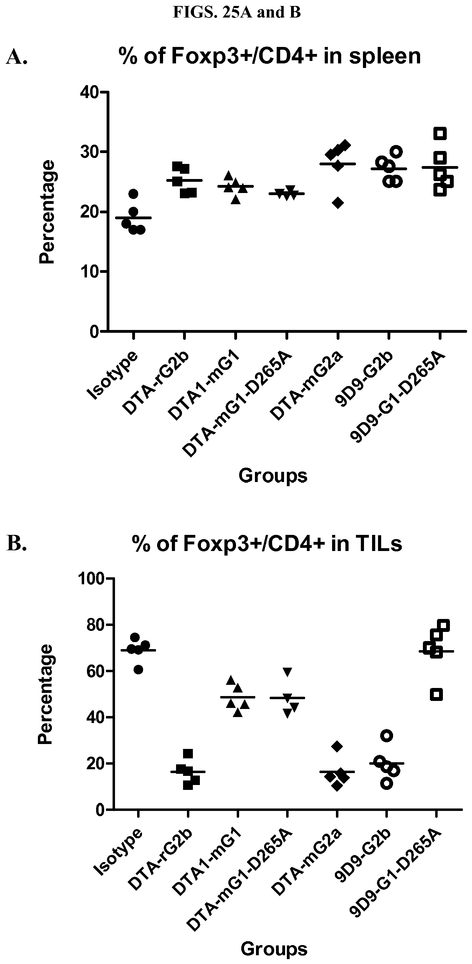

The effects of the different anti-GITR isotypes on the populations of T_{regs} in SalN TILs and spleens from the treated mice are shown in Figure 25. All of the anti-GITR isotype variants tested induced relatively small increases of about 20-40% in the level of CD4⁺Foxp3⁺ T_{regs} in the spleen. The highest increase was induced by treatment with the mouse anti-GITR IgG2a isotype, which caused the same increase as treatment with the anti-CTLA-4 IgG2b and IgG1-D265A antibodies (Figure 25A). The latter anti-CTLA-4 isotypes were used as positive controls in this GITR study as T_{reg} depletion had previously been observed with IgG2b isotype.

In contrast to the effect of T_{regs} in the periphery, the anti-GITR m2a and r2b isotypes, as well as the anti-CTLA-4 2b isotypes all lowered the level of T_{regs} at the tumor site by at least 3.5-fold (Figure 25B). The anti-GITR IgG1 isotype and the IgG1-D265A mutant both induced smaller reductions of about 35% in the percentage of T_{regs}, whereas the anti-CTLA-4 IgG1-D265A mutant caused no change in the percentage of T_{regs} in TILs (Figure 25B). Thus, as observed in the MC38 tumor model, the anti-GITR mG2a and rG2b isotypes induces significant T_{reg} depletion in the tumor environment, much more so than the IgG1 and IgG1-D265A antibodies, which correlates with tumor growth inhibition.

### EXAMPLE 13

### Effect of Afucosylation on Anti-Tumor Activity of Variant Anti-CTLA-4 Isotypes in MC38 Tumor Model

The anti-tumor activity of nonfucosylated (NF) anti-CTLA-4 (9D9) isotypes was assessed in the MC38 tumor model. These nonfucosylated variants were generated using a CHO cell line lacking fucosyltransferase for transfections. C57BL/6 mice were subcutaneously injected with 2 x 10⁶ MC38 tumor cells per implant, and after 11 days mice were randomized into treatment groups having a mean tumor volume of about 230 mm³/2. Anti-CTLA-4 antibodies of four different isotypes (IgG1D265A, IgG2a, IgG2a-NF, IgG2b and IgG2b-NF), were administered IP on Days 11, 13 and 15 at 200 µg per dose in a volume of 200 µl.

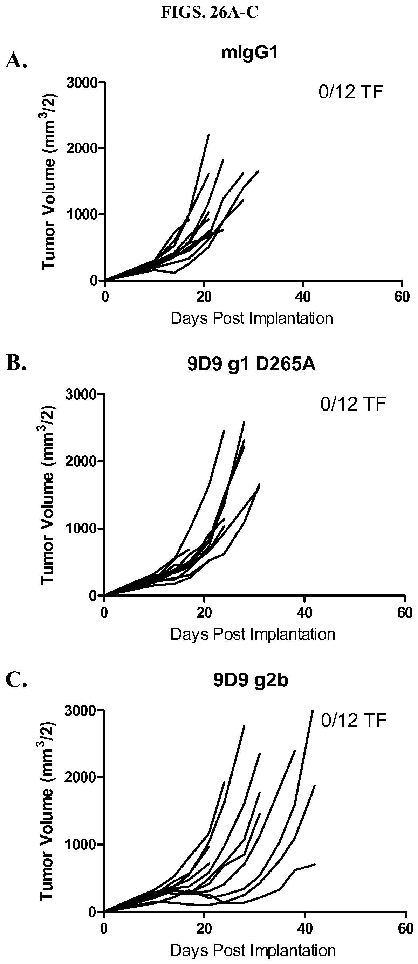

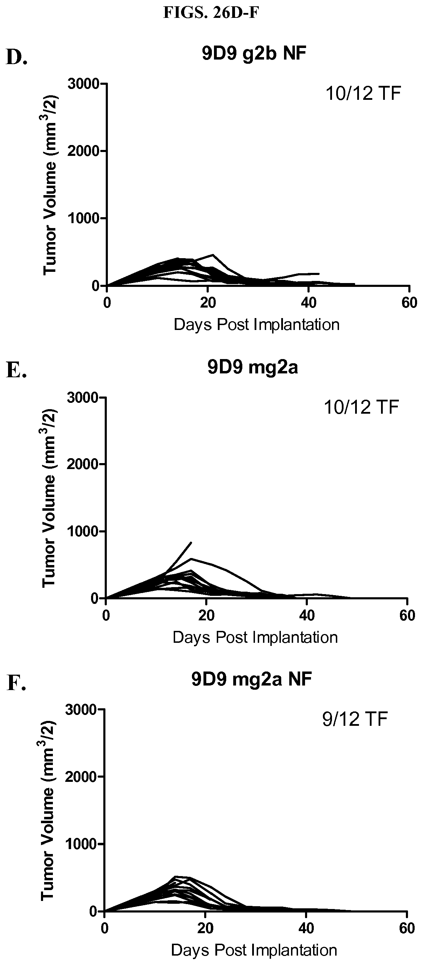

As previously observed *(see* Example 4), the IgG1D265A mutant (Figures 26B) had a minimal effect on inhibiting growth of tumors compared to the mouse IgG1 control (Figure 26A), whereas the IgG2b isotype noticeably inhibited tumor growth (Figure 27C), though to a lesser extent than the IgG2a (Figure 26E) which potently reduced the rate of tumor growth resulting in 10 out of 12 TF mice. Afucosylation of the IgG2b isotype dramatically potentiated its tumor- inhibiting activity (Figure 26D), resulting in 10 out of 12 TF mice, similar to the activity seen with the IgG2a isotype. These data confirm that afucosylation, which is known to increase binding of the Fc region to activating FcRs, may be used to increase depletion of T_{regs} at the tumor site and improve the anti-tumor efficacy of Treg-targeting Fc fusion proteins. The nonfucosylated IgG2a isotype exhibited similar inhibition of tumor growth (Figure 26F) to the normal IgG2a isotype (Figure 26E). The IgG2a isotype is so potent in inhibiting tumor growth that no enhancement is observed with the IgG2a-NF variant.

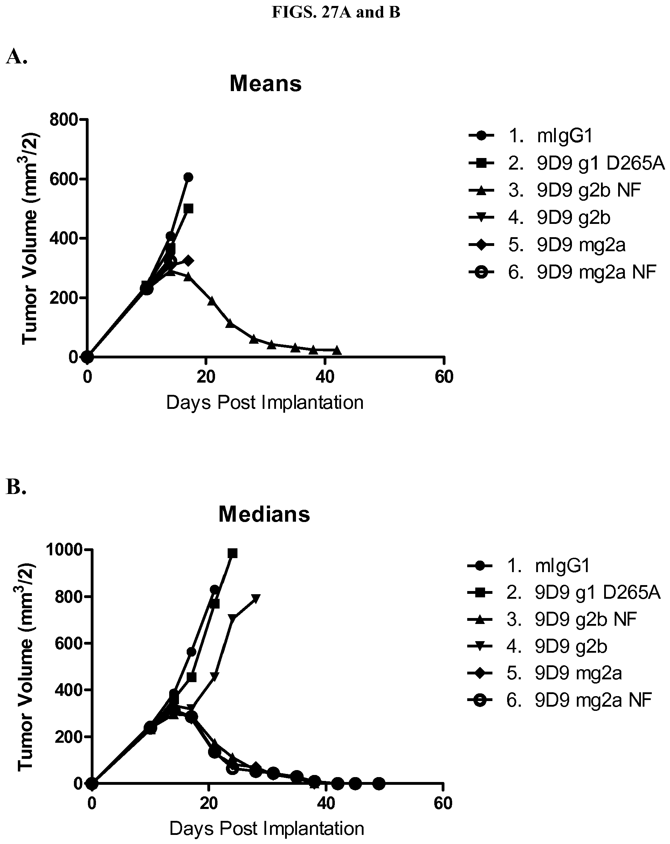

The changes in mean and tumor volumes in the treated groups of mice are shown in Figures 27A and B, which confirm the individual mouse data shown in Figure 26 and illustrate the high potency of the IgG2b-NF, IgG2a and IgG2a-NF isotypes compared to the IgG1D265A and IgG1 isotypes.

### REFERENCE EXAMPLE 14

### Anti-Tumor Activity of Variant Anti-OX40 Isotypes in Murine CT26 Tumor Model

In order to determine the relative anti-tumor potency of different isotypes of an agonistic anti-OX40 antibody, three isotypic variants of anti-OX40 antibody OX86 (Al-Shamkhani *et al.*, 1996) were engineered: anti-OX40 rat IgG1 (OX40-rg1), anti-OX40-mouse IgG1 (OX40-mg1), and anti-OX40 mouse IgG2a (OX40-mg2a). These isotype variants were tested together with a mouse IgG1 isotype control (a recombinant human anti-diphtheria toxin antibody with a mouse IgG1 isotype) for anti-tumor activity in a syngeneic CT26 colon carcinoma mouse model.

BALB/c mice were subcutaneously injected with 1 x 10⁶ CT26 tumor cells. Mice were treated IP with the antibodies, formulated in PBS, on Days 3, 7 and 10 at 200 µg per dose in a volume of 200 µl. Tumor volumes were measured twice weekly.

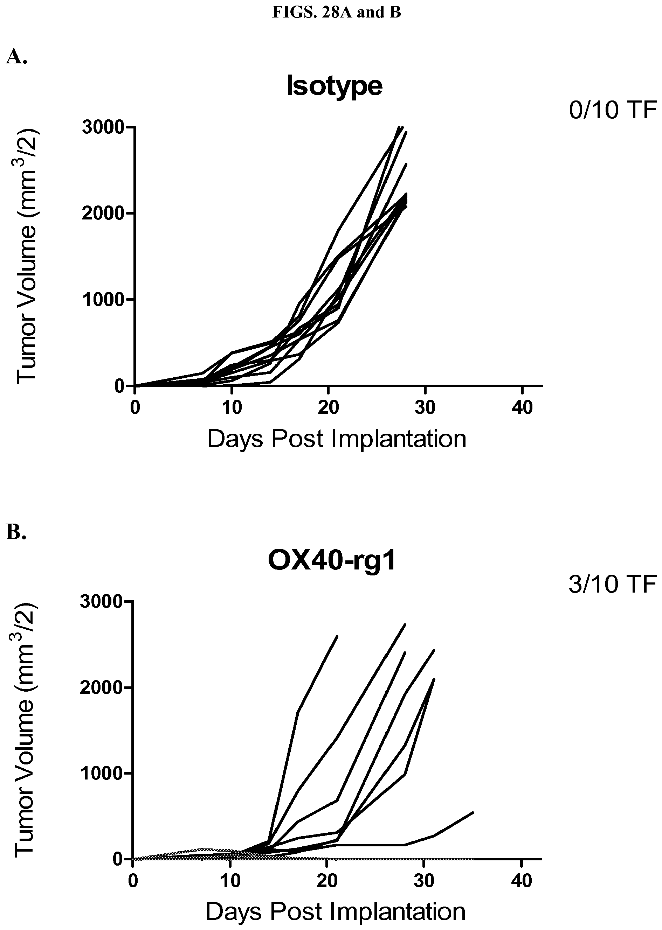

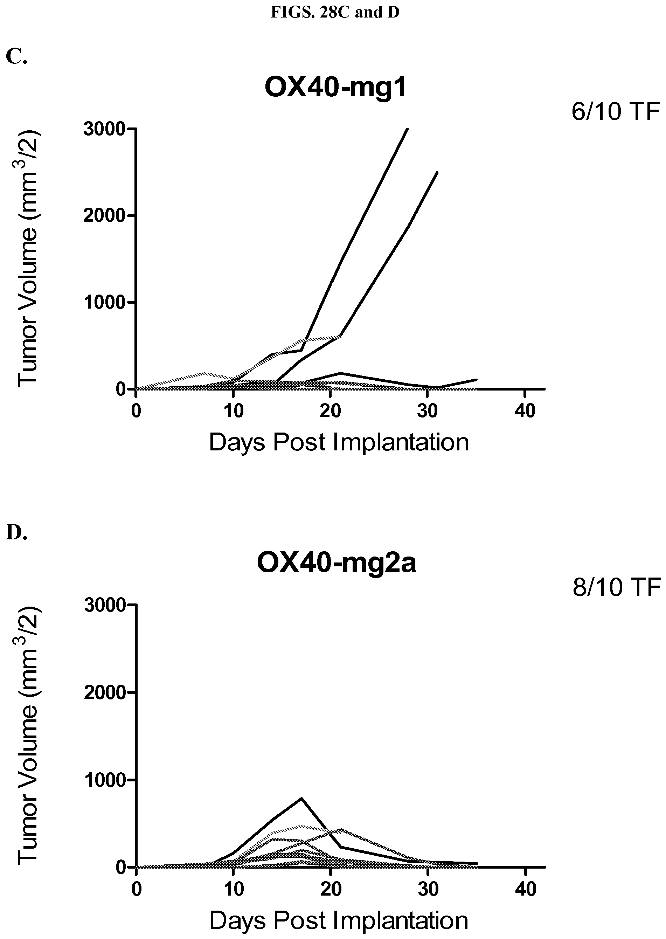

As shown in Figure 28, the anti-OX40 rat IgG1 isotype exhibited a moderate level of tumor growth inhibition (Figure 28B) compared to control IgG (Figure 28A) with 3 of 10 mice treated with OX40-rg1 being TF after up to 35 days, whereas the OX40-mg1 isotype exhibited significant tumor growth inhibition with 6 of 10 mice treated with OX40-mg1 being TF (Figure 28C). However, as observed with anti-CTLA and anti-GITR antibodies, the OX40-m2a isotype showed the most potent anti-tumor activity with 8 of 10 mice treated with OX40-mg2a being TF (Figure 28D). These data demonstrate that the anti-mOX40 isotype (OX40-mg2a) that preferentially binds to activating mouse Fc receptors displays superior anti-tumor efficacy over isotype variants (OX40-rG1 and OX40-mG1) that preferentially bind to the murine inhibitory Fc receptor, FcRIIb, in the CT26 tumor model. Similar data was observed in C57BL/6 mice inoculated subcutaneously with 2 x 10⁶ MC38 colon carcinoma cells (data not shown).

The experiment was repeated using a staged (therapeutic) model by implanting 1 x 10⁶ CT26 tumor cells into BALB/c. After 7 days, tumor volumes were determined and mice were randomized into treatment groups so as to have comparable mean tumor volumes (45-50 mm3/2). Antibodies (OX40-mG1, OX40-mG1D265A, OX40-mGl and a mouse IgG1 isotype control) were administered intraperitoneally on Days 7, 10, and 14 at 200 µg per dose, and tumor volumes were measured twice weekly.

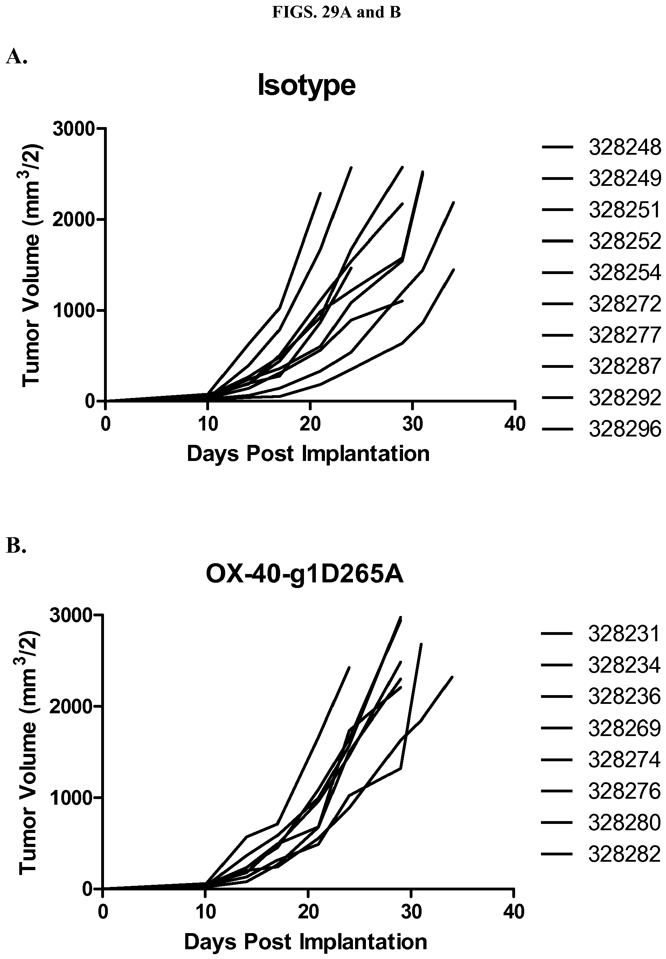

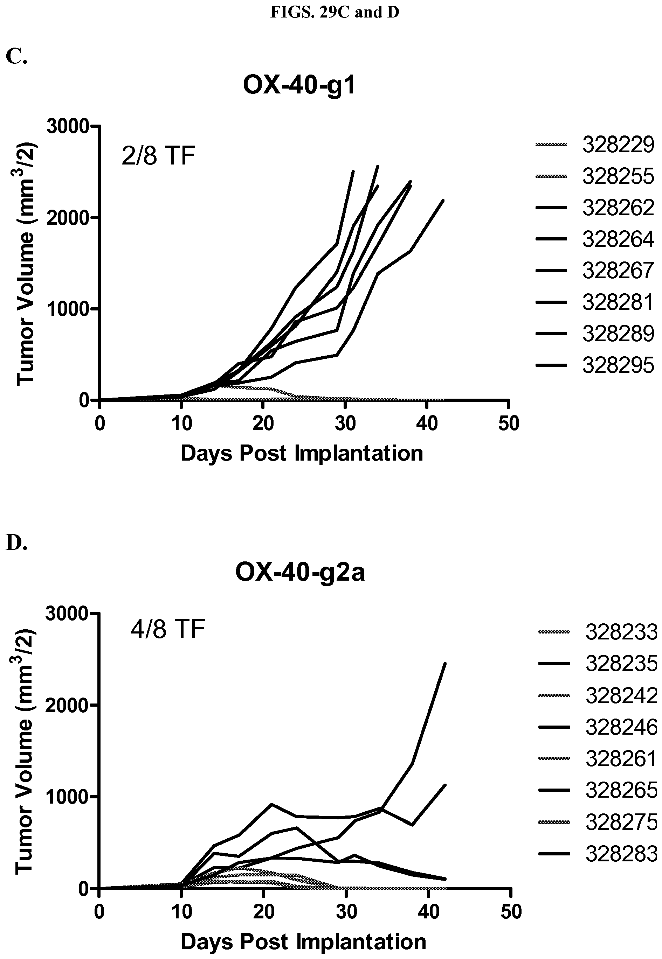

The results, shown in Figure 29, are consistent with those shown in Figure 28 except that somewhat lower levels of tumor inhibition were observed as the tumors had been allowed to grow for a longer time before administration of the antibodies. Thus, as seen previously, the OX40-mg1 isotype exhibited a moderate level of tumor growth inhibition (Figure 29C) compared to control IgG (Figure 29A) with 2 of 8 mice being TF after up to 42 days, and the OX40-m2a isotype showed stronger anti-tumor activity with 4 of 8 mice being TF (Figure 28D). These data reconfirm the finding that anti-OX40 isotype variants that preferentially binds to activating mouse Fc receptors more potently inhibit tumor growth than isotypes that preferentially bind to the murine inhibitory Fc receptor. The data further show that antibody-mediated agonism of mouse OX40 is dependent on FcR-mediated cross-linking as the anti-mOX40 antibody reformatted as a variant which cannot bind Fc receptors (OX40-g1D265A) displayed no antitumor activity (Figure 29B) compared to the IgG isotype control (Figure 29A).

### REFERENCE EXAMPLE 15

### Isotype-Dependent Anti-Tumor Activity of ICOS-targeting Fc Fusion Proteins in SalN Tumor Model

Anti-mouse ICOS antibody 17G9 is a rat IgG2b agonistic monoclonal antibody that blocks binding between ICOS and B7h and is known to enhance T cell responses, including T cell proliferation and cytokine production (McAdam *et al.*, 2000). ICOS ligand (ICOSL) binds specifically to ICOS and acts as a costimulatory signal for T cell proliferation and cytokine secretion. ICOSL-fusion proteins were generated containing the extracellular domain of murine ICOSL fused to either murine IgG1 Fc (ICOSL-muIgG1) or human IgG1 Fc (ICOSL-hIgG1). ICOSL-hIgG1 and antibody 17G9 preferentially interact with mouse activating FcRs whereas ICOSL-mIgG1 preferentially interacts with the mouse inhibitory FcR.

The anti-tumor potency of different isotypes of Fc fusion proteins that bind specifically to ICOS was investigated in a SalN sarcoma model. A/J mice were subcutaneously injected with 2 x 10⁶ SalN tumor cells. At Day 7 post-implantation, tumor-bearing mice were randomized and dosed with 10 mg/kg of Fc fusion protein by IP injection three times, once every three days (Q3D x 3).

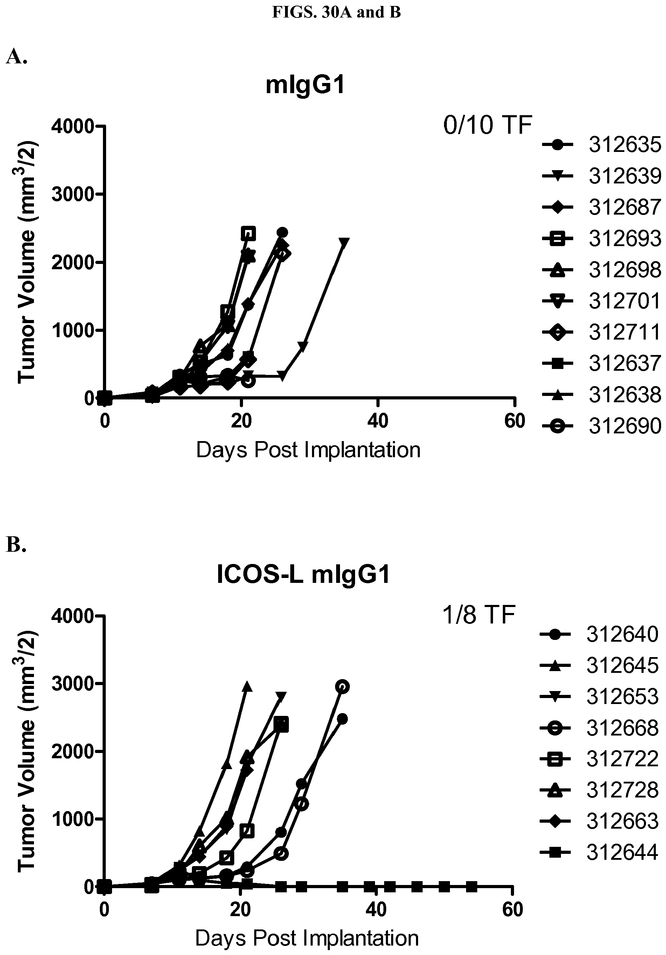

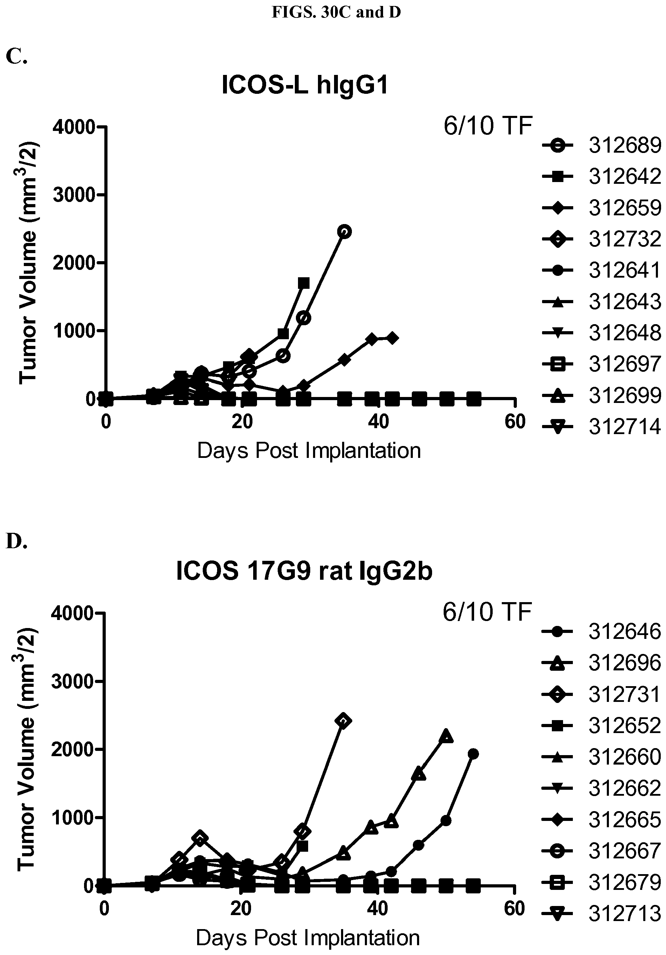

The results are shown in Figure 30. ICOSL-mIgG1 did not exhibit significant anti-tumor activity (Figure 30B) compared to control mouse IgG1 (Figure 30A). In contrast, both ICOSL-hIgG1 (which was previously shown to exhibit anti-tumor efficacy; *see* Ara *et al.*, 2003) and 17G9 exhibited strong anti-tumor activity, each having 6 out of 10 TF mice (Figures 30C and D). Thus, pronounced anti-tumor activity in this mouse SaN1 tumor model correlates with the ability of the Fc portion of the Fc fusion protein to bind to mouse activating FcRs.

### REFERENCE EXAMPLE 16

### Effects of Agonist Anti-ICOS Antibody on T_{reg} Populations in MC38 Tumor Model

MC38 colon tumor cells (2 x 10⁶ cells per implant) were implanted subcutaneously into C57BL/6 mice. At Day 7 post-implantation, tumor-bearing mice were randomized and dosed with 10 mg/kg of the rat IgG2b antibody, 17G9, or mouse IgG1 control antibody, by IP injection Q3D x 3. On Day 15 post-implantation, tumors were harvested, dissociated into single cell suspensions and stained for flow cytometry *(see* Example 3).

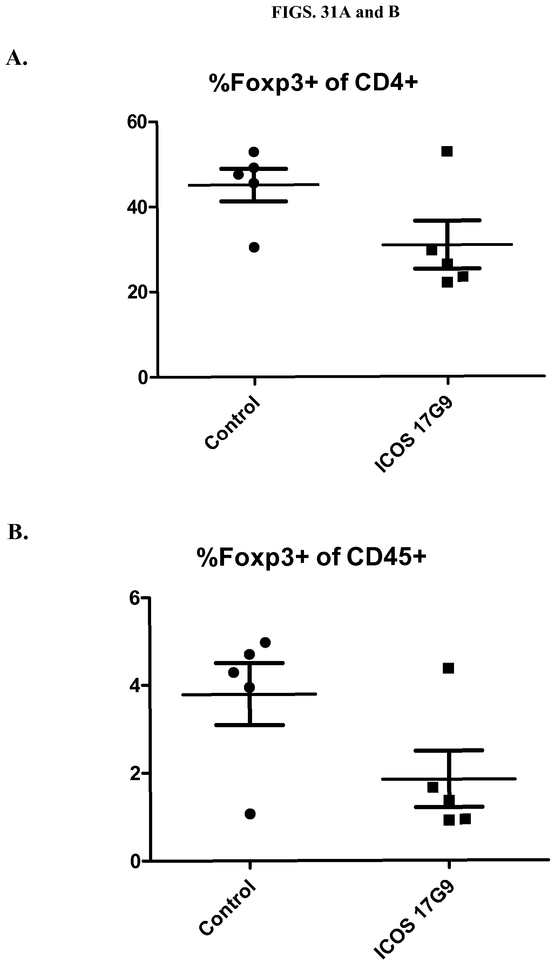

As shown in Figures 31A and B, treatment with 17G9 results in reductions of Foxp3⁺ regulatory cells at the tumor site of MC38 tumors, expressed as a percentage of either CD4⁺ cells or as a percentage of CD45⁺ total lymphocytes.

### REFERENCE EXAMPLE 17

### Anti-Tumor Activity of Variant Anti-PD-1 Isotypes in MC38 Tumor Model

### Experiment #1

The anti-tumor activity of different isotypes of anti-mouse PD-1 antibody 4H2 was assessed in a staged MC38 colon tumor model as previously described (Example 4). 4H2 is a chimeric rat-mouse anti-mPD-1 antibody constructed from a rat IgG2a anti-mouse PD-1 antibody in which the Fc-portion was replaced with an Fc-portion from a mouse IgG1 isotype (WO 2006/121168). It blocks binding of mPD-L1 and mPD-L2 binding to mPD-1, stimulates a T cell response, and exhibits anti-tumor activity. C57BL/6 mice were each subcutaneously injected with 2 x 10⁶ MC38 tumor cells. After 7 days, the mice were randomized into 4 treatment groups and test antibodies were administered IP at 200 µg per dose in a volume of 200 µl as follows: Group 1: mouse IgG1 control (IgG); Group 2: anti-PD-1 IgG1; Group 3: anti-PD-1 IgG1D265A; Group 4: anti-PD-1 IgG2a.

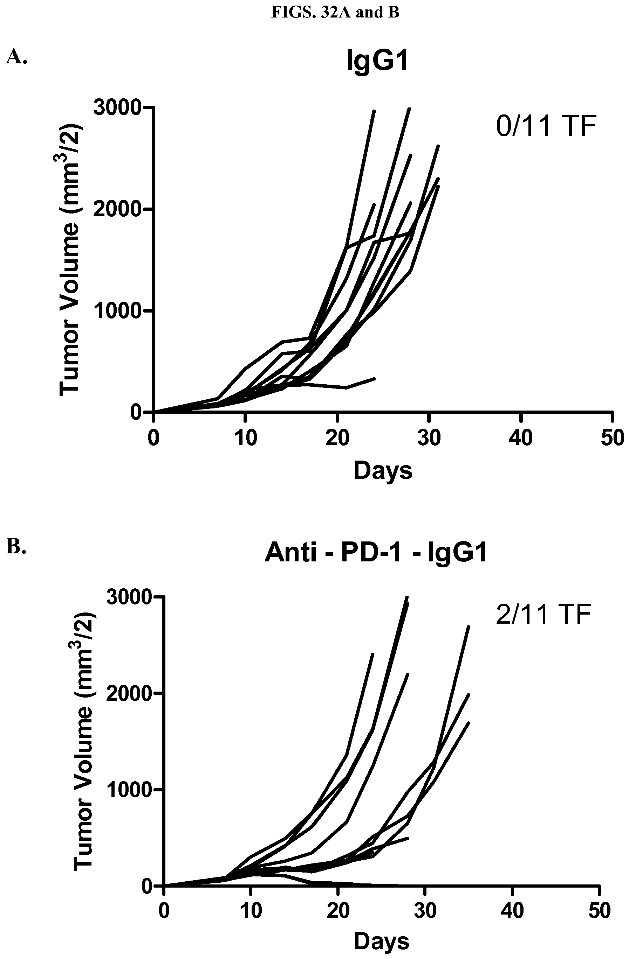

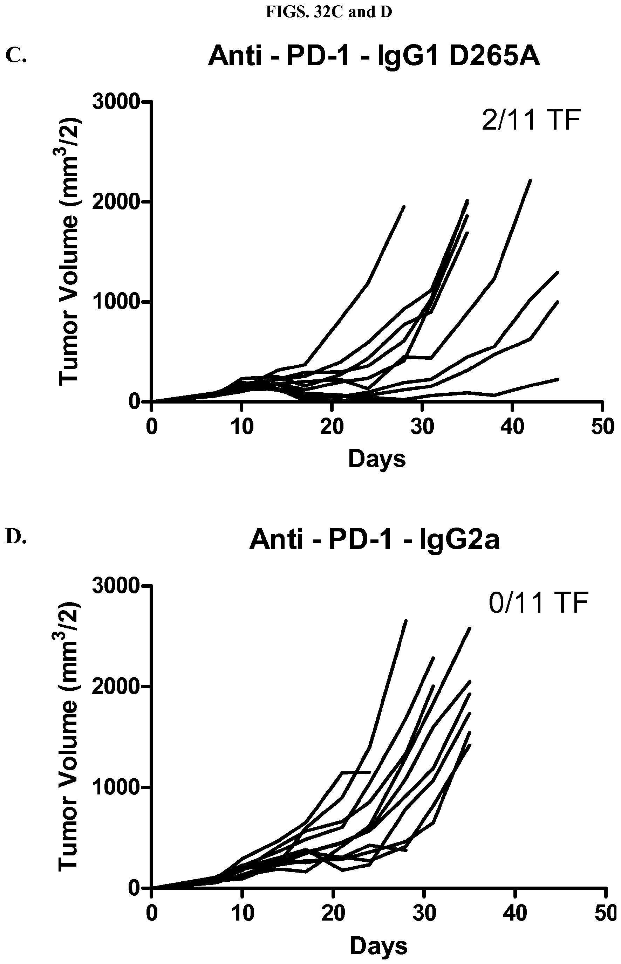

As shown in Figure 32, the 3 anti-PD-1 isotypes showed low levels of anti-tumor activity, with the IgG1 treatment producing 2 TF mice out of 11 (Figure 32B), and the IgGlD265A treatment also producing 2 TF mice out of 11 though this isotype appeared to have somewhat greater anti-tumor activity generally (Figure 32C). Treatment with the IgG2a isotype produced no TF mice out of 11 (Figure 32D) but generally exhibited slightly greater anti-tumor activity than the mouse IgG1 control (Figure 32A). Thus, whereas the anti-PD-1 IgG2a isotype exhibited some anti-tumor activity, this was less than that exhibited by the anti-PD-1 IgG1 or IgG1D265A isotypes. Clearly, in contrast to the results obtained with anti-CTLA-4, GITR, OX40 and ICOS IgG2a antibodies, the anti-PD-1 IgG2a isotype did not potentiate anti-tumor activity relative to the G1 and G1D265A isotypes.

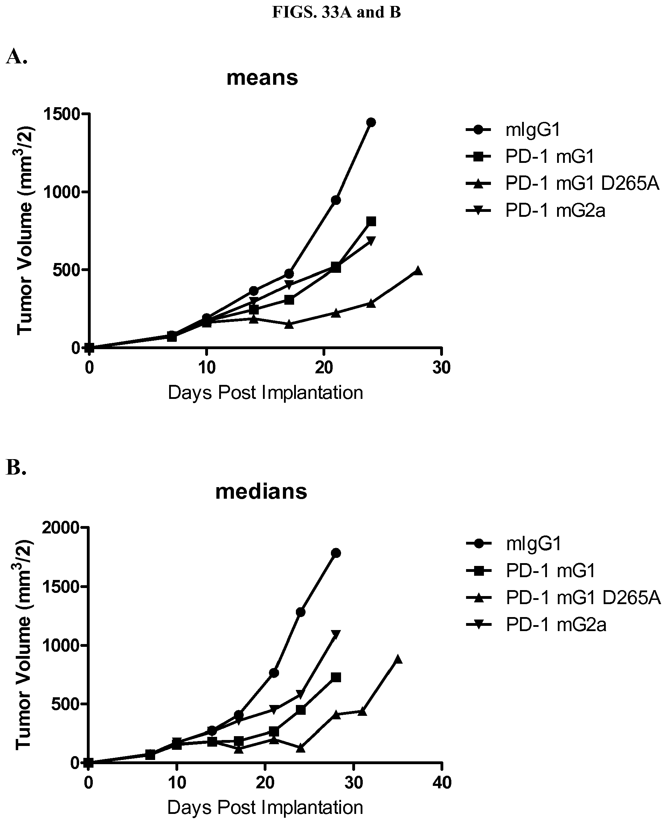

The changes in mean tumor volumes and median tumor volumes of the mice of groups treated with the different anti-PD-1 isotypes are shown in Figures 33A and B. These plots confirm the individual mouse data shown in Figure 32, clearly revealing that the IgG1D265A isotype exhibits the strongest inhibitory effect on MC38 tumor growth.

### Effects of anti-PD-1 isotypes on MC38 T cell subsets in TILs

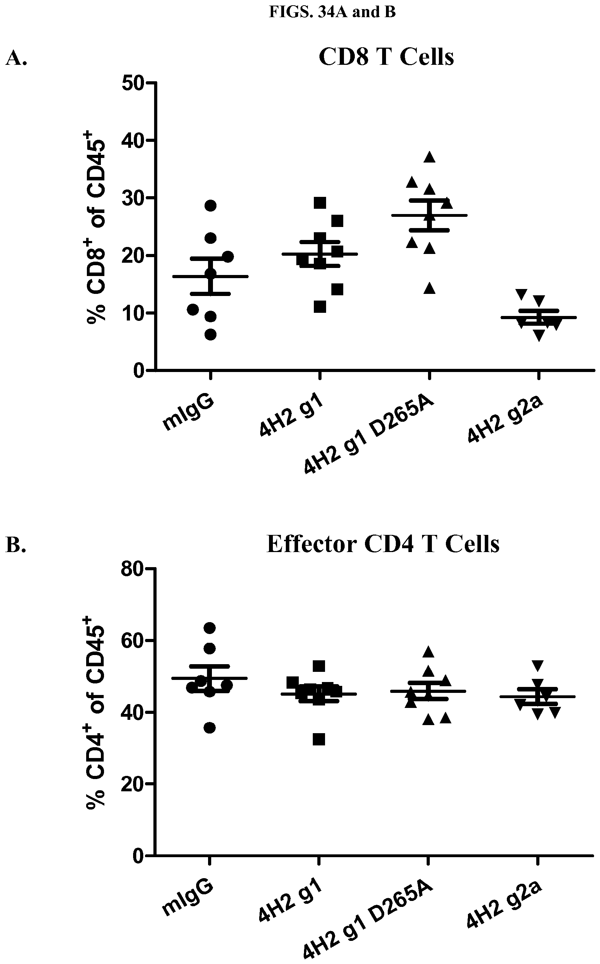

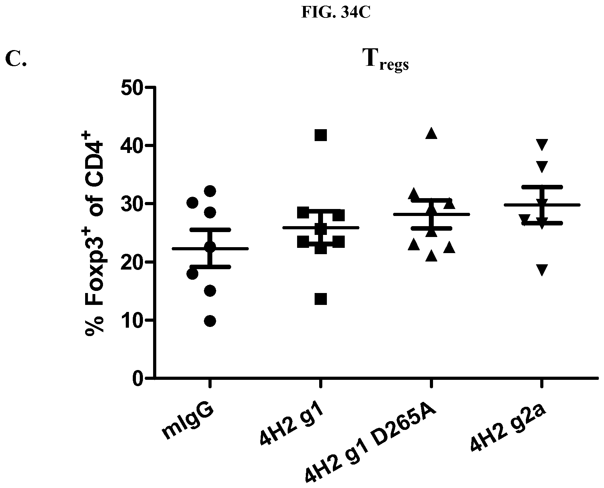

The percentages of T cell subsets that infiltrate the MC38 tumor in mice treated with the different anti-PD-1 isotypes were compared. Figure 34A shows that whereas 4H2-G1 and G1D265A isotypes induced small increase of about 20% and 50%, respectively, in the percentage of CD8⁺ cells compared to both the mouse IgG1 control, the IgG2a isotype caused an approximately 50% decrease in the percentage of CD8⁺ cells. These 3 4H2 isotypes caused virtually no change in the percentage of CD4⁺ cells compared to the mouse IgG1 isotype control Figure 34B, and induced small increases in the percentage of CD4⁺FoxP3⁺ T_{regs}, with the IgG2a isotype causing the most significant increase (Figure 34C). These results diverge from those obtained with the corresponding isotypes anti-CTLA-4, GITR, OX40 and ICOS antibodies.

Flow cytometric measurement of the level of PD-1 expression on different subsets of T cells in MC38 TILs showed that it was most highly expressed on CD8⁺ T_{effs}, with progressively lower levels of expression on T_{regs} and CD4⁺ T_{effs} *(see* Example 18).

### Experiment #2

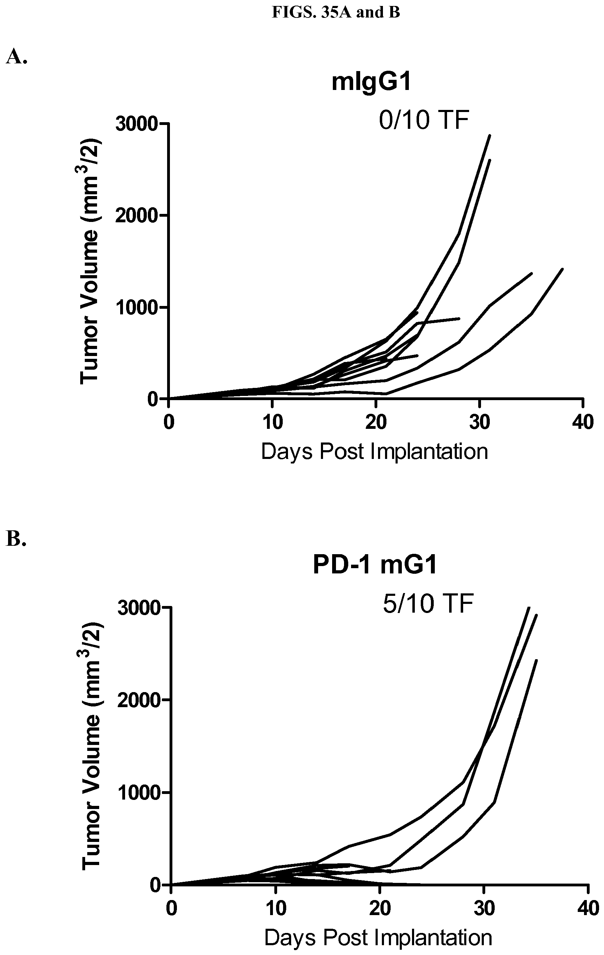

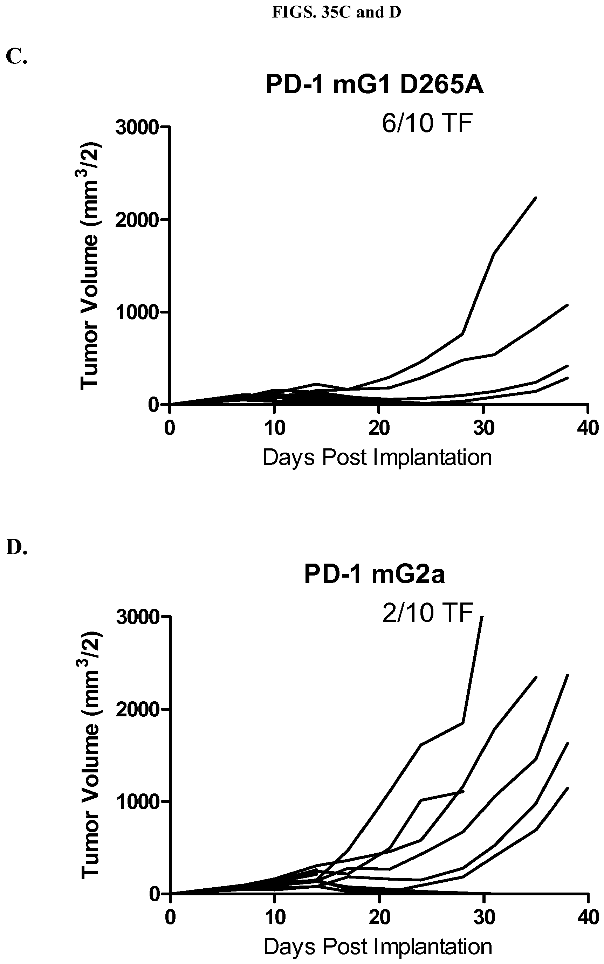

The above tests of anti-tumor activity of the different anti-PD-1 isotypes in the MC38 tumor model was repeated except for the immunomonitoring experiment. The trends in the results, shown in Figures 34 and 35, are similar to those in Experiment #1 but are accentuated and more clearly show the differences between the different isotypes in inhibiting tumor growth. The anti-PD-1 IgG1 (Figure 34B) and IgG1D265A (Figure 34C) treatments produced 5 and 6 TF mice, respectively, out of 10, whereas treatment with the IgG2a isotype produced 2 TF mice out of 10 (Figure 34D), compared to the mouse IgG1 control which produced no TF mice out of 10 (Figure 32A). The differences in the results between Experiments #1 and #2 are summarized in Table 6.

**Table 6. Anti-tumor activity of anti-PD-1 isotypes in Experiments #1 and #2**

| Treatment | Experiment #1 | Experiment #2 |
|---|---|---|
| | No. of Tumor-Free Mice | No. of Tumor-Free Mice |
| mIgG | 0/11 | 0/10 |
| Anti-PD-1 IgG1 | 2/11 | 5/10 |
| Anti-PD-1 IgG1 | 2/11 | 6/10 |
| Anti-PD-1 IgG2a | 0/11 | 2/10 |

The changes in mean tumor volumes and median tumor volumes, shown in Figures 35A and B, also clearly confirm that the IgG1D265A isotype exhibits the most potent inhibitory effect on MC38 tumor growth, followed by the IgG1 isotype, with the IgG2a isotype exhibiting much lower anti-tumor activity.

### EXAMPLE 18

### Expression Analysis of Receptors on T Cells in the Tumor and Spleen of Tumor-Bearing Mice

Both T_{regs} and conventional T (T_{conv}) cells in the tumor microenvironment express a wide array of costimulatory and coinhibitory receptors. However, engagement of receptors on T_{regs} may have dramatically different effects on cell function compared to engagement of the same target on T_{convs}. For example, agonistic antibodies to OX40 potentiate T_{conv} activation while inhibiting T_{reg} function. Furthermore, the level of expression of each receptor can vary substantially among different T cell subsets and on the same type of T cell in the tumor microenvironment or in the periphery.

**Table 7. Reagents used for flow cytometry analysis of T cell receptor expression**

| Marker | Fluorochrome | Clone | Staining Concentration (µg/ml) |
|---|---|---|---|
| CD4 | 700 | GK1.5 | 0.001 |
| Thy1.2 | BV510 | 53-2.1 | 0.001 |
| Foxp3 | GFP | n/a | |
| Live/Dead | APC/780 | n/a | |
| CD27 | PerCP/710 | LG.7F9 | 0.002 |
| CD137 | PE | 17B5 | 0.002 |
| GITR | PE/Cy7 | DTA-1 | 0.002 |
| OX-40 | BV421 | OX-86 | 0.002 |
| ICOS | APC | C398.4A | 0.002 |

The relative expression levels of a variety of costimulatory and coinhibitory receptors on T_{regs} and T_{convs} were determined at tumor sites and in the spleen. For measuring expression levels of CD27, CD137, GITR, OX40 and ICOS, 9 female in-house bred Foxp3-GFP mice (C57BL/6 background, JAX #: 006772) were injected subcutaneously with 1 x 10⁶ MC38 cells in 100 µl of PBS in the right flank. The mice were sacrificed 14 days after MC38 tumor cell implantation. Spleens and tumors were harvested and pressed through 100-µm cell strainers to generate single cell suspensions. Red blood cells were lysed in spleen samples using ACK lysis buffer (10 mM KHCO₃, 1mM EDTA, 150mM NH₄Cl, pH7.3). Cells were counted, and 2 x 10⁶ live cells from each sample were stained in FACS staining buffer (PBS + 2% FBS, 2mM EDTA) for 30 min at 4°C using the antibodies indicated in Table 7. Cells were washed twice with FACS staining buffer and analyzed immediately by flow cytometry.

In a parallel experiment, expression levels of CD27, GITR, OX40, ICOS, CTLA-4, PD-1, LAG-3, TIM-3 and TIGIT were measured following subcutaneous implantation of 2 x 10⁶ SalN sarcoma cells into A/J mice. At Day 7 post-implantation, tumor-bearing mice were randomized and dosed with 10 mg/kg of antibody by IP injection, Q3D x 3. On Day 15 post-implantation, tumors were harvested, dissociated into single cell suspensions and stained for flow cytometry. The data from this SalN experiment confirm and complement the data obtained from the MC38 tumor analysis.

The relative levels of expression determined from these two experiments are summarized in Table 8. For the co-stimulatory receptors ICOS, OX40 and CD137, expression was higher on T_{regs} than on T_{convs}. and expression on T_{regs} in the tumor was higher than on T_{regs} in the spleen, but expression of GITR and CD27 was about as high on CD8 T_{effs} and on Tregs in both the tumor and the spleen. Furthermore, receptor expression was generally higher on Treg cells in the tumor compared to those in the spleen. For the co-inhibitory receptors, expression on different T cell subsets in the tumor was generally higher on the same types of cells in the spleen. With the exception of CTLA-4, expression of the co-inhibitory receptors was higher on CD8 T_{effs} than on T_{regs} or CD4 cells. The expression of TIGIT was very low or undetectable on all T cell subsets.

**Table 8. Relative expression levels of receptors on T cells in tumor versus spleen**

| Receptor | Costimulatory Receptors | | | | | |
|---|---|---|---|---|---|---|
| | Tumor | | | Periphery (Spleen) | | |
| | T_{reg} | CD8⁺ | CD4⁺ | T_{reg} | CD8⁺ | CD4⁺ |
| ICOS | +++ | + | + | + | - | +/- |
| GITR | ++++ | +++ | + | +++ | ++ | ++ |
| OX40 | ++ | | + | + | - | - |
| CD27 | +++ | +++ | + | +++ | +++ | +++ |
| CD137 | +++ | + | - | + | - | - |
| | Costimulatory Receptors | | | | | |
| CTLA-4 | +++ | + | + | + | - | - |
| PD-1 | + | ++ | +/- | | +/- | - |
| LAG-3 | + | ++ | + | - | - | - |
| TIM-3 | + | +++ | - | - | - | - |
| TIGIT | +/- | - | - | - | - | - |

### REFERENCES

Albanesi M et al. (2012) Cutting Edge: FcγRIII (CD16) and FcγRI (CD64) are responsible for anti-glycoprotein 75 monoclonal antibody TA99 therapy for experimental metastatic B16 melanoma. J Immunol 189(12): 5513-7.
Al-Shamkhani A et al. (1996) OX40 is differentially expressed on activated rat and mouse T cells and is the sole receptor for the OX40 ligand. Eur J Immunol 26: 1695-9.
Ara G et al. (2003) Potent activity of soluble B7RP-1-Fc in therapy of murine tumors in syngeneic hosts. Int J Cancer 103(4): 501-7.
Ascierto PA et al. (2011) Anti-CTLA4 monoclonal antibodies: the past and the future in clinical application. J Transl Med 9: 196.
Attia P et al. (2005) Autoimmunity correlates with tumor regression in patients with metastatic melanoma treated with anti-cytotoxic T-lymphocyte antigen-4. J Clin Oncol 23(25): 6043-53.
Baitsch L et al. (2012) Extended co-expression of inhibitory receptors by human CD8 T-cells depending on differentiation, antigen-specificity and anatomical localization. PloS One 7(2): e30852.
Berman DM et al. (2009) Association of peripheral blood absolute lymphocyte count (ALC) and clinical activity in patients (pts) with advanced melanoma treated with ipilimumab [abstr] J Clin Oncol 27(suppl): 15s.3020.
Berger R et al. (2008) Phase I safety and pharmacokinetic study of CT-011, a humanized antibody interacting with PD-1, in patients with advanced hematologic malignancies. Clin Cancer Res 14: 3044-51.
Bevaart L et al. (2006) The high-affinity IgG receptor, FcγRI, plays a central role in antibody therapy of experimental melanoma. Cancer Res 66: 1261-4.
Birebent B et al. (2004) Suppressive properties of human CD4+ CD25+ regulatory T cells are dependent on CTLA-4 expression. Eur J Immunol 34: 3485-96.
Bour-Jordan H et al. (2011) Intrinsic and extrinsic control of peripheral T-cell tolerance by costimulatory molecules of the CD28/B7 family. Immunol Rev 241(1): 180-205.
Bradbury ARM et al. (2011) Beyond natural antibodies: the power of in vitro display technologies. Nat Biotechnol 29(3): 245-54.
Brahmer J et al. (2012) Safety and activity of anti-PD-L1 antibody in patients with advanced cancer. N Engl J Med 366(26): 2455-65.
Bruhns P et al. (2009) Specificity and affinity of human Fcγ receptors and their polymorphic variants for human IgG subclasses. Blood 113: 3716-25.
Bulliard Y et al. (2013) Activating Fcγ receptors contribute to the antitumor activities of immunoregulatory receptor-targeting antibodies. J Exp Med 210(9): 1685-93.
Cartron G et al. (2004) From the bench to the bedside: ways to improve rituximab efficacy. Blood 104(9): 2635-42.
Clynes RA et al. (2000) Inhibitory Fc receptors modulate in vivo cytotoxicity against tumor targets. Nat Med 2000;6:443-46.
Cohen AD et al. (2010) Agonist anti-GITR monoclonal antibody induces melanoma tumor immunity in mice by altering regulatory T cell stability and intra-tumor accumulation. PLoS One 5(5): e10436.
Comin-Anduix B et al. (2008) Detailed analysis of immunologic effects of the cytotoxic T lymphocyte-associated antigen 4-blocking monoclonal antibody tremelimumab in peripheral blood of patients with melanoma. J Transl Med 6: 22.
Corse E and Allison JP (2012) Cutting edge: CTLA-4 on effector T cells inhibits in trans. J Immunol 189: 1123-27.
Coyle AJ and Gutierrez-Ramos JC (2001) The expanding B7 superfamily: increasing complexity in costimulatory signals regulating T cell function. Nat Immunol 2(3): 203-9.
Crowe JS et al. (1992) Humanized monoclonal antibody CAMPATH-1H: myeloma cell expression of genomic constructs, nucleotide sequence of cDNA constructs and comparison of effector mechanisms of myeloma and Chinese hamster ovary cell-derived material. Clin Exp Immunol 87: 105-10.
Dall'Ozzo S et al. (2004) Rituximab-dependent cytotoxicity by natural killer cells: influence of FCGR3A polymorphism on the concentration-effect relationship. Cancer Res 64: 4664-9.
Driessens et al. (2009) Costimulatory and coinhibitory receptors in anti-tumor immunity. Immunol Rev 229(1): 126-44.
Emmerich J et al. (2012) IL-10 directly activates and expands tumor-resident CD8(+) T cells without de novo infiltration from secondary lymphoid organs. Cancer Res 72: 3570-81.
European Publication No. EP1176195, entitled "Method for controlling the activity of immunologically functional molecule," published January 30, 2002 by Hanai N *et al.*
Flies DB et al. (2011) Blockade of the B7-H1/PD-1 Pathway for Cancer Immunotherapy. Yale J Biol Med 84: 409-21.
Gennari R et al. (2004) Pilot study of the mechanism of action of preoperative trastuzumab in patients with primary operable breast tumors overexpressing HER2. Clin Cancer Res 10: 5650-5.
Hamid O et al. (2009) Association of baseline and on-study tumor biopsy markers with clinical activity in patients (pts) with advanced melanoma treated with ipilimumab [abstr] J Clin Oncol 27(suppl): 15s.9008.
Hamid O et al. (2011) A prospective phase II trial exploring the association between tumor microenvironment biomarkers and clinical activity of ipilimumab in advanced melanoma. J Transl Med 9: 204.
Harding FA et al. (1992) CD28-mediated signaling co-stimulates murine T cells and prevents induction of anergy in T cell clones. Nature 356: 607-10.
Harris NL and Ronchese F (1999) The role of B7 costimulation in T-cell immunity. Immunol Cell Biol 77(4): 304-11.
He YF et al. (2004) Blocking programmed death-1 ligand-PD-1 interactions by local gene therapy results in enhancement of antitumor effect of secondary lymphoid tissue chemokine. J Immunol 173: 4919-28.
Hodi FS et al. (2008) Immunologic and clinical effects of antibody blockade of cytotoxic T lymphocyte-associated antigen 4 in previously vaccinated cancer patients. Proc Natl Acad Sci USA 105: 3005-10.
Hodi FS et al. (2010) Improved survival with ipilimumab in patients with metastatic melanoma. N Engl J Med 363:711-23.
Hollinger and Hudson (2005) Engineered antibody fragments and the rise of single domains. Nature Biotech 23(9): 1126-36.
Hoos A et al. (2010) Development of ipilimumab: contribution to a new paradigm for cancer immunotherapy. Semin Oncol 37: 533-46.
Huang RR et al. (2011) CTLA4 blockade induces frequent tumor infiltration by activated lymphocytes regardless of clinical responses in humans. Clin Cancer Res 17(12): 4101-9.
Hurwitz AA et al. (1996) Specific blockade of CTLA-4/B7 interactions results in exacerbated clinical and histologic disease in an actively-induced model of experimental allergic encephalomyelitis. J Neuroimmunol 73: 57-62.
Idusogie EE et al. (2000) Mapping of the C1q binding site on rituxan, a chimeric antibody with a human IgG1 Fc. J Immunol 164: 4178-84.
Ji RR et al. (2102) An immune-active tumor microenvironment favors clinical response to ipilimumab. Cancer Immunol Immunother 61: 1019-31.
Keler T et al. (2003) Activity and safety of CTLA-4 blockade combined with vaccines in cynomolgus macaques. J Immunol 171: 6251-59.
Krummel MF and Allison JP (1995) CD28 and CTLA-4 have opposing effects on the response of T cells to stimulation. J Exp Med 182: 459-65.
Ku GY et al. (2010) Single-institution experience with ipilimumab in advanced melanoma patients in the compassionate use setting: lymphocyte count after 2 doses correlates with survival. Cancer 116: 1767-75.
Lazar GA et al. (2006) Engineered antibody Fc variants with enhanced effector function. Proc Natl Acad Sci USA 103:4005-10.
Leach DR et al. (1996) Enhancement of antitumor immunity by CTLA-4 blockade. Science 271(5256): 1734-6.
Lenschow DJ et al. (1996) CD28/B7 system of T cell costimulation. Annu Rev Immunol 14: 233-58.
Liakou CI et al. (2008) CTLA-4 blockade increases IFNgamma-producing CD4+ICOShi cells to shift the ratio of effector to regulatory T cells in cancer patients. Proc Natl Acad Sci USA 105: 14987-92.
Lonberg N et al. (1994) Antigen-specific human antibodies from mice comprising four distinct genetic modifications. Nature 368 (6474): 856-9.
Louis E et al. (2004) Association between polymorphism in IgG Fc receptor IIIa coding gene and biological response to infliximab in Crohn's disease. Aliment Pharmacol Ther 19: 511-9.
Maker AV et al. (2005) Analysis of the cellular mechanism of antitumor responses and autoimmunity in patients treated with CTLA-4 blockade. J Immunol 175(11): 7746-54.
McAdam AJ et al. (2000) Mouse inducible costimulatory molecule (ICOS) expression is enhanced by CD28 costimulation and regulates differentiation of CD4+ T cells. J Immunol 165: 5035-40.
Mellman I et al. (2011) Cancer immunotherapy comes of age. Nature 480: 480-9. Miescher S et al. (2004) A single recombinant anti-RhD IgG prevents RhD immunization: association of RhD-positive red blood cell clearance rate with polymorphisms in the FcγRIIA and FcγIIIA genes. Blood 103: 4028-35.
Mitsui J et al. (2010) Two distinct mechanisms of augmented antitumor activity by modulation of immunostimulatory/inhibitory signals. Clin Cancer Res 16: 2781-91.
Mossner E et al. (2010) Increasing the efficacy of CD20 antibody therapy through the engineering of a new type II anti-CD20 antibody with enhanced direct and immune effector cell-mediated B-cell cytotoxicity. Blood 115: 4393-402.
Musolino A et al. (2008) Immunoglobulin G fragment C receptor polymorphisms and clinical efficacy of trastuzumab-based therapy in patients with HER-2/neu-positive metastatic breast cancer. J Clin Oncol 26(11): 1789-96.
Mumm JB et al. (2011) IL-10 elicits IFNγ-dependent tumor immune surveillance. Cancer Cell 20: 781-96.
Natsume A et al. (2009) Improving effector functions of antibodies for cancer treatment: Enhancing ADCC and CDC. Drug Des Devel Ther 3: 7-16.
Nimmerjahn F and Ravetch JV (2005) Divergent immunoglobulin G subclass activity through selective Fc receptor binding. Science 310: 1510-2.
Nimmerjahn F and Ravetch JV (2008) Fcγ receptors as regulators of immune responses. Nat Rev Immunol 8(1): 34-47.
Nimmerjahn F and Ravetch JV (2010) Antibody-mediated modulation of immune responses. Immunol Rev 236: 265-75.
Nocentini G and Riccardi C (2005) GITR: a multifaceted regulator of immunity belonging to the tumor necrosis factor receptor superfamily. Eur J Immunol 35: 1016-22.
Nordstrom JL et al. (2011) Anti-tumor activity and toxico-kinetics analysis of MGAH22, an anti-HER2 monoclonal antibody with enhanced Fcγ receptor binding properties. Breast Cancer Res 13: R123.
Olafsen and Wu (2010) Antibody vectors for imaging. Semin Nucl Med 40(3):167-81.
O'Mahony and Janik (2006) Comment on "Analysis of the cellular mechanism of antitumor responses and autoimmunity in patients treated with CTLA-4 blockade." J Immunol 176(9): 5136.
Onishi Y et al. (2008) Foxp3+ natural regulatory T cells preferentially form aggregates on dendritic cells in vitro and actively inhibit their maturation. Proc Natl Acad Sci USA 105: 10113-18.
Pardoll DM (2012a) Immunology beats cancer: a blueprint for successful translation. Nat Immunol 13(12): 1129-32.
Pardoll DM (2012b) The blockage of immune checkpoints in cancer immunotherapy. Nat Rev Cancer 12: 252-64.
PCT Publication No. WO 99/54342, entitled "Glycosylation engineering of antibodies for improving antibody-dependent cellular cytotoxicity," published October 28, 1999 by Umaña *P et al.*
PCT Publication No. WO 02/43478, entitled "Transgenic transchromosomal rodents for making human antibodies," published June 6, 2002 by Medarex, Inc. and Kirin Beer Kabushiki Kaisha.
PCT Publication No. WO 03/035835, entitled "Glycoprotein compositions," published May 1, 2003 by Genentech, Inc.
PCT Publication No. WO 2006/089231, entitled "Monoclonal antibodies against prostate specific membrane antigen (PSMA) lacking in fucosyl residues," published August 24, 2006 by Medarex, Inc.
PCT Publication No. WO 2006/121168, entitled "Human monoclonal antibodies to Programmed Death 1(PD-1) and methods for treating cancer using anti-PD-1 antibodies alone or in combination with other immunotherapeutics," published November 16, 2006 by ONO Pharmaceutical Co., Ltd. and Medarex, Inc.
PCT Publication No. WO 2009/101611, entitled "Monoclonal antibodies for tumor treatment," published August 20, 2009 by Curetech Ltd.
Peggs KS et al. (2006) Principles and use of anti-CTLA4 antibody in human cancer. Curr Opin Immunol 16: 206-13.
Peggs KS et al. (2008) Cell intrinsic mechanisms of T-cell inhibition and application to cancer therapy. Immunol Rev 224: 141-65.
Peggs KS et al. (2009) Blockade of CTLA-4 on both effector and regulatory T cell compartments contributes to the antitumor activity of anti-CTLA-4 antibodies. J Exp Med 206(8): 1717-25.
Perrin PJ et al. (1996) CTLA-4 blockade enhances clinical disease and cytokine production during experimental allergic encephalomyelitis. J Immunol 157: 1333-36.
Quezada SA et al. (2006) CTLA4 blockade and GM-CSF combination immunotherapy alters the intratumor balance of effector and regulatory T cells. J Clin Invest 116(7): 1935-45.
Qureshi OS et al. (2011) Trans-endocytosis of CD80 and CD86: a molecular basis for the cell-extrinsic function of CTLA-4. Science 332: 600-03.
Read S et al. (2000) Cytotoxic T lymphocyte-associated antigen 4 plays an essential role in the function of CD25+CD4(+) regulatory cells that control intestinal inflammation. J Exp Med 192(2): 295-302.
Ribas A et al. (2007) Tremelimumab (CP-675,206), a cytotoxic T lymphocyte associated antigen 4 blocking monoclonal antibody in clinical development for patients with cancer. Oncologist 12: 873-83.
Ribas A (2010) Clinical development of the anti-CTLA-4 antibody tremelimumab. Semin Oncol 37(5): 450-4.
Rosenberg SA (2006) Response to comment on "Analysis of the cellular mechanism of antitumor responses and autoimmunity in patients treated with CTLA-4 blockade." J Immunol 176(9): 5136.
Rudd CE, Taylor A, Schneider H (2009) CD28 and CTLA-4 coreceptor expression and signal transduction. Immunol Rev 229: 12-26.
Salomon B and Bluestone JA (2001) Complexities of CD28/B7: CTLA-4 costimulatory pathways in autoimmunity and transplantation. Annu Rev Immunol 19:225-52.
Schreier PH et al. (1981) Multiple differences between the nucleic acid sequences of the IgG2aa and IgG2ab alleles of the mouse. Proc Natl Acad Sci USA 78: 4495-99.
Schwartz RH (2003) T cell anergy. Annu Rev Immunol 21: 305-34.
Selby MJ et al. (2013) Anti-CTLA-4 antibodies of IgG2a isotype enhance antitumor activity through reduction of intratumoral regulatory T cells. Cancer Immunol Res 1: 32-42.
Shields RL et al. (2001) High resolution mapping of the binding site on human IgG1 for FcγRI, FcγRII, FcγRIII, and FcRn and design of IgG1 variants with improved binding to the FcyR. J Biol Chem 276: 6591-6604.
Shields RL et al. (2002) Lack of fucose on human IgG1 N-linked oligosaccharide improves binding to human FcγRIII and antibody-dependent cellular toxicity. J Biol Chem 277: 26733-40.
Shimizu J et al. (2002) Stimulation of CD25+CD4+ regulatory T cells through GITR breaks immunological self-tolerance. Nat Immunol 3: 135-42.
Simpson TR et al. (2013) Fc-dependent depletion of tumor-infiltrating regulatory T cells co-defines the efficacy of anti-CTLA-4 therapy against melanoma. J Exp Med 210: 1695-710.
Stavenhagen JB et al. (2007) Fc optimization of therapeutic antibodies enhances their ability to kill tumor cells in vitro and controls tumor expansion in vivo via low-affinity activating Fcγ receptors. Cancer Res 67: 8882-90.
Takahashi T et al. (2000) Immunologic self-tolerance maintained by CD4+ CD25+ regulatory T cells constitutively expressing cytotoxic T lymphocyte-associated antigen 4. J Exp Med 192: 303-10.
Tamura K et al. (2011) FcγR2A and 3A polymorphisms predict clinical outcome of trastuzumab in both neoadjuvant and metastatic settings in patients with HER2-positive breast cancer. Ann Oncol 22(6): 1302-7.
Tarentino AL et al. (1975) The isolation and structure of the core oligosaccharide sequences of IgM. Biochem 14: 5516-23.
Tivol EA et al. (1995) Loss of CTLA-4 leads to massive lymphoproliferation and fatal multiorgan tissue destruction, revealing a critical negative regulatory role of CTLA-4. Immunity 3: 541-47.
Topalian SL et al. (2012) Safety, activity, and immune correlates of anti-PD-1 antibody in cancer. N Engl J Med 366(26): 2443-54.
Turk MJ et al. (2004) Concomitant tumor immunity to a poorly immunogenic melanoma is prevented by regulatory T cells. J Exp Med 200:771-82.
Umaña P et al. (1999) Engineered glycoforms of an antineuroblastoma IgG1 with optimized antibody-dependent cellular cytotoxic activity. Nat Biotech 17:176-80.
U.S. Patent No. 5,977,318, entitled "CTLA4 receptor and uses thereof," issued November 2, 1999 to Linsley PS *et al.*
U.S. Patent No. 6,682,736, entitled "Human monoclonal antibodies to CTLA-4," issued January 27, 2004 to Hanson DC *et al.*
U.S. Patent No. 6,984,720, entitled "Human CTLA-4 antibodies," issued January 10, 2006 to Korman AJ *et al.*
U.S. Patent No. 7,605,238, entitled "Human CTLA-4 antibodies and their uses," issued October 20, 2009 to Korman AJ *et al.*
U.S. Publication No. 2004/0110704, entitled "Cells of which genome is modified," published June 10, 2004 by Yamane N *et al.*
U.S. Publication No. 2012/0276086, entitled "Monoclonal antibodies against CD30 lacking in fucosyl and xylosyl residues," published November 1, 2012, by Black AN *et al.*
van der Merwe PA et al. (1997) CD80 (B7-1) binds both CD28 and CTLA-4 with a low affinity and very fast kinetics. J Exp Med 185: 393-403.
Walunas TL et al. (1994) CTLA-4 can function as a negative regulator of T cell activation. Immunity 1(5): 405-13.
Wang CJ et al. (2012) Cutting edge: cell-extrinsic immune regulation by CTLA-4 expressed on conventional T cells. J Immunol 189: 1118-22.
Waterhouse P et al. (1995) Lymphoproliferative disorders with early lethality in mice deficient in Ctla-4. Science 270: 985-8.
Weber J (2010) Immune checkpoint proteins: a new therapeutic paradigm for cancer-preclinical background: CTLA-4 and PD-1 blockade. Semin Oncol 37(5): 430-9.
Weng WK and Levy R (2003) Two immunoglobulin G fragment C receptor polymorphism independently predict response to rituximab in patients with follicular lymphoma. J Clin Oncol 21: 3940-7.
Wing K et al. (2008) CTLA-4 control over Foxp3+ regulatory T cell function. Science 322: 271-5.
Yamane-Ohnuki N et al. (2004) Establishment of FUT8 knockout Chinese hamster ovary cells: an ideal host cell line for producing completely defucosylated antibodies with enhanced antibody-dependent cellular cytotoxicity. Biotechnol Bioeng 87: 614-22.
Yuan J et al. (2011) Integrated NY-ESO-1 antibody and CD8+ T-cell responses correlate with clinical benefit in advanced melanoma patients treated with ipilimumab. Proc Natl Acad Sci USA 108:16723-28.
Zalevsky J et al. (2010) Enhanced antibody half-life improves in vivo activity. Nat Biotechnol 28: 157-9.

## Claims

1. Afucosylated ipilimumab for use in treating cancer.

## Patentansprüche

1. Afucosyliertes Ipilimumab zur Verwendung bei der Behandlung von Krebs.

## Revendications

1. Ipilimumab afucosylé pour une utilisation dans le traitement du cancer.
